# EUROPEAN PATENT SPECIFICATION

(11) **EP 1 455 777 B1**
(45) Date of publication and mention of the grant of the patent: **21.11.2007**
(21) Application number: 02788198.6
(22) Date of filing: 19.12.2002
(51) Int. Cl.: A61K 31/33, C07D 295/12, C07D 207/06, C07D 209/44, C07D 215/06, C07D 223/04, C07D 333/34, C07D 311/70, C07D 285/14, C07D 271/12, C07D 333/62, C07D 513/04, A61P 25/18, A61P 25/28

(54) **GLYT1 TRANSPORTER INHIBITORS AND USES THEREOF IN TREATMENT OF NEUROLOGICAL AND NEUROPSYCHIATRIC DISORDERS**
GLYT1 TRANSPORTER INHIBITOREN UND DEREN ANWENDUNG IN DER BEHANDLUNG VON NEUROLOGISCHEN UND NEUROPSYCHIATISCHEN KRANKHEITEN
INHIBITEURS DE TRANSPORTEURS GLYT1 ET UTILISATONS DE CEUX-CI DANS LE TRAITEMENT DE TROUBLES NEUROLOGIQUES ET NEUROPSYCHIATRIQUES

(30) Priority: 21.12.2001 GB 0130696
(43) Date of publication of application: 15.09.2004
(73) Proprietor: SMITHKLINE BEECHAM PLC, Brentford, Middlesex TW8 9GS (GB)
(72) Inventor: COULTON, Steven, GlaxoSmithKline, Harlow, Essex CM19 5 AW (GB); HADLEY, Michael, Stewart, GlaxoSmithKline, Harlow, Essex CM19 5AW (GB); HERDON, Hugh, Jonathan, GlaxoSmithKline, Harlow, Essex CM19 5AW (GB); JIN, Jian, GlaxoSmithKline, King of Prussia, PA 19406 (US); JOINER, Graham, J., GlaxoSmithKline, Harlow, Essex CM19 5AW (GB); PORTER, Roderick, Alan, GlaxoSmithKline, Harlow, Essex CM19 5AW (GB); RAHMAN, Shahzad, Sharooq, GlaxoSmithKline, Harlow, Essex CM19 5AW (GB)
(74) Representative: Kondo, Rie
(86) International application number: PCT/GB2002/005819
(87) International publication number: WO 2003/055478

(56) References cited:
- EP-A- 0 076 072
- WO-A-99/34790

## Description

The present invention relates to glycine transporter inhibiting compounds, their use in the manufacture of medicaments for treating neurological and neuropsychiatric disorders, in particular psychoses, dementia or attention deficit disorder. The invention further comprises processes to make these compounds and pharmaceutical formulations thereof.

Synaptic transmission is a complex form of intercellular communication that involves a considerable array of specialised structures in both the pre-and post-synaptic terminal and surrounding glial cells (Kanner and Schuldiner, CRC Critical Reviews in Biochemistry, 22, 1987:1032). Transporters sequester neurotransmitter from the synapse, thereby regulating the concentration of neurotransmitter in the synapse, as well as its duration therein, which together influence the magnitude of synaptic transmission. Further, by preventing the spread of transmitter to neighbouring synapses, transporters maintain the fidelity of synaptic transmission. Last, by sequestering released transmitter into the presynaptic terminal, transporters allow for transmitter reutilisation.

Neurotransmitter transport is dependent upon extracellular sodium and the voltage difference across the membrane; under conditions of intense neuronal firing, as, for example, during seizure, transporters can function in reverse, releasing neurotransmitter in a calcium-independent non-exocytotic manner (Atwell et al., Neuron, 11, 1993: 401-407). Pharmacologic modulation of neurotransmitter transporters thus provides a means for modifying synaptic activity, which provides useful therapy for the treatment of neurological and psychiatric disturbances.

The amino acid glycine is a major neurotransmitter in the mammalian central nervous system, functioning at both inhibitory and excitatory synapses. By nervous system, both the central and peripheral portions of the nervous system are intended. These distinct functions of glycine are mediated by two different types of receptor, each of which is associated with a different class of glycine transporter. The inhibitory actions of glycine are mediated by glycine receptors that are sensitive to the convulsant alkaloid strychnine, and are thus referred to as "strychnine-sensitive". Such receptors contain an intrinsic chloride channel that is opened upon binding of glycine to the receptor; by increasing chloride conductance, the threshold for firing of an action potential is increased. Strychnine-sensitive glycine receptors are found predominantly in the spinal cord and brainstem, and pharmacological agents that enhance the activation of such receptors will thus increase inhibitory neurotransmission in these regions.

Glycine also functions in excitatory transmission by modulating the actions of glutamate, the major excitatory neurotransmitter in the central nervous system. See Johnson and Ascher, Nature, 325, 1987: 529-531; Fletcher et al., Glycine Transmission, Otterson and Storm-Mathisen, eds., 1990: 193-219. Specifically, glycine is an obligatory co-agonist at the class of glutamate receptor termed N-methyl-D-aspartate (NMDA) receptor. Activation of NMDA receptors increases sodium and calcium conductance, which depolarises the neuron, thereby increasing the likelihood that it will fire an action potential. NMDA receptors are widely distributed throughout the brain, with a particularly high density in the cerebral cortex and hippocampal formation.

Molecular cloning has revealed the existence in mammalian brains of two classes of glycine transporters, termed GlyT1 and GlyT2. GlyT1 is found predominantly in the forebrain and its distribution corresponds to that of glutaminergic pathways and NMDA receptors (Smith, et al., Neuron, 8, 1992: 927-935). Molecular cloning has further revealed the existence of three variants of GIyT1, termed GIyT-la, GlyT-1b and GlyT-1c (Kim et al., Molecular Pharmacology, 45, 1994: 608-617), each of which displays a unique distribution in the brain and peripheral tissues. The variants arise by differential splicing and exon usage, and differ in their N-terminal regions. GlyT2, in contrast, is found predominantly in the brain stem and spinal cord, and its distribution corresponds closely to that of strychnine-sensitive glycine receptors (Liu et al., J. Biological Chemistry, 268, 1993: 22802-22808; Jursky and Nelson, J. Neurochemistry, 64, 1995 : 1026-1033). Another distinguishing feature of glycine transport mediated by GlyT2 is that it is not inhibited by sarcosine as is the case for glycine transport mediated by GlyT1. These data are consistent with the view that, by regulating the synaptic levels of glycine, GlyT1 and GlyT2 selectively influence the activity of NMDA receptors and strychnine-sensitive glycine receptors, respectively.

NMDA receptors are critically involved in memory and learning (Rison and Staunton, Neurosci. Biobehav. Rev.. 19 533-552 (1995); Danysz et al, Behavioral Pharmacol., 6 455-474 (1995)); and, furthermore, decreased function of NMDA-mediated neurotransmission appears to underlie, or contribute to, the symptoms of schizophrenia (Olney and Farber, Archives General Psychiatry, 52, 998-1007 (1996). Thus, agents that inhibit GlyT1 and thereby increase glycine activation of NMDA receptors can be used as novel antipsychotics and anti-dementia agents, and to treat other diseases in which cognitive processes are impaired, such as attention deficit disorders and organic brain syndromes. Conversely, over-activation of NMDA receptors has been implicated in a number of disease states, in particular the neuronal death associated with stroke and possibly neurodegenerative diseases, such as Alzheimer's disease, multi-infarct dementia, AIDS dementia, Huntington's disease, Parkinson's disease, amyotrophic lateral sclerosis or other conditions in which neuronal cell death occurs, such as stroke or head trauma. Coyle & Puttfarcken, Science, 262, 689-695 (1993); Lipton and Rosenberg, New Engl. J. of Medicine, 330, 613-622 (1993); Choi, Neuron, 1, 623-634 (1988). Thus, pharmacological agents that increase the activity of GlyT1 will result in decreased glycine-activation of NMDA receptors, which activity can be used to treat these and related disease states. Similarly, drugs that directly block the glycine site of the NMDA receptors can be used to treat these and related disease states.

Glycine transport inhibitors are already known in the art, for example as disclosed in published International Applications WO97/45423 (Trophix Pharmaceuticals, Inc.), and WO97/45115 (Trophix Pharmaceuticals Inc.). The classes of compounds disclosed in these applications inhibit glycine transport *via* the GlyT1 or GlyT2 transporters.

In published International Application WO99/34790 (Allelix Neuroscience, Inc.), there is disclosed a class of compounds which also inhibits glycine transport via the GlyT1 or GlyT2 transporters, with preferred compounds showing selectivity for the inhibition of glycine transport *via* GlyT2 versus GlyT1.

Further prior art publications disclosing glycine transport inhibitors include published International Applications WO99/45011 (Janssen Pharmaceutica N.V.), WO00/07978. (Akzo Nobel N.V.) and WO01/87855 (Yamanouchi Pharmaceutical Co. Ltd.). Published International Applications WO01/32602 and WO01/81308 (both NPS Allelix Corp.) disclose classes of compounds which inhibit glycine transport (or reuptake) *via* the GlyT1 transporter. Published International Application WO01/36423 (Akzo Nobel N.V.) discloses a class of compounds that selectively inhibit glycine transport by the human GlyT1 transporter as compared to the human GlyT2 transporter.

However, there still remains the need to identify further compounds that can inhibit GlyT1 transporters, including those that inhibit GlyT1 transporters selectively over GlyT2 transporters. Such compounds would thus be suitable for the treatment of certain neurological and neuropsychiatric disorders, including psychoses such as schizophrenia, dementia and other forms of impaired cognition such as attention deficit disorders and organic brain syndromes. Other neuropsychiatric disorders include drug-induced (phencyclidine, ketamine and other dissociative anaesthetics, amphetamine and other psychostimulants and cocaine) psychosis, psychosis associated with affective disorders, brief reactive psychosis, schizoaffective psychosis, and psychosis NOS, "schizophrenia-spectrum" disorders such as schizoid or schizotypal personality disorders, or illness associated with psychosis (such as major depression, manic depressive (bipolar) disorder, Alzheimer's disease and post-traumatic stress syndrome), and NMDA receptor-related disorders such as autism, depression, benign forgetfulness, childhood learning disorders and closed head injury.

Published European Patent Application EP-A-0076072 (Beecham-Wuelfing GmbH), discloses *inter alia* compounds of the following general structure: wherein 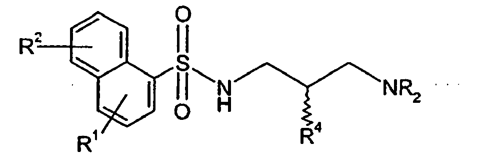 one of R¹ and R² is hydrogen and the other is selected from C₁₋₄ alkyl, hydroxyl, C₁₋₄ alkoxy, C₁₋₄ alkanoyloxy, hydrogen, halogen, nitro, cyano and amino optionally substituted by one or two C₁₋₄ alkyl groups or by C₁₋₄ alkanoyl; R⁴ is hydroxy or C₁₋₄ alkoxy; and
NR₂ is 1-pyrrolidyl, 1-piperidyl, morpholino or 4-methyl-1-piperazyl, optionally substituted by one or two methyl groups;
and salts thereof, their use as anti-arrhythmic agents, processes for their preparation and pharmaceutical compositions containing them.

Published International Application WO01/14315 (The Scripps Research Institute), discloses the compound of the following structure: and a process for its preparation. 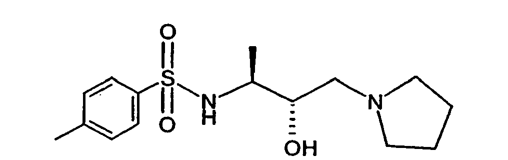 Drosdow and Stawrowskaja (Zh. Obshch. Khim.; 9; 1939; 1642-1644), refer to the compounds of the following structure: 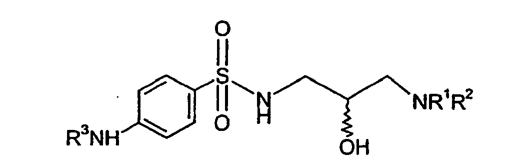 wherein NR₁R₂ is 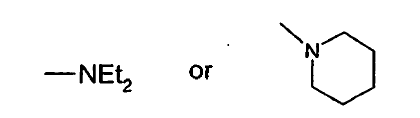 and R³ is hydrogen or MeC(O)-.

Soviet Patent No. 466218 (Kuliew et al.), refers to the compounds of the following structure: 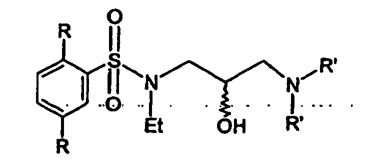 wherein R' is C₂-C₄ alkyl and R is methyl, Cl or Br.

Hori and Janda (J. Organic Chem. 1998, 63(3), 889-894), disclose the compounds of the following structure: 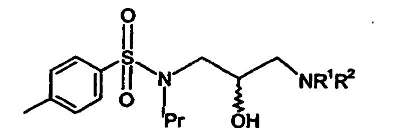 wherein NR¹R² is

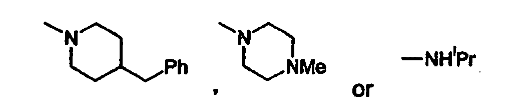

It has now surprisingly been found that a class of sulfonamide compounds, including certain of the known compounds referred to above, inhibit GlyT1 transporters and are thus useful in the treatment of certain neurological and neuropsychiatric disorders, including schizophrenia.

Thus, in one aspect, there is provided the use of a compound of formula (I), 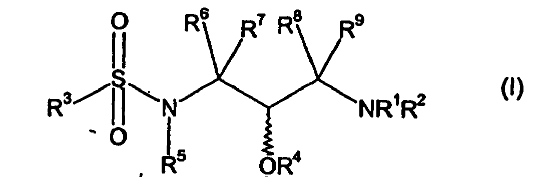 or a salt, solvate ester or amide thereof, for the manufacture of a medicament for treating a disorder mediated by GlyT1, wherein: R¹ and R² are independently selected from hydrogen. C₁-C₆ alkyl and C₃-C₆ cycloalkyl, with the proviso that R¹ and R² do not both represent hydrogen, or R¹ and R² together with the nitrogen atom to which they are attached are linked to form a 4-, 5-, 6- or 7-membered saturated ring, wherein one or more of the carbon atoms is optionally replaced by a heteroatom independently selected from N, O and S, said saturated ring being optionally substituted by one or more groups independently selected from C₁-C₆ alkyl, C₃-C₆ cycloalkyl, C₃-C₆ cycloalkylC₁-C₄ alkyl, aryl and arylC₁-C₄ alkyl, and said saturated ring being further optionally bridged by a C₁-C₃ alkylene group, and said saturated ring being further optionally fused to a C₅-C₇ alicyclic or 5- or 6-membered aromatic or heteroaromatic ring optionally substituted by one or more groups independently selected from C₁-C₆ alkyl and C₃-C₆ cycloalkyl;
R³ is 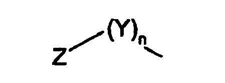 wherein
Y is C₁-C₂ alkylene, C₂ alkenylene or C₂ alkynylene,
   and
n is 0 or 1, and
Z is a 5- to 8-membered monocyclic or 6- to 10-membered bicyclic aromatic ring system wherein one or more of the carbon atoms is optionally replaced by a heteroatom independently selected from N, O and S, and said ring system being optionally substituted by one or more groups independently selected from -hal, -R¹⁰, -CF₃, -C₁₋₆alkylsulphonyl, -OR¹¹, -COOR¹², -CN, -NO₂, -NR¹³R¹⁴, -C(O)NR¹⁵R¹⁶, -NR¹⁷C(O)R¹⁸, -C(O)R¹⁹, -C(NR²⁰)NR²¹R²², -C(NOR²³)R²⁸,
   wherein
hal is F, Cl, Br or I,
R¹⁰ is C₁-C₆ alkyl, C₃-C₆ cycloalkyl, C₃-C₆ cycloalkylC₁-C₄ alkyl, aryl, aryloxy or aryl C₁-C₄ alkyl, optionally substituted by one or more groups independently selected from hal, C₁-C₆ alkyl, -OR¹¹, -COOR¹² -CN, -NO₂ and -NR¹³R¹⁴,
R¹¹, R¹², R¹³, R¹⁴, R¹⁵, R¹⁸, R¹⁷, R¹⁸, R²⁰, R²¹, R²², R²³ and R²⁸ are independently selected from hydrogen and C₁-C₆ alkyl;
R⁴ and R¹⁹ are independently selected from hydrogen, C₁-C₆ alkyl, C₃-C₆ cycloalkyl, aryl or arylC₁-C₄ alkyl, optionally substituted by one or more groups independently selected from hal, C₁-C₆ alkyl, -OR²⁴, -COOR²⁵ -CN, -NO₂ and -NR²⁶R²⁷;
R⁶**,** R⁷, R⁸ and R⁹ are independently selected from hydrogen, C₁-C₆ alkyl or arylC₁-C₄ alkyl, or R⁶ and R⁷ together form a C₃-C₆ cycloalkyl group, or R⁸ and R⁹ together form a C₃-C₆ cycloalkyl group; wherein the C₁-C₆ alkyl, arylC₁-C₄ alkyl group, the C₃-C₆ cycloalkyl group formed by R⁶ and R⁷, and the C₃₋C₆ cycloalkyl group formed by R⁸ and R⁹, are optionally substituted by one or more groups independently selected from hal, C₁-C₆ alkyl, -OR²⁴, -COOR²⁵, -CN, -NO₂ and -NR²⁶R²⁷,
   wherein R²⁴, R²⁵, R²⁸ and R²⁷ are independently selected from hydrogen and C₁-C₆ alkyl; and
R⁵ is independently selected from hydrogen, C₁-C₆ alkyl, C₃-C₆ cycloalkyl, aryl and arylC₁-C₄ alkyl, optionally substituted by one or more groups independently selected from hal, C₁-C₆ alkyl, -OR²⁴, -COOR²⁵ -CN, -NO₂ and -NR²⁶R²⁷,
wherein R²⁴, R²⁵, R²⁶ and R²⁷ are as hereinbefore defined.
and wherein the term "aryl" refers to a 5- to 7-membered aromatic or heteroaromatic ring system wherein the heteroatomic ring contains at least one heteroatom selected from N, O and S.

Suitably, the 4-, 5-, 6- or 7-membered saturated ring formed by R¹ and R² together with the nitrogen atom to which they are linked is selected from the group comprising: azetidine, azepine, pyrrolidine, imidazolidine, piperidine, morpholine, thiomorpholine, piperazine.

Suitably, the 5- to 8-membered aromatic monocyclic moiety of Z is selected from the group comprising: furan, thiophene, pyrrole, oxazole, thiazole, imidazole, pyrazole, isoxazole, isothiazole, oxadiazole, triazole, thiadiazole, benzene, pyran, pyridine, pyridazine, pyrimidine, pyrazine, piperazine, triazine.

Suitably, the 6- to 10-membered aromatic bicyclic moiety of Z is selected from the group comprising: thienofuran, indolizine, indole, isoindole, indoline, benzofuran, benzothiophene, indazole, benzimidazole, benzthiazole, purine, quinolizine, quinoline, isoquinoline, cinnoline, phthalazine, quinazoline, quinoxaline, naphthyridine, pteridine, chroman, chromene, isochroman, indene, imidazoleisothiazole, benzothiadiazole, benzofuran, naphthalene, azulene.

Preferably, the compound of formula (I) as hereinbefore described has the following stereochemical configuration:

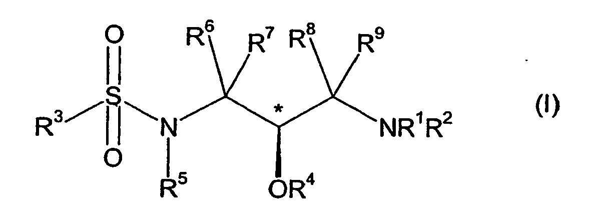

It will be understood by the skilled artisan that the stereochemical configuration at the chiral centre marked with a * will be assigned the Cahn-Ingold-Prelog notation of (R).

In one embodiment, R¹ and R² together with the nitrogen atom to which they are attached are linked to form a 4-, 5-, 6- or 7-membered heterocyclic ring, wherein the sole heteroatom is the nitrogen atom to which R¹ and R² are attached, said ring being optionally substituted as hereinbefore described, and said ring being further optionally fused to a C₅-C₇ alicyclic or 5- or 6-membered aromatic or heteroaromatic ring as hereinbefore described.

In another embodiment, R¹ and R² together with the nitrogen atom to which they are attached are linked to form a 5- or 6-membered ring, wherein one or more of the carbon atoms is optionally replaced by a heteroatom independently selected from N, O and S, said ring being optionally substituted as hereinbefore described, and said ring being further optionally fused to a C₅-C₇ alicyclic or 5- or 6-membered aromatic or heteroaromatic ring as hereinbefore described.

In another embodiment, R¹ and R² together with the nitrogen atom to which they are attached are linked to form a 5- or 6-membered heterocyclic ring, wherein the sole heteroatom is the nitrogen atom to which R¹ and R² are attached, said ring being optionally substituted as hereinbefore described, preferably by one of more groups independently selected from C₁-C₆ alkyl and C₃-C₆ cycloalkyl, more preferably by one or more groups independently selected from C₁-C₄ alkyl, most preferably methyl, ethyl or isopropyl.

In another embodiment, R¹ and R² are independently selected from C₁-C₆ alkyl, preferably C₃-C₆ alkyl.

In another embodiment, n is 0.

In another embodiment, Z is a 5- to 8-membered monocyclic ring system as hereinbefore described. Preferably, Z is a 5- or 6-membered monocyclic ring system as hereinbefore described. More preferably, Z is phenyl or thienyl, optionally substituted as hereinbefore described, preferably by one or more groups independently selected from -hal, -R¹⁰, -CF₃, -C₁-C₆alkylsulphonyl, -OR¹¹, -COOR¹², -CN, -NO₂, -NR¹³R¹⁴ as hereinbefore defined, more preferably by one or more groups independently selected from -hal, C₁-C₆ alkyl, C₁-C₆ alkoxy, CF₃, -CN and C₃-C₆ cycloalkyl.

In another embodiment, Z is a 6- to 10-membered bicyclic ring system as hereinbefore described. Preferably, Z is naphthyl, naphthyridine, quinolyl, isoquinolyl, benzothienyl, chromanyl, chromenyl, imidazoleisothiazolyl, benzothiadiazolyl, benzofuryl, optionally substituted as hereinbefore described. More preferably, Z is naphthyl or quinolyl (preferably 5-quinolyl), optionally substituted as hereinbefore described, preferably by one or more groups independently selected from hal, C₁-C₆ alkyl, C₁-C₆ alkoxy, CF₃, -CN and C₃-C₆ cycloalkyl. Most preferably, Z is 1-naphthyl, optionally substituted by one or more groups independently selected from -hal, C₁-C₆ alkyl, C₁-C₆ alkoxy, CF₃, -CN and C₃-C₆ cycloalkyl.

In another embodiment, R⁴ is hydrogen or C₁-C₆ alkyl, preferably hydrogen.

In another embodiment, R⁵ is selected from hydrogen, C₁-C₆ alkyl, aryl and benzyl, optionally substituted by one or more groups independently selected from hal, C₁-C₆ alkyl and OR²⁴. Preferably, R⁵ is hydrogen.

In another embodiment, R⁶, R⁷, R⁸ and R⁹ are independently selected from hydrogen and C₁-C₆ alkyl, preferably hydrogen.

In another aspect of the present invention, there is provided the use of a compound of formula (Ia) for the manufacture of a medicament for treating disorders mediated by GlyT1, said compound having the formula (Ia): 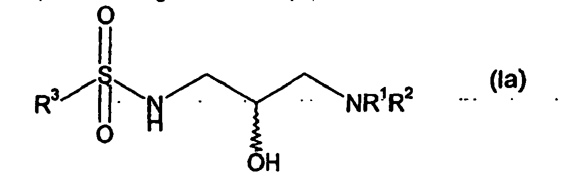 or a salt or solvate, amide or ester thereof, wherein:
R¹ and R² are independently selected from C₃-C₆ alkyl, or
R¹ and R² together with the nitrogen atom to which they are attached are linked to form a 5-, 6-, or 7- membered heterocyclic ring, wherein the sole heteroatom is the nitrogen atom to which R¹ and R² are attached, said ring being optionally substituted by one or more groups independently selected from C₁-C₄ alkyl and C₃-C₆ cycloalkyl, and said ring being further optionally fused to a C₆ alicyclic or aromatic ring, and said ring being further optionally bridged by a methylene group;
R³ is 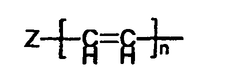 wherein
n is 0 or 1. and
Z is a 5- or 6-membered monocylic or 8- to 10-membered bicyclic aromatic ring system
   wherein one or more of the carbon atoms is optionally replaced by a heteroatom independently selected from N, O and S, and said ring system being optionally substituted by one or more groups independently selected from -hal, -R¹⁰, -OR¹¹, - COOR¹², -CN, -NO₂, -NR¹³R¹⁴, -CF₃, and -C₁-C₆alkylsulphonyl,
   wherein
hal is F, Cl, Br or I,
R¹⁰ is C₁-C₄ alkyl or phenyl, optionally substituted by one or more hal groups, and
R¹¹, R¹², R¹³ and R¹⁴ are independently selected from hydrogen and methyl.

Suitably, when n is 1, the C₂ alkenylene group may be in the cis or *trans* configuration, preferably the *trans* configuration.

In another embodiment, the compound of formula (Ia) as hereinbefore described has the following stereochemical configuration:

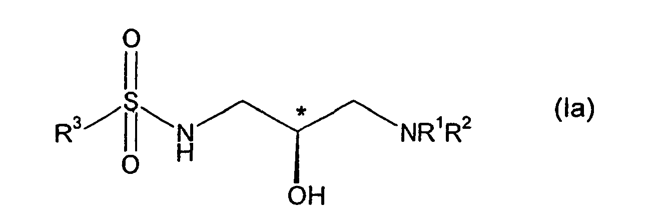

It will be understood by the skilled artisan that the stereochemical configuration at the chiral centre marked with a * will be assigned the Cahn-Ingold-Prelog notation of (R).

In another embodiment, R¹ and R² together with the nitrogen atom to which they are attached are linked to form a pyrrolidinyl ring, said ring being optionally substituted by one of more groups independently selected from C₁-C₄ alkyl preferably methyl, ethyl or isopropyl. Preferably, the pyrrolidinyl ring formed by R¹ and R² together with the nitrogen atom to which they are attached is substituted by one or more C₁-C₄ alkyl groups, preferably methyl, ethyl or isopropyl groups, preferably at the 2- and/or 5-positions, more preferably at the 2-position. More preferably, the pyrrolidinyl ring formed by R¹ and R² together with the nitrogen atom to which they are attached is substituted by one isopropyl group, preferably at the 2- position.

In another embodiment, R¹ and R² together with the nitrogen atom to which they are attached are linked to form a piperidinyl ring, said ring being optionally substituted by one of more groups independently selected from C₁-C₄ alkyl, preferably methyl or ethyl. Preferably, the piperidinyl ring formed by R¹ and R² together with the nitrogen atom to which they are attached is substituted by one or more methyl or ethyl groups, preferably at the 2- and 6-positions. More preferably, the piperidinyl ring formed by R¹ and R² together with the nitrogen atom to which they are attached is substituted by one methyl group, preferably at the 2- position, or by two methyl groups, preferably at the 2- and 6- positions.

In another embodiment, n is 0.

In another embodiment, Z is selected from 2- or 3-thiophene, phenyl, 1- or 2-naphthyl, optionally substituted by one or more groups independently selected from - hal, -R¹⁰, -OR¹¹, -COOR¹², -CN, -NO₂, -NR¹³R¹⁴,
wherein
hal, R¹⁰, R¹¹, R¹², R¹³ and R¹⁴ are as hereinbefore defined.

Preferably, Z is 1-naphthyl, or 5-quinolinyl, optionally substituted by one or more groups independently selected from -hal, -R¹⁰, -OR¹¹, -COOR¹², -CF₃, -C₁-C₆alkylsulphonyl, -CN, -NO₂, and -NR¹³R¹⁴,
wherein
hal, R¹⁰, R¹¹, R¹², R¹³ and R¹⁴ are as hereinbefore defined.

Preferably, R³ is 1-naphthyl or 5-quinolinyl.

Examples of preferred compounds of the invention include
Naphthalene-1-sulfonic acid [(R)-2-hydroxy-3-piperidin-1-ylpropyl]-amide
Naphthalene-1-sulfonic acid {(2R)-hydroxy-3-[(2R,S)-methylpiperidin-1-yl]-propyl}-amide
Naphthalene-1-sulfonic acid {3-[(2R,6S)-dimethylpiperidin-1-yl]-(2R)-hydroxypropyl}-amide
Naphthalene-1-sulfonic acid {3-[(2S)-ethylpiperidin-1-yl]-(2R)-hydroxypropyl}-amide)
Naphthalene-1-sulfonic acid {3-[(2R)-ethylpiperidin-1-yl]-(2R)-hydroxypropyl}-amide
Naphthalene-1-sulfonic acid ((R)-2-hydroxy-3-pyrrolidin-1-ylpropyl)amide trifluoroacetate
Naphthalene-1-sulfonic acid [(R)-2-hydroxy-3-(2-methylpyrrolidin-1-yl)propyl]amide trifluoroacetate
Naphthalene-1-sulfonic acid [(R)-3-(2,4-dimethylpyrrolidin-1-yl)-2-hydroxypropyl]amide trifluoroacetate
Naphthalene-1-sulfonic acid [(R)-2-hydroxy-3-(2-isopropylpyrrolidin-1-yl)propyl]amide trifluoroacetate
Naphthalene-1-sulfonic acid [(R)-3-(2,5-dimethylpyrrolidin-1-yl)-2-hydroxypropyl]amide trifluoroacetate
Naphthalene-1-sulfonic acid[(R)-3-(2-cyclohexylpyrrolidin-1-yl)-2-hydroxypropyl]amide
Naphthalene-1-sulfonic acid[(R)-2-hydroxy-3-(2-isobutylpyrrolidin-1-yl)propyl]amide
Naphthalene-1-sulfonic acid[(R)-3-(2-ethylpyrrolidin-1-yl)-2-hydroxypropyl]amide trifluoroacetate
Naphthalene-1-sulfonic acid[(R)-3-(2-*tert*-butylpyrrolidin-1-yl)-2-hydroxypropyl]amide trifluoroacetate
Naphthalene-1-sulfonic acid[(R)-3-(2-cyclopropylpyrrolidin-1-yl)-2-hydroxypropyl]amide trifluoroacetate
Naphthalene-1-sulfonic acid[(R)-2-hydroxy-3-(3-methylpiperidin-1-yl)propyl]amide trifluoroacetate
Naphthalene-1-sulfonic acid[(R)-2-hydroxy-3-(5-ethyl-2-methylpiperidin-1 - yl)propyl]amide
Naphthalene-1-sulfonic acid[(R)-2-hydroxy-3-(2-ethylpiperidin-1-yl)propyl]amide trifluoroacetate
Naphthalene-1-sulfonic acid[(R)-2-hydroxy-3-(2-isopropylpiperidin-1-yl)propyl]amide trifluoroacetate
Naphthalene-1-sulfonic acid[(R)-(sec-butylpropylamino)hydroxypropyl]amide trifluoroacetate
Naphthalene-1-sulfonic acid[(R)-(*tert*-butylpropylamino)hydroxypropyl]amide trifluoroacetate
Naphthalene-1-sulfonic acid[(R)-3-(1,3-dihydroisoindol-2-yl)-2-hydroxypropyl]amide trifluoroacetate
Naphthalene-1-sulfonic acid[(R)-2-hydroxy-3-(octahydroisoquinolin-2-yl)propyl]amide trifluoroacetate
Naphthalene-1-sulfonic acid[(R)-2-hydroxy-3-(octahydroquinolin-2-yl)propyl]amide
Naphthalene-1-sulfonic acid[(R)-2-hydroxy-3-((1 S,5R)-1 ,3,3-trimethyl-6-azabicycfo[3.2.1]oct-6-yl)-propyl]amide trifluoroacetate
Naphthalene-1-sulfonic acid[(S)-3-(2,4-dimethylpyrrolidin-1-yl)-2-hydroxypropyl]amide trifluoroacetate
Naphthalene-1-sulfonic acid [(S)-2-hydroxy-3-(2-methylpyrrolidin-1-yl)propyl]amide trifluoroacetate
Naphthalene-1-sulfonic acid[(S)-2-hydroxy-3-(3-methylpiperidin-1-yl)propyl]amide trifluoroacetate
Naphthalene-1-sulfonic acid[3-(2,4-dimethylpyrrolidin-1-yl)-2-hydroxypropyl]amide 5-Dimethylamino-naphthalene-1-sulfonic acid [(R)-3-(2,4-dimethyl-pyrrolidin-1-yl)-2-hydroxy-propyl]-amide
Naphthalene-2-sulfonic acid [(R)-3-(2,4-dimethyl-pyrrolidin-1-yl)-2-hydroxy-propyl]-amide
2,5-Dichloro-N-[(R)-3-(2,4-dimethyl-pyrrolidin-1-yl)-2-hydroxy-propyl]-benzenesulfonamide
*N*-[(R)-3-(2,4-Dimethyl-pyrrolidin-1-yl)-2-hydroxy-propyl]-2-nitro-benzenesulfonamide
3,5-Dichloro-*N*-[(R)-3-(2,4-dimethyl-pyrrolidin-1-yl)-2-hydroxy-propyl]-2-hydroxy-benzenesulfonamide
*N*-[(R)-3-(2,4-Dimethyl-pyrrolidin-1-yl)-2-hydroxy-propyl]-2,4,6-triisopropyl-benzenesulfonamide
*N*-[(R)-3-(2,4-Dimethyl-pyrrolidin-1-yl)-2-hydroxy-propyl]-4-nitro-benzenesulfonamide
*N-*[(R)-3-(2,4-Dimethyl-pyrrolidin-1-yl)-2-hydroxy-propyl]-4-methoxy-benzenesulfonamide
(E)-2-Phenyl-ethenesulfonic acid [(R)-3-(2,4-dimethyl-pyrrolidin-1-yl)-2-hydroxypropyl]-amide
2-[(R)-3-(2,4-Dimethyl-pyrrolidin-1-yl)-2-hydroxy-propylsulfamoyl]-benzoic acid methyl ester
*N*-[(R)-3-(2,4-Dimethyl-pyrrolidin-1-yl)-2-hydroxy-propyl]-bis-trifluoromethyl-benzenesulfonamide
3,4-Dichloro-*N*-[(R)-3-(2,4-dimethyl-pyrrolidin-1-yl)-2-hydroxy-propyl]-benzenesulfonamide
*N*-[(R)-3-(2,4-Dimethyl-pyrrolidin-1-yl)-2-hydroxy-propyl]-4-propyl-benzenesulfonamide
4-Bromo-*N*-[(R)-3-(2,4-dimethyl-pyrrolidin-1-yl)-2-hydroxy-propyl]-2,5-difluoro-benzenesulfonamide
*N*-[(R)-3-(2,4-Dimethyl-pyrrolidin-1-yl)-2-hydroxy-propyl]-3-fluoro-benzenesulfonamide
4-Chloro-*N*-[(R)-3-(2,4-Dimethyl-pyrrolidin-1-yl)-2-hydroxy-propyl]-3-fluoro-benzenesulfonamide
2-Chloro-*N*-[(R)-3-(2,4-dimethyl-pyrrolidin-1-yl)-2-hydroxy-propyl]-benzenesulfonamide
2,5-Dichloro-thiophene-3-sulfonic acid [(R)-3-(2,4-dimethyl-pyrrolidin-1-yl)-2-hydroxypropyl]-amide
*N*-[(R)-3-(2,4-Dimethyl-pyrrolidin-1-yl)-2-hydroxy-propyl]-C-trifluoromethyl-benzenesulfonamide
*N*-[(R)-3-(2,4-Dimethyl-pyrrolidin-1-yl)-2-hydroxy-propyl]-3-methyl-benzenesulfonamide
2,3-Dichloro-*N*-[(R)-3-(2,4-dimethyl-pyrrolidin-1-yl)-2-hydroxy-propyl]-benzenesulfonamide
3-Bromo-5-chloro-thiophene-2-sulfonic acid [(R)-3-(2,4-dimethyl-pyrrolidin-1-yl)-2-hydroxy-propyl]-amide
2-Cyano-*N*-[(R)-3-(2,4-dimethyl-pyrrolidin-1-yl)-2-hydroxy-propyl]-benzenesulfonamide
*N*-[(R)-3-(2,4-Dimethyl-pyrrolidin-1-yl)-2-hydroxy-propyl]-2,5-difluoro-benzenesulfonamide
5-Bromo-2-chloro-*N*-[(R)-3-(2,4-dimethy)-pyrrolidin-1-yl)-2-hydroxy-propyl]-benzenesulfonamide
2,2,5,7,8-Pentamethyl-chroman-6-sulfonic acid [(R)-3-(2,4-dimethyl-pyrrolidin-1-yl)-2-hydroxy-propyl]-amide
Benzo[1,2,5]thiadiazole-4-sulfonic acid [(R)-3-(2,4-dimethyl-pyrrolidin-1-yl)-2-hydroxy-propyl]-amide
Benzo[1,2,5]oxadiazole-4-sulfonic acid [(R)-3-(2,4-dimethyl-pyrrolidin-1-yl)-2-hydroxy-propyl]-amide
Biphenyl-4-sulfonic acid [(R)-3-(2,4-dimethyl-pyrrolidin-1-yl)-2-hydroxy-propyl]-amide
*N*-[(R)-3-(2,4-Dimethyl-pyrrolidin-1-yl)-2-hydroxy-propyl]-4-methyl-3-nitrobenzenesulfonamide
5-Chloro-3-methyl-benzo[b]thiophene-2-sulfonic acid [(R)-3-(2,4-dimethyl-pyrrolidin-1-yl)-2-hydroxy-propyl]-amide
4-Butyl-*N*-[(R)-3-(2,4-dimethyl-pyrrolidin-1-yl)-2-hydroxy-propyl]-benzenesulfonamide
5-Chloro-benzo[1,2,5]oxadiazole-4-sulfonic acid [(R)-3-(2,4-dimethyl-pyrrolidin-1-yl)-2-hydroxy-propyl]-amide
4-Butyl-*N*-[(R)-3-(2,4-dimethyl-pyrrolidin-1-yl)-2-hydroxy-propyl]-benzenesulfonamide
6-Chloro-imidazo[2,1-b]thiazole-5-sulfonic acid [(R)-3-(2,4-dimethyl-pyrrolidin-1-yl)-2-hydroxy-propyl]-amide
*N*-[(R)-3-(2,4-Dimethyl-pyrrolidin-1-yl)-2-hydroxy-propyl]-3-methoxy-benzenesulfonamide
5-lodo-naphthalene-1-sulfonic acid [(R)-3-(2,4-dimethyl-pyrrolidin-1-yl)-2-hydroxypropyl]-amide
2-Bromo-*N*-[(R)-3-(2,4-dimethyl-pyrrolidin-1-yl)-2-hydroxy-propyl]-benzenesulfonamide
*N*-[(R)-3-(2,4-Dimethyl-pyrrolidin-1-yl)-2-hydroxy-propyl]-5-fluoro-2-methyl-benzenesulfonamide
Naphthalene-1-sulfonic acid [(R)-3-(2,4-dimethyl-pyrrolidin-1-yl)-2-hydroxy-propyl]-amide
*N*-[(R)-3-(2,4-Dimethyl-pyrrolidin-1-yl)-2-hydroxy-propyl]-2,4,6-trimethyl-benzenesulfonamide
*N*-[(R)-3-(2,4-Dimethyl-pyrrolidin-1-yl)-2-hydroxy-propyl]-trifluoromethyl-benzenesulfonamide
Naphthalene-1-sulfonic acid [(R)-3-(2,6-diethylpiperidin-1-yl)-2-hydroxy-propyl]-amide
2,3-Dichloro-N-[(R)-3-((2S,6R)-2,6-dimethyl-piperidin-1-yl)-2-hydroxy-propyl]-benzenesulfonamide
2,3,4-Trichloro-*N*-[(R)-3-((2S,6R)-2,6-dimethyl-piperidin-1-yl)-2-hydroxy-propyl]-benzenesulfonamide
2,5-Dichlorothiophene-3-sulfonic acid [(R)-3-((2S,6R)-2,6-dimethyl-piperidin-1-yl)-2-hydroxy-propyl]-amide
4,5-Dibromo-thiophene-2-sulfonic acid [(R)-3-((2R,6S)-2,6-dimethyl-piperidin-1-yl)-2-hydroxy-propyl]amide
4-Bromo-2,5-dichloro-thiophene-3-sulfonic acid [(R)-3-((2S,6R)-2,6-dimethyl-piperidin-1-yl)-2-hydroxy-propyl]-amide
5-Chloro-naphthalene-1-sulfonic acid [(R)-3-((2S,6R)-2,6-dimethyl-piperidin-1-yl)-2-hydroxy-propyl]-amide
5-Chloro-naphthalene-2-sulfonic acid [(R)-3-((2S,6R)-2,6-dimethyl-piperidin-1-yl)-2-hydroxy-propyl]-amide
Naphthalene-2-sulfonic acid [(R)-3-((2S,6R)-2,6-dimethyl-piperidin-1-yl)-2-hydroxypropyl]-amide
N-[(R)-3-((2S,6R)-2,6-Dimethyl-piperidin-1-yl)-2-hydroxy-propyl]-benzenesulfonamide
4'-Chloro-biphenyl-4-sulfonic acid [(R)-3-((2S,6R)-2,6-dimethyl-piperidin-1-yl)-2-hydroxy-propyl]-amide
Biphenyl-4-sulfonic acid [(R)-3-((2S,6R)-2,6-dimethyl-piperidin-1-yl)-2-hydroxypropyl]-amide
*N*-[(R)-3-((2S,6R)-2,6-Dimethyl-piperidin-1-yl)-2-hydroxy-propyl]-4-phenoxy-benzenesulfonamide
3,4-Dichloro-*N*-[(R)-3-((2S,6R)-2,6-dimethyl-piperidin-1-yl)-2-hydroxy-propyl]-benzenesulfonamide
Quinoline-5-sulfonic acid [(R)-3-((2S,6R)-2,6-dimethyl-piperidin-1-yl)-2-hydroxypropyl]-amide
Quinoline-8-sulfonic acid [(R)-3-((2S,6R)-2,6-dimethyl-piperidin-1-yl)-2-hydroxypropyl]-amide
5-lodo-naphthalene-1-sulfonic acid [(R)-3-((2S,6R)-2,6-dimethyl-piperidin-1-yl)-2-hydroxy-propyl]-amide
5-Acetyl-naphthalene-1-sulfonic acid [(R)-3-((2S,6R)-2,6-dimethyl-piperidin-1-yl)-2-hydroxy-propyl]-amide
Isoquinoline-5-sulfonic acid [(R)-3-((S)-2-ethyl-piperidin-1-yl)-2-hydroxy-propyl]-amide
Quinoline-8-sutfonic acid [(R)-3-((S)-2-ethyl-piperidin-1-yl)-2-hydroxy-propyl]-amide
5-Chloro-3-methyl-benzo[b]thiophene-2-sulfonic add [(R)-3-((S)-2-ethyl-piperidin-1-yl)-2-hydroxy-propyl]-amide
*N*-[(R)-3-((S)-2-Ethylpiperidin-1-yl)-2-hydroxy-propyl]-1-phenyl-methanesulfonamide
2,3-Dichloro-*N*-[(R)-3-((S)-2-ethyl-piperidin-1-yl)-2-hydroxy-propyl]-benzenesulfonamide
Thiophene-2-sulfonic acid [(*R*)-3-((S)-2-ethyl-pipeddin-1-yl)-2-hydroxy-propyl]-amide
2,5-Dichlorothiophene-3-sulfonic acid [(R)-3-((S)-2-ethyl-piperidin-1-yl)-2-hydroxypropyl]-amide
5-Methoxynaphthalene-1-sulfonic acid [(R)-3-((2R,6S)-2,6-dimethyl-pipeddin-1-yl)-2-hydroxy-propyl]-amide
5-Cyanonaphthalene-1-sulfonic acid [(R)-3-((2R,6S)-2.6-dimethyl-piperidin-1-yl)-2-hydroxy-propyl]-amide
4-Cyanonaphthalene-1-sulfonic acid [(R)-3-((2R,6S)-2.6-dimethyl-piperidin-1-yl)-2-hydroxy-propyl]-amide
4-Bromonaphthalene-1-sulfonic add [(R)-3-((2R,6S)-2.6-dimethyl-piperldin-1-yl)-2-hydroxy-propyl]-amide
4-Fluoronaphthalene-1-sulfonic acid [(R)-3-((2R,6S)-2,6-dimethyl-piperidin-1-yl)-2-hydroxy-propyl]-amide
N-[(R)-3-((2R,6S)-2,6-Dimethyl-piperidin-1-yl)-2-hydroxy-propyl]-2,3-dimethyl-benzenesulfonamide
N-[(R)-3-((2R,6S)-2,6-Dimethyl-piperidin-1-yl)-2-hydroxy-propyl]-3,4-dimethyl-benzenesulfonamide
N-[(R)-3-((2R,6S)-2,6-Dimethyl-piperidin-1-yl)-2-hydroxy-propyl]-2,3-dimethoxy-benzenesulfonamide
7-Trifluoromethyl-quinoline-5-sulfonic acid [(R)-3-((2R,6S)-2.6-dimethyl-pipefidin-1-yl)-2-hydroxy-propyl]-amide
7-Fluoro-2-methyl-quinoline-5-suffonic acid [(R)-3-((2R,6S)-2,6-dimethyl-piperidin-1-yl)-2-hydroxy-propyl]-amine
and salts, solvate esters or amides thereof.

As used herein, the term "disorders mediated by GlyT1" refers to disorders that may be treated by the administration of a medicament that alters the activity of the GlyT1 transporter. As hereinbefore described, the action of GlyT1 transporters affects the local concentration of glycine around NMDA receptors. As a certain amount of glycine is needed for the efficient functioning of NMDA receptors, any change to that local concentration can affect NMDA-mediated neurotransmission. As hereinbefore described, changes in NMDA-mediated neurotransmission have been implicated in certain neuropsychiatric disorders such as dementia, depression and psychoses, for example schizophrenia, and learning and memory disorders, for example attention deficit disorders and autism. Thus, alterations in the activity of the GlyT1 transporter are expected to influence such disorders.

As used herein, the term "C₁-C₆ alkyl" refers to a straight or branched chain hydrocarbon which contains at least 1, and at most 6, carbon atoms. Examples of "C₁-C₆ alkyl" groups useful in the present invention include, but are not limited to, methyl, ethyl, n-propyl, i-propyl, n-butyl, i-butyl, t-butyl, n-pentyl, n-hexyl.

In a like manner, the term "C₁-C₄ alkyl" refers to a straight or branched chain hydrocarbon which contains at least 1, and at most 4, carbon atoms. Examples of "C₁-C₄ alkyl" groups useful in the present invention include, but are not limited to, methyl, ethyl, n-propyl, i-propyl, n-butyl, i-propyl and t-butyl.

As used herein, the term "C₃-C₆ cycloalkyl" refers to a non-aromatic cyclic hydrocarbon ring having from three to six carbon atoms. Exemplary "C₃-C₆ cycloalkyl" groups include cyclopropyl, cyclobutyl, cyclopentyl and cyclohexyl.

As used herein, the term "C₃-C₆ cycloalkylC₁-C₄ alkyl" refers to a C₃-C₆ cycloalkyl group, as hereinbefore defined, attached through a C₁-C₄ alkylene linker, wherein C₁-C₄ alkylene is as defined herein. Examples of "C₃-C₆ cycloalkylC₁-C₄ alkyl" include, but are not limited to, cyclohexylmethyl.

As used herein, the term "aryl" refers to a 5- to 7-membered aromatic or heteroaromatic ring system wherein the heteroatomic ring contains at least one heteroatom selected from N, O and S. Exemplary "aryl" groups include thiophenyl, furanyl and phenyl.

As used herein, the term "aryl C₁-C₄ alkyl" refers to an aryl group, as hereinbefore defined, attached through a C₁-C₄ alkylene linker, wherein C₁-C₄ alkylene is as defined herein. Examples of "aryl C₁-C₄ alkyl" include, but are not limited to, benzyl, phenethyl, pyridylmethyl and phenylpropyl.

As used herein, the terms "C₁-C₂ alkylene", "C₁-C₃ alkylene" and "C₁-C₄ alkylene" refer to a straight or branched chain divalent hydrocarbon radical, which contains at least 1, and at most 2, 3 or 4, carbon atoms respectively. Examples of "C₁-C₂ alkylene", "C₁-C₃ alkylene" and "C₁-C₄ alkylene" groups useful in the present invention include methylene, ethylene, n-propylene and n-butylene.

As used herein; the term "C₂ alkenylene" refers to a divalent hydrocarbon radical with a double bond, which contains 2 carbon atoms.

As used herein, the term "C₂ alkynylene" refers to a divalent hydrocarbon radical with a triple bond, which contains 2 carbon atoms.

As used herein, the term "hal" is an abbreviation for "halogen" and refers to fluorine, chlorine, bromine, or iodine.

As used herein, the term "optionally" means that the subsequently described event(s) may or may not occur, and includes both event(s) which occur, and event(s) that do not occur.

As used herein, the term "substituted" refers to substitution with the named substituent or substituents, multiple degrees of substitution being allowed unless otherwise stated.

As used herein, the term "salt" refers to any salt of a compound according to the present invention prepared from an inorganic or organic acid or base, quaternary ammonium salts and internally formed salts. Physiologically acceptable salts are particularly suitable for medical applications because of their greater aqueous solubility relative to the parent compounds. Such salts must clearly have a physiologically acceptable anion or cation. Suitably physiologically acceptable salts of the compounds of the present invention include acid addition salts formed with inorganic acids such as hydrochloric, hydrobromic, hydroiodic, phosphoric, metaphosphoric, nitric and sulfuric acids, and with organic acids, such as tartaric, acetic, trifluoroacetic, citric, malic, lactic, fumaric, benzoic, formic, propionic, glycolic, gluconic, maleic, succinic, camphorsulfuric, isothionic, mucic, gentisic, isonicotinic, saccharic, glucuronic, furoic, glutamic, ascorbic, anthranilic, salicylic, phenylacetic, mandelic, embonic (pamoic), methanesulfonic, ethanesulfonic, pantothenic, stearic, sulfinilic, alginic, galacturonic and arylsulfonic, for example benzenesulfonic and p-toluenesulfonic, acids; base addition salts formed with alkali metals and alkaline earth metals and organic bases such as N,N-dibenzylethylenediamine, chloroprocaine, choline, diethanolamine, ethylenediamine, meglumaine (N-methylglucamine), lysine and procaine; and internally formed salts. Salts having a non-physiologically acceptable anion or cation are within the scope of the invention as useful intermediates for the preparation of physiologically acceptable salts and/or for use in non-therapeutic, for example, *in vitro,* situations.

As used herein, the term "solvate" refers to a complex of variable stoichiometry formed by a solute (in this invention, a compound of formula (1) or formula (1a), or a salt or physiologically functional derivative thereof) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include water, ethanol and acetic acid. Most preferably the solvent used is water.

As used herein, the term "physiologically functional derivative" refers to any pharmaceutically acceptable derivative of a compound of the present invention, for example, an ester or an amide, which upon administration to a mammal is capable of providing (directly or indirectly) a compound of the present invention or an active metabolite thereof. Such derivatives are clear to those skilled in the art, without undue experimentation, and with reference to the teaching of Burger's Medicinal Chemistry And Drug Discovery, 5th Edition, Vol 1: Principles and Practice, which is incorporated herein by reference to the extent that it teaches physiologically functional derivatives.

The compounds of formulae (I) and (Ia) have the ability to crystallise in more than one form, a characteristic, which is known as polymorphism, and it is understood that such polymorphic forms ("polymorphs") are within the scope of formulae (I) and (Ia). Polymorphism generally can occur as a response to changes in temperature or pressure or both and can also result from variations in the crystallisation process. Polymorphs can be distinguished by various physical characteristics known in the art such as x-ray diffraction patterns, solubility, and melting point.

Certain of the compounds described herein may exist in stereoisomeric forms (i.e. they may contain one or more asymmetric carbon atoms or may exhibit *cis-trans* isomerism). The individual stereoisomers (enantiomers and diastereoisomers) and mixtures of these are included within the scope of the present invention. Likewise, it is understood that compounds of formulae (I) and (Ia) may exist in tautomeric forms other than that shown in the formulae and these are also included within the scope of the present invention.

As referred to above, individual enantiomers of compounds of formulae (I) and (Ia) may be prepared and an indication of the preferred stereochemistry for such enantiomers has been given. In a preferred embodiment, an optically pure enantiomer is desired. The term "optically pure enantiomer" means that the compound contains greater than about 90 % of the desired isomer by weight, preferably greater than about 95 % of the desired isomer by weight, and most preferably greater than about 99 % of the desired isomer by weight, said weight percent based upon the total weight of the isomer(s) of the compound.

It is to be understood that the following embodiments refer to compounds within the scope of both formula (I) and formula (Ia) as defined above unless specifically limited by the definition of each formula or specifically limited otherwise. It is also understood that the embodiments of the present invention described herein, including uses and compositions, are applicable to both formula (I) and formula (Ia).

The invention is related to a method of treating a mammal, including a humann suffering from or susceptible to a disorder mediated by GlyT1 which comprises administering an effective amount of a GlyT1 inhibiting compound of formula (I) or (Ia) as hereinbefore defined or a salt, solvate or a physiologically functional derivative thereof.

The disorders mediated by GlyT1 referred to herein include neurological and neuropsychiatric disorders, including psychoses such as schizophrenia, dementia and other forms of impaired cognition such as attention deficit disorders and organic brain syndromes. Other neuropsychiatric disorders include drug-induced (phencyclidine, ketamine and other dissociative anesthetics, amphetamine and other psychostimulants and cocaine) psychosis, psychosis associated with affective disorders, brief reactive psychosis, schizoaffective psychosis, and psychosis NOS, "schizophrenia-spectrum" disorders such as schizoid or schizotypal personality disorders, or illness associated with psychosis (such as major depression, manic depressive (bipolar) disorder, Alzheimer's disease and post-traumatic stress syndrome), and NMDA receptor-related disorders such as autism, depression, benign forgetfulness, childhood learning disorders and closed head injury.

Preferably, the disorders mediated by GlyT1 to be treated by the use or method as hereinbefore described are psychoses, including schizophrenia, dementia and attention deficit disorders, particularly schizophrenia.

As used herein, the term "effective amount" means that amount of a drug or pharmaceutical agent that will elicit the biological or medical response of a tissue, system, animal or human that is being sought, for instance, by a researcher or clinician.

As indicated above, certain of the compounds of formulae (I) and (la) are known compounds, e.g. within synthetic organic chemistry publications. However, several of such compounds have not previously been disclosed to have utility in medical therapy.

Accordingly, in a further aspect of the invention, there is provided a compound as defined above under Examples of preferred compounds of the invention and salts, solvates, esters and amides thereof for use in therapy, but not including
N-[3-(2,4-dimethyl-1-pyrrolidinyl)-2-hydroxypropyl]-1-naphthalenesulfonamide,
N-[2-hydroxy-3-(2-methyl-1-plperidinyl)propyl]-1-naphthalenesulfonamide,
N-[2-hydroxy-3-(1-piperidinyl)propyl]-1-naphthalenesulfonamide, and
N-[2-hydroxy-3-(1-pyrrolidinyl)propyl]-1-naphthalenesulfonamide,
and salts thereof.

Compounds for use according to the invention may be administered as the raw material but the active ingredients are preferably provided in the form of pharmaceutical compositions.

Accordingly, in a further aspect of the invention, there is provided a pharmaceutical composition comprising as active ingredient the compound as defined above under Examples of preferred compounds of the invention or a salt, solvate, ester or amide thereof, but not including
N-[3-(2,4-dimethyl-1-pyrrolidinyl)-2-hydroxypropyl]-1-naphthalenesulfonamide,
N-[2-hydroxy-3-(2-methyl-1-piperidinyl)propyl]-1-naphthalenesulfonamide,
N-[2-hydroxy-3-(1-piperidinyl)propyl]-1-naphthalenesulfonamide, and N-[2-hydroxy-3-(1-pyrrolidinyl)propyl]-1-naphthalenesulfonamide, and salts thereof,
and at least one pharmaceutically acceptable carrier, diluent or excipient.

These pharmaceutical compositions may be used in the treatment of clinical conditions for which a GlyT1 inhibitor is indicated such as, for example, schizophrenia. The carrier must be pharmaceutically acceptable to the recipient and must be compatible with, i.e. not have a deleterious effect upon, the other ingredients in the composition. The carrier may be a solid or a liquid and is preferably formulated with at least one compound of formula (I) or (1a) as hereinbefore described as a unit dose formulation. If desired, other physiologically active ingredients may also be incorporated in the pharmaceutical compositions of the invention.

Possible formulations include those suitable for oral, sub-lingual, buccal, parenteral (for example, subcutaneous, intramuscular, or intravenous), rectal, topical and intranasal administration and in forms suitable for administration by inhalation or insufflation (either through the mouth or nose). The most suitable means of administration for a particular patient will depend on the nature and severity of the conditions being treated and on the nature of the active compound, but, where possible, oral administration is preferred.

Formulations suitable for oral administration may be provided as discrete units, such as tablets, capsules, cachets, or lozenges, each containing a predetermined amount of the active compound; as powders or granules; as solutions or suspensions in aqueous or non-aqueous liquids; or as oil-in-water or water-in-oil emulsions.

Formulations suitable for sublingual or buccal administration include lozenges comprising the active compound and, typically, a flavoured base, such as sugar and acacia or tragacanth and pastilles comprising the active compound in an inert base, such as gelatin and glycerin or sucrose and acacia.

Formulations suitable for parenteral administration typically comprise sterile aqueous solutions containing a predetermined concentration of the active compound; the solution is preferably isotonic with the blood of the intended recipient Although such solutions are preferably administered intraveneously, they may also be administered by subcutaneous or intramuscular Injection.

Formulations suitable for rectal administration are preferably provided as unit-dose suppositories comprising the active ingredient and one or more solid carriers forming the suppository base, for example, cocoa butter.

Formulations suitable for topical or intranasal application include ointments, creams, lotions, pastes, gels, sprays, aerosols and oils. Suitable carriers for such formulations include petroleum jelly, lanolin, polyethylene glycols, alcohols, and combinations thereof.

The formulations of the invention may be prepared by any suitable method, typically by uniformly and intimately admixing the active compound(s) with liquids or finely divided solid carriers, or both, in the required proportions and then, if necessary, shaping the resulting mixture Into the desired shape.

For example, a tablet may be prepared by compressing an intimate mixture comprising a powder or granules of the active Ingredient and one or more optional Ingredients, such as a binder, lubricant, inert diluent or surface active dispersing agent, or by moulding an intimate mixture of powdered active ingredient and inert liquid diluent.

Aqueous solutions for parenteral administration are typically prepared by dissolving the active compound in sufficient water to give the desired concentration and then rendering the resulting solution sterile and isotonic.

It will be appreciated that reference to treatment is intended to include prophylaxis as well as the alleviation of established symptoms.

It will be appreciated that the precise dose administered will depend on the age and condition of the patient and the frequency and route of administration and will be at the ultimate discretion of the attendant physician. The compound may be administered in single or divided doses and may be administered one or more times, for example 1 to 4 times per day.

A proposed dose of the active ingredient for use according to the invention for oral, sub-lingual, parenteral, buccal, rectal, intranasal or topical administration to a human (of approximately 70 kg bodyweight) for the treatment of neurological and neuropsychiatric disorders mediated by a GlyT1 inhibitor, Including schizophrenia, may be about 1 to about 1000 mg, preferably about 5 to about 500 mg, more preferably about 10 to about 100 mg of the active ingredient per unit dose which could be administered, for example, 1 to 4 times per day.

As indicated above, certain of the compounds of formulae (I) and (Ia) are known compounds. However, the present invention also relates to novel compounds encompassed within the definitions of formulae (I) and (Ia).

In a further aspect of the invention, there is provided a compound as defined above under Examples of preferred compounds of the invention and salts, solvates esters and amides thereof but not including
4-amino-N-(2-hydroxy-3-piperidin-1-yl-propyl)-benzenesulfonamide,
N-[3-(2,4-dimethyl-1-pyrrolidinyl)-2-hydroxypropyl]-1-naphthalenesulfonamide,
N-[2-hydroxy-3-(2-methyl-1-piperidinyl)propyl]-1-naphthalenesulfonamide,
N-[2-hydroxy-3-(1-piperidinyl)propyl]-1-naphthalenesulfonamide, and
N-[2-hydroxy-3-(1-pyrrolidinyl)propyl]-1-naphthalenesulfonamide, and salts thereof.

The compounds of this invention may be made by a variety of methods, including standard chemistry. Any previously defined variable will continue to have the previously defined meaning unless otherwise indicated. Illustrative general synthetic methods are set out below and then specific compounds of the invention are prepared in the working Examples.

Compounds of general formula (I) or formula (Ia) may be prepared by methods disclosed in the documents hereinbefore referred to and by methods known in the art of organic synthesis as set forth in part by the following synthesis schemes. Generally, the following schemes are illustrated using compounds of formula (Ia), but it is recognised that such schemes are easily adaptable by the skilled artisan to prepare compounds of formula (I). It is also recognised that in all of the schemes described below, it Is well understood that protecting groups for sensitive or reactive groups are employed where necessary In accordance with general principles of chemistry. Protecting groups are manipulated according to standard methods of organic synthesis (T. W. Greene and P. G. M. Wuts (1991) Protecting Groups In Organic Synthesis, John Wiley & Sons). These groups are removed at a convenient stage of the compound synthesis using methods that are readily apparent to those skilled in the art. The selection of processes as well as the reaction conditions and order of their execution shall be consistent with the preparation of compounds of formula (I) or (Ia). Those skilled in the art will recognise if a stereocentre exists in compounds of formula (I) or (Ia). Accordingly, the present invention includes both possible stereoisomers and includes not only racemic compounds but the individual enantiomers as well. Where the stereochemistry is indicated as being variable at certain positions, a mixture of stereoisomers may be obtained, this mixture having been separated where indicated. Stereoisomers may be separated by high-performance liquid chromatography or other appropriate means. When a compound is desired as a single enantiomer, it may be obtained by stereospecific synthesis or by resolution of the final product or any convenient intermediate. Resolution of the final product, an intermediate, or a starting material may be effected by any suitable method known in the art. See, for example, Stereochemistry of Organic Compounds by E. L. Eliel, S. H. Wilen, and L. N. Mander (Wiley-Interscience, 1994).

Typical reaction routes for the preparation of a compound of formula (I) as hereinbefore defined, wherein R⁵, R⁶, R⁷, R⁸ and R⁹ are hydrogen, are shown in Schemes 1 and 2.

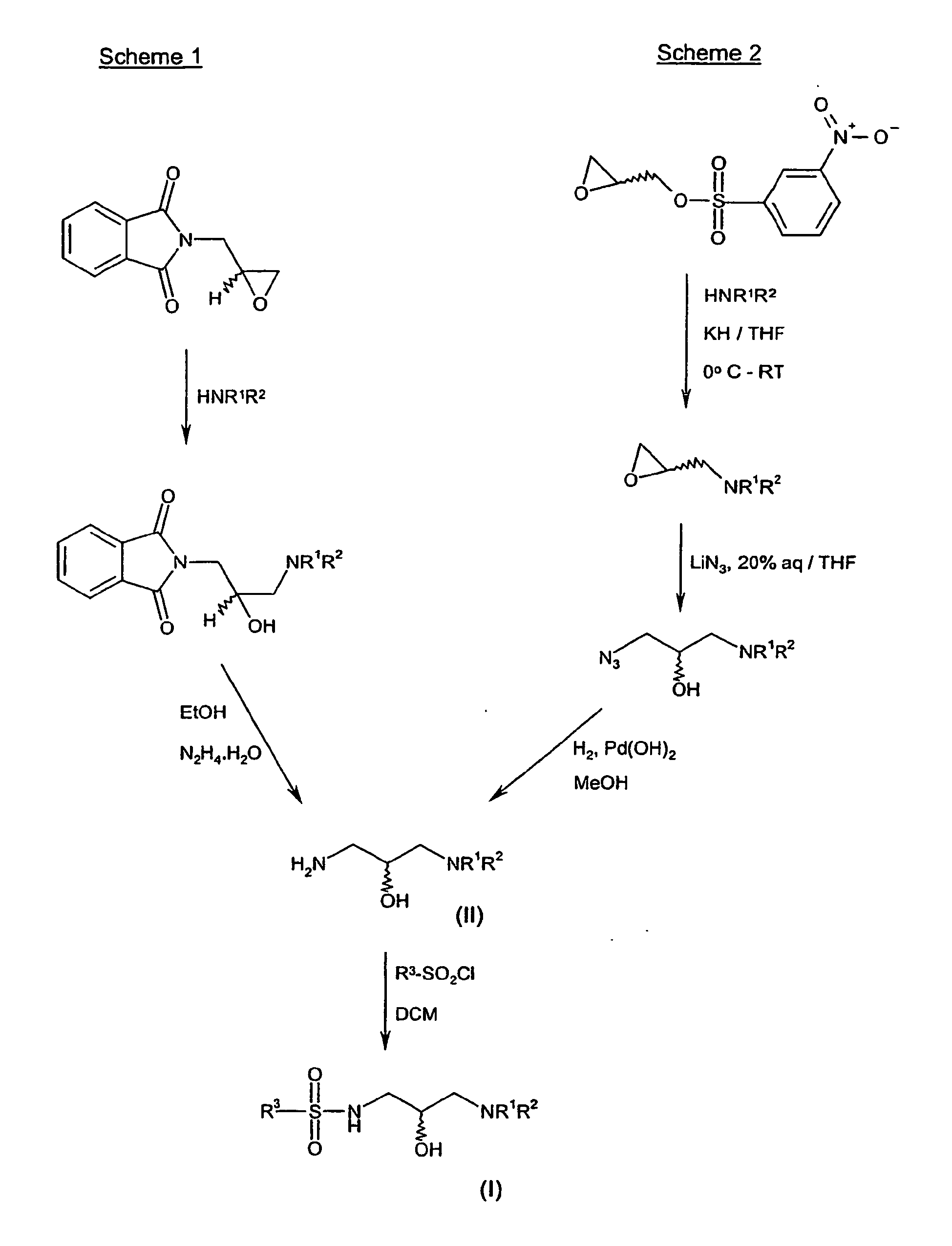

In Scheme 1, the compounds of formula (I) may be prepared using methodology similar to that described by Gutcait A. et al., Tetrahedron Asymmetry, 1996, 7(6), 1641-1648.

In Scheme 2, the reduction of the azide may be carried out using all methods known to those skilled in the art, for example, hydrogenation in the presence of catalyst such as palladium on carbon, Pd(OH)₂ and those known in the art, see for example March, Advanced Organic Chemistry, 4th edition, Wiley Interscience. The reduction of the azide is preferably carried out by hydrogenation in the presence of a catalyst such as palladium on carbon.

As indicated above, Schemes 1 and 2 can be adapted to prepare compounds wherein R⁴, R⁵, R⁶, R⁷, R⁸ and R⁹ are other than hydrogen.

Thus, in a further aspect of the invention, there is provided a process for the preparation of the compound of formula (I) as hereinbefore defined by reacting a compound of formula (II) 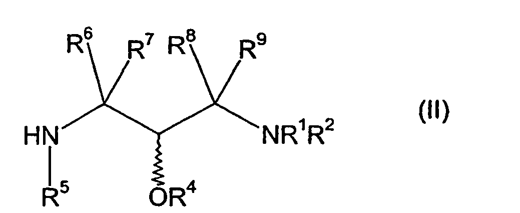 wherein R⁴, R⁵, R⁶, R⁷, R⁸ and R⁹ are as hereinbefore defined, with a compound of formula (III)

R³SO₂L (III)

wherein R³ is as hereinbefore defined and L is a suitable leaving group, such a, for example, a halogen, preferably chlorine.

The invention is further illustrated by the following non-limiting examples.

### Examples

As used herein the symbols and conventions used in these processes, schemes and examples are consistent with those used in the contemporary scientific literature, for example, the *Journal of the American Chemical Society* or the *Journal of Biological Chemistry.* Unless otherwise noted, all starting materials were obtained from commercial suppliers and used without further purification. Specifically, the following abbreviations may be used in the examples and throughout the specification:

| | |
|---|---|
| g (grams); | mg (milligrams); |
| L (litres); | ml (millilitres); |
| µL (microlitres); | psi (pounds per square inch); |
| M (molar); | mM (millimolar); |
| i. v. (intravenous); | Hz (Hertz); |
| MHz (megahertz); | mol (moles); |
| mmol (millimoles); | RT (room temperature); |
| min (minutes); | h (hours); |
| mp (melting point); | TLC (thin layer chromatography); |
| Tᵣ (retention time); | RP (reverse phase); |
| MeOH (methanol); | I-PrOH (isopropanol); |
| TEA (triethylamine); | TFA (trifluoroacetic acid); |
| TFAA (trifluoroacetic anhydride); | THF (tetrahydrofuran); |
| DMSO (dimethylsulfoxide); | EtOAc (ethyl acetate); |
| DME (1,2-dimethoxyethane); | DCM (dichloromethane); |
| DCE (dichloroethane); | DMF (*N*,*N*-dimethylformamide); |
| DMPU (*N*,*N'*-dimethylpropyleneurea); | (CDI (1,1-carbonyldiimidazole); IBCF |
| (isobutyl chloroformate); HOAc | (acetic acid); |
| HOSu (*N*-hydroxysuccinimide); | HOBT (1-hydroxybenzotriazole); |
| mCPBA (meta-chloroperbenzoic acid; | EDC (ethylcarbodiimide hydrochloride); BOC |
| (*tert*-butyloxycarbonyl); | FMOC (9-fluorenylmethoxycarbonyl); |
| DCC (dicyclohexylcarbodiimide); | CBZ (benzyloxycarbonyl); |
| Ac (acetyl); | atm (atmosphere); |
| TMSE (2-(trimethylsilyl)ethyl); | TMS (trimethylsilyl); |
| TIPS (triisopropylsilyl); | TBS (t-butyldimethylsilyl); |
| Me (methyl); | |
| HPLC (high pressure liquid chromatography); | |
| Et (ethyl); | tBu (tert-butyl). |

All references to ether are to diethyl ether, brine refers to a saturated aqueous solution of NaCl. Unless otherwise indicated, all temperatures are expressed in °C (degrees Centigrade). All reactions conducted under an inert atmosphere at room temperature unless otherwise noted.

¹H NMR spectra were recorded on a Bruker B-ACS 60 400MHz, Bruker DPX 400 or a Bruker DPX 250. Chemical shifts are expressed in parts per million (ppm, δ units). Coupling constants are in units of hertz (Hz). Splitting patterns describe apparent multiplicities and are designated as s (singlet), d (doublet), t (triplet), q (quartet), dd (double doublet), dt (double triplet), m (multiplet), br (broad).

Low-resolution mass spectra (MS) were recorded on a HP1100 series spectrometer; MS and liquid chromatography MS were recorded on a Micromass MS2 Platform LC spectrometer. All mass spectra were taken under electrospray ionisation (ESI), chemical ionisation (CI), electron impact (EI) or by fast atom bombardment (FAB) methods. All reactions were monitored by thin-layer chromatography on 0.25 mm E. Merck silica gel plates (60F-254), visualised with UV light, 5% ethanolic phosphomolybdic acid or p-anisaldehyde solution. Flash column chromatography was performed on silica gel (230-400 mesh, Merck).

The following descriptions set out the syntheses of intermediates particularly useful in the synthesis of compounds of formulae (I) and (1a).

### Description 1

### General description exemplified for (R)-1-azido-3-pyrrolidin-1-ylpropan-2-ol (D1).

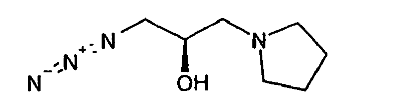

The title compound was prepared using the method of Dhanoa et al, Tet. Lett., 33 (13) p.1725-8, (1992). Pyrrolidine (0.41 ml; 5mmol) was added to a suspension of potassium hydride (35% in mineral oil) (630mg; 5.5mmol) in anhydrous tetrahydrofuran (THF) (20ml) under argon. After stirring for 2h, a solution of (S)-(+)-glycidyl nosylate (1.19g; 4.6mmol) in anhydrous THF (5ml) was added over 2 minutes. The mixture was stirred overnight at ambient temperature, then filtered through kieselguhr and washed with THF. Finally, the mixture was concentrated under reduced pressure to approximately a quarter of the original volume (n.b. use cold water bath - volatile product). The resulting THF solution of crude epoxide was treated with lithium azide (20% solution in water) (5 eq; 6.2ml) then heated at 70° overnight. The cooled solution was evaporated under reduced pressure and the residue treated with saturated aqueous NaHCO₃ solution and extracted into ethyl acetate (x 2). The organic phase was dried (Na₂SO₄) and evaporated under reduced pressure to a light brown oil. Purified by column chromatography, eluting with methanol/dichloromethane (2.5%, 4%) to afford the pure hydroxy azide as a pale orange oil (168mg, 21% over two steps). MH⁺ 171.

### Description 2

### General description exemplified for (S)-1-amino-3-pyrrolidin-1-ylpropan-2-ol (D2)

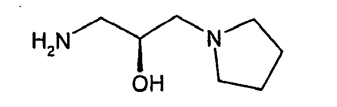

A solution of (R)-1-azido-3-pyrrolidin-1-ylpropan-2-ol (D1) (168mg; 0.988mmol) in methanol (10ml) was hydrogenated at atmospheric pressure and ambient temperature over Pearlmans' catalyst (130mg) overnight. The catalyst was removed by filtration through kieselguhr, and the filtrate evaporated under reduced pressure, to yield the title compound as a clear colourless oil (130mg; 91 %).

### Description 3

### (R)-1-Azido-3-(2-methylpyrrolidin-1-yl)propan-2-ol (D3)

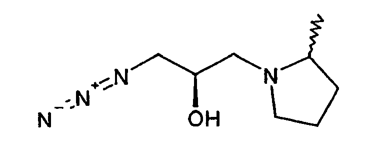

The title compound was prepared from 2-methylpyrrolidine (430mg; 5.06mmol) using the method outlined in Description 1 (119mg; 14% over 2 steps). MH⁺ 185. ¹H NMR (400MHz, CDCl₃) δ: 3.86-3.78 (m, 1H), 3.4-3.31 (m, 1H), 3.29-3.13 (m,2H), 3.03-2.57 (m,2H), 2.53-2.42 (m, 1H), 2.2-2.11 (m,1H), 1.98-1.89 (m,1H), 1.83-1.67 (m,2H), 1.45-1.35 (m,1H), 1.09 (t,3H).

### Description 4

### (S)-1-Amino-3-(2-methylpyrrolidin-1-yl)propan-2-ol (D4).

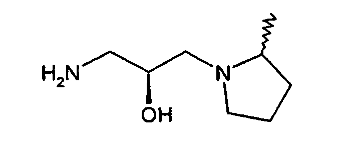

The title compound was obtained from (R)-1-azido-3-(2-methylpyrrolidin-1-yl)propan-2-ol (D3) (119mg; 0.646mmol) using the method outlined in Description 2 (80mg; 78%) MH⁺ 159.

### Description 5

### 2,4-Dimethylpyrrolidine (D5)

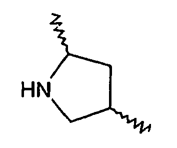

The title compound was prepared by the method of DeJong and Wibaut, Recl. Trav. Chim. Pays-Bas., 49 p.237-46 (1930). A solution of 2,4-dimethylpyrrole (10g; 0.105mol) in glacial acetic acid (500ml) was hydrogenated at atmospheric pressure and ambient temperature over platinum oxide (2g). After 4h, a further 2g platinum oxide was added and the reaction continued overnight. The catalyst was removed by filtration, and the resulting filtrate was basified strongly with potassium hydroxide pellets (with addition of ice and external card-ice/acetone cooling). The product was obtained by steam distillation of the basic solution, followed by extraction with diethyl ether. The organic phase was dried (Na₂SO₄) and evaporated under reduced pressure (n.b. use cold water bath - volatile product) to leave the crude product which was further purified by bulb-to-bulb distillation as a clear, colourless oil. (5g; 48%). ¹H NMR (400MHz, CDCl₃) δ: 3.2-3.1 (m,1H), 3.05-2.99 (m, 1H), 2.56-2.5 (m, 1H), 2.22-2.13 (m, 1H), 2.11-2.03 (m,1H), 1.15 (d,3H), 1.00 (d,3H), 0.88-0.79 (m,1H).

### Description 6:

### (R)-1-Azido-3-(2,4-dimethylpyrrolidin-1-yl)propan-2-ol (D6)

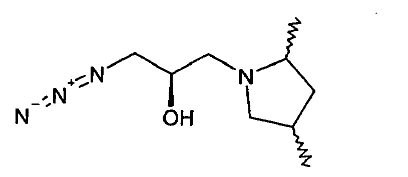

The title compound was prepared from 2,4-dimethylpyrrolidine (D5) (193mg;1.94mmol) using the method outlined in Description 1 (91mg; 26% over 2 steps) MH⁺ 199.

### Description 7:

### (S)-1-Amino-3-(2,4-dimethylpyrrolidin-1-yl)propan-2-ol (D7)

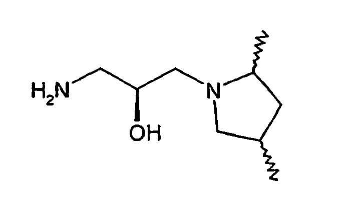

The title compound was prepared from (R)-1-azido-3-(2,4-dimethylpyrrolidin-1-yl)propan-2-ol (D6) (91 mg, 0.459mmol) using the method outlined in Description 2 (69mg; 87%). MH⁺ 173.

### Description 8

### (R)-1-Azido-3-(2-isopropylpyrrolidin-1-yl)propan-2-ol (D8)

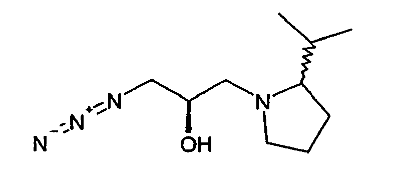

The title compound was prepared from 2-isopropylpyrrolidine (565mg; 5mmol) using the method outlined in Description 1 (379mg; 39% over 2 steps). MH⁺ 213.

### Description 9

### (S)-1-Amino-3-(2-isopropylpyrrolidin-1-yl)propan-2-ol (D9)

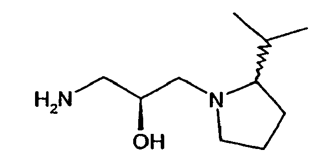

The title compound was prepared from (R)-1-azido-3-(2-isopropylpyrrolidin-1-yl)propan-2-ol (D8) (379mg; 1.78mmol) using the method outlined in Description 2 (303mg; 91%).

### Description 10

### (R)-1-Azido-3-(2,5-dimethylpyrrolidin-1-yl)-propan-2-ol (D10)

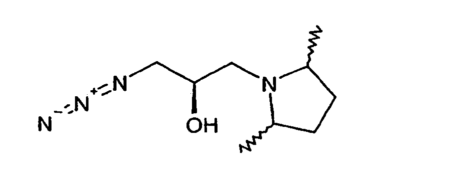

The title compound was prepared from 2,5-dimethylpyrrolidine (495mg; 5mmol) using the method outlined in Description 1 (185mg; 20% over 2 steps). MH⁺ 199.

### Description 11

### (S)-1-Amino-3-(2,5-dimethylpyrrolidin-1-yl)-propan-2-ol (D11)

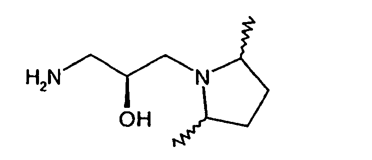

The title compound was prepared from (R)-1-azido-3-(2,5-dimethylpyrrolidin-1-yl)-propan-2-ol (D10) (185mg; 0.93mmol) using the method outlined in Description 2 (145mg; 90%).

### Description 12

### (R)-1-Azido-3-(2-cyclohexylpyrrolidin-1-yl)propan-2-ol (D12)

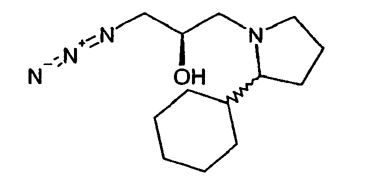

The title compound was prepared from 2-cyclohexylpyrrolidine (1.0g; 6.54mmol) using the method outlined in Description 1 (220mg; 15% over 2 steps). MH⁺ 253.

### Description 13

### (S)-1-Amino-3-(2-cyclohexylpyrrolidin-1-yl)propan-2-ol (D13)

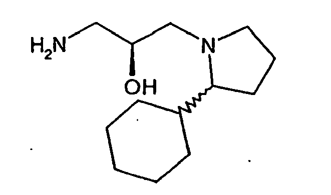

The title compound was prepared from (R)-1-azido-3-(2-cyclohexylpyrrolidin-1-yl)propan-2-ol (D12) (220mg; 0.873mmol) using the method outlined in Description 2 (177mg; 90%). MH⁺ 227.

### Description 14

### (R)-1-Azido-3-(2-isobutylpyrrolidin-1-yl)propan-2-ol (D14)

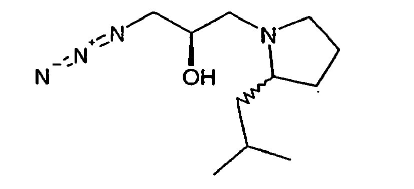

The title compound was prepared from 2-isobutylpyrrolidine (700mg; 5.5mmol) using the method outlined in Description 1 (369mg; 33% over 2 steps). MH⁺ 227.

### Description 15

### (S)-1-Amino-3-(2-isobutylpyrrolidin-1-yl)propan-2-ol (D15)

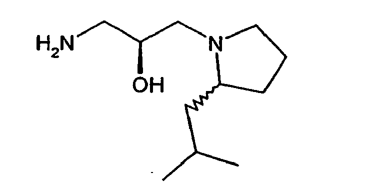

The title compound was prepared from (R)-1-azido-3-(2-isobutylpyrrolidin-1-yl)propan-2-ol (D14) (369mg; 1.6mmol) using the method outlined in Description 2 (302mg; 92%). MH⁺ 201.

### Description 16

### 2-Ethylpyrrolidine (D16)

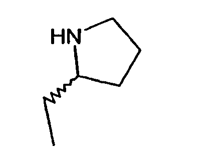

The title compound was prepared from 2-ethyl pyrrole (2.0g; 0.021 mol) using the method outlined in Description 5 (525mg; 25%). MH⁺ 100.

### Description 17

### (R)-1-Azido-3-(2-ethylpyrrolidin-1-yl)propan-2-ol (D17)

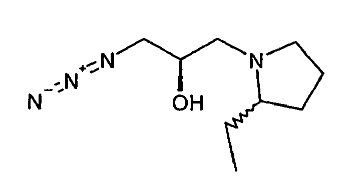

The title compound was prepared from 2-ethylpyrrolidine (D16) (525mg; 5.3mmol) using the method outlined in Description 1 (82mg; 8% over 2 steps).

### Description 18

### (S)-1-Amino-3-(2-ethylpyrrolidin-1-yl)propan-2-ol (D18)

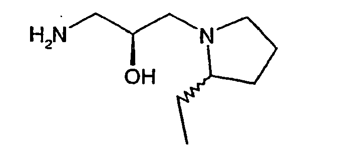

The title compound was prepared from (R)-1-azido-3-(2-ethylpyrrolidin-1-yl)propan-2-ol (D17) (82mg; 0.414mmol) using the method outlined in Description 2 (65mg; 91%).

### Description 19

### 2-tert-Butylpyrrolidine hydrochloride (D19)

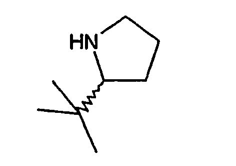

To a solution of 5-*tert-*butyl-3,4-dihydro-2*H*-pyrrole (prepared from 2-pyrrolidone using the method of Mundy et al, J.Org. Chem., 39, (13) p.1963, 1974) (200mg; 1.6mmol) in methanol (10ml) was added sodium borohydride portionwise (121 mg; 3.2mmol) and the solution was stirred at ambient temperature for 2h. 1.0M ethereal HCl (5ml) was added portionwise, and the mixture was stirred for 1 hr. The white precipitate was removed by filtration and the organic phase was evaporated under reduced pressure, to afford the title compound as a white powder (128mg; 49%). MH⁺ 128. ¹H NMR (400MHz, d4-MeOH) δ: 3.35-3.2 (m,3H), 2.12-1.95 (m, 3H), 1.83-1.72 (m,1H), 1.04 (s, 9H).

### Description 20

### (R)-1-Azido-3-(2-tert-butylpyrrolidin-1-yl)propan-2-ol (D20)

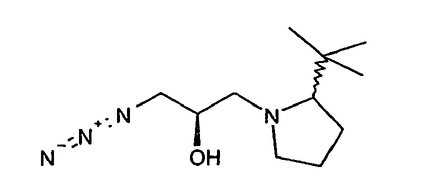

The title compound was prepared from 2-*tert*-butylpyrrolidine hydrochloride (D19) (817mg, 5mmol) using the method outlined in Description 1, (n.b. 2.6 equivalents of potassium hydride used) (282mg; 27% over 2 steps). MH⁺ 227.

### Description 21

### (S)-1-Amino-3-(2-tert-butylpyrrolidin-1-yl)propan-2-ol (D21)

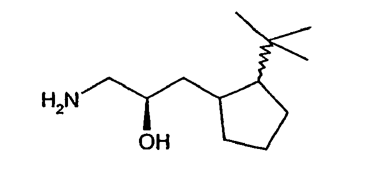

The title compound was prepared from (R)-1-azido-3-(2-*tert*-butylpyrrolidin-1-yl)propan-2-ol (D20) (282mg; 1.25mmol) using the method outlined in description 2 (112mg; 45%). MH⁺ 201.

### Description 22

### 2-cyclopropylpyrrolidine hydrochloride (D22)

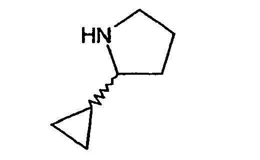

The title compound was prepared from 5-cyclopropyl-3,4-dihydro-2*H* -pyrrole (prepared from 2-pyrrolidone using the method of Mundy et al, J.Org. Chem., 39, (13) p.1963, 1974) (1.4g; 12.8mmol) using the method outlined in Description 19 (1.54g; 81%). MH⁺ 112.

### Description 23

### (R)-1-Azido-3-(2-cyclopropylpyrrolidin-1-yl)propan-2-ol (D23)

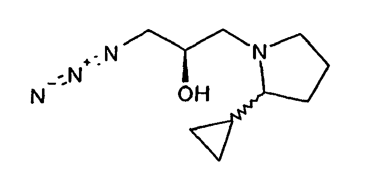

The title compound was prepared from 2-cyclopropylpyrrolidine hydrochloride (D22) (1.54g; 0.01 mol) using the method outlined in Description 1 (n.b. 2.6 equivalents of potassium hydride used) (420mg; 21% over 2 steps). MH⁺ 211.

### Description 24

### (S)-1-Amino-3-(2-cyclopropylpyrrolidin-1-yl)propan-2-ol (D24)

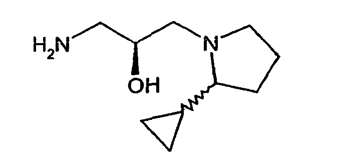

The title compound was prepared from (R)-1-azido-3-(2-cyclopropylpyrrolidin-1-yl)propan-2-ol (D23) (420mg; 20mmol) using the method outlined in Description 2 (336mg; 91%). MH⁺ 185.

### Description 25

### (R)-1-Azido-3-(3-methylpiperidin-1-yl)propan-2-ol (D25)

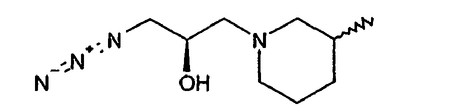

The title compound was prepared from 3-methylpiperidine (0.5g; 5mmol) using the method outlined in Description 1 (404mg; 44% over 2 steps). MH⁺ 199.

### Description 26

### (S)-1-Amino-3-(3-methylpiperidin-1-yl)propan-2-ol (D26)

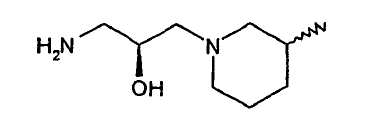

The title compound was prepared from (R)-1-azido-3-(3-methylpiperidin-1-yl)propan-2-ol (D25) (404mg; 2.04mmol) using the method outlined in Description 2 (310mg; 88%). MH⁺ 173.

### Description 27

### (R)-1-Azido-3-(5-ethyl-2-methylpiperidin-1-yl)propan-2-ol (D27)

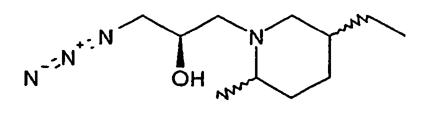

The title compound was prepared from 5-ethyl-2-methylpiperidine (635mg; 5mmol) using the method outlined in Description 1 (244mg; 23% over 2 steps). MH⁺ 227.

### Description 28

### (S)-1-Amino-3-(5-ethyl-2-methylpiperidin-1-yl)propan-2-ol (D28)

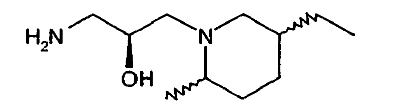

The title compound was prepared from (R)-1-azido-3-(5-ethyl-2-methylpiperidin-1-yl)propan-2-ol (D27) (244mg; 0.108mmol) using the method outlined in Description 2 (140mg; 65%).

### Description 29

### (R)-1-Azido-3-(2-ethylpiperidin-1-yl)propan-2-ol (D29)

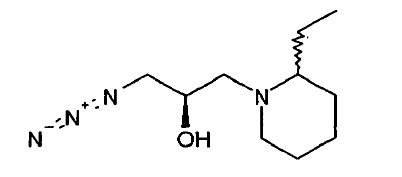

The title compound was prepared from 2-ethylpiperidine (0.67ml ; 5.05mmol) using the method outlined in Description 1 (255mg; 26% over 2 steps). MH⁺ 213.

### Description 30

### (S)-1-Amino-3-(2-ethylpiperidin-1-yl)propan-2-ol (D30)

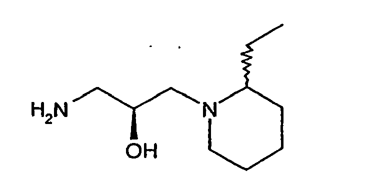

The title compound was prepared from (R)-1-azido-3-(2-ethylpiperidin-1-yl)propan-2-ol (D29) (255mg; 1.2mmol) using the method outlined in Description 2 (188mg; 84%). MH⁺ 187.

### Description 31

### 2-lsopropylpiperidine (D31)

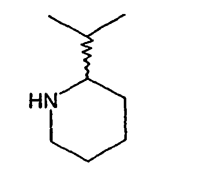

The title compound was prepared from 2-isopropylpyridine (10g; 0.083mol) using the method outlined in Description 5 (7.3g; 70%). MH⁺ 128.

### Description 32

### (R)-1-Azido-3-(2-isopropylpiperidin-1-yl)propan-2-ol (D32)

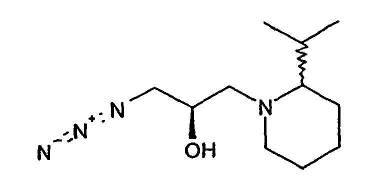

The title compound was prepared from 2-lsopropylpiperidine (D31) (641mg; 5.05mmol) using the method outlined in Description 1 (80mg; 8% over 2 steps). MH⁺ 227.

### Description 33

### (S)-1-Amino-3-(2-isopropylpiperidin-1-yl)propan-2-ol (D33)

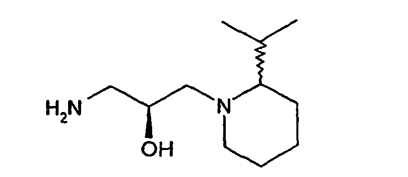

The title compound was prepared from (R)-1-azido-3-(2-isopropylpiperidin-1-yl)propan-2-ol (D32) (80mg; 0.353mmol) using the method outlined in Description 2 (50mg; 71%).

### Description 34

### (R)-Azido-(sec-butylpropylamino)propan-2-ol (D34)

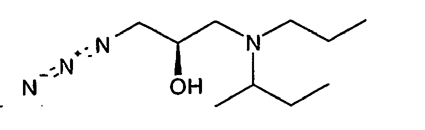

The title compound was prepared from N-n-propyl-N-sec-butylamine (575mg; 5mmol) using the method outlined in Description 1 (133mg; 14% over 2 steps). MH⁺ 215.

### Description 35

### (S)-Amino-(sec-butylpropylamino)propan-2-ol (D35)

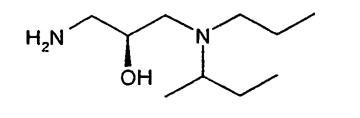

The title compound was prepared from (R)-azido-(sec-butylpropylamino)propan-2-ol (D34) (133mg; 0.62mmol) using the method outlined in Description 2 (79mg; 68%). MH⁺ 189.

### Description 36

### tert-butylpropylamine (D36)

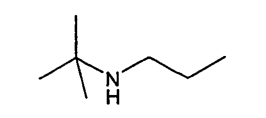

A solution of *tert*-butylamine 12.28g; 0.168mol) and n-propyl bromide (10.34g; 0.084mol) in N,N-dimethylformamide (30ml) was heated at gentle reflux overnight. On cooling, the crude product crystallised as the hydrobromide salt, and was was triturated with diethyl ether and filtered, to give a white crystalline solid. (11.45g; 70%). The salt was free-based by dissolving in 15% aq. sodium hydroxide solution, and removing the upper oily layer. Bulb-to-bulb distillation gave the pure product as a colourless oil. ¹H NMR (400MHz, d4-MeOH) δ: 2.49 (t,2H), 1.53-1.42 (m,2H), 1.1 (s,9H), 0.95 (t,3H).

### Description 37

### (R)-Azido-(tert butylpropylamino)propan-2-ol (D37)

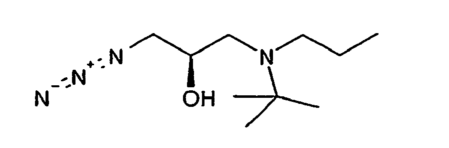

The title compound was prepared from *tert*-butylpropylamine (D36) (632mg; 5.5mmol) using the method outlined in Description 1 (40mg; 4% over 2 steps).

### Description 38

### (S)-Amino-(tert-butylpropylamino)propan-2-ol (D38)

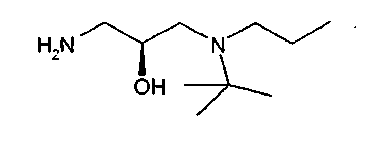

The title compound was prepared from (R)-azido-(*tert*-butylpropylamino)propan-2-ol (D37) (40mg; 0.187mmol) using the method outlined in Description 2 (32mg; 91%). MH⁺ 189.

### Description 39

### (R)-1-Azido-3-(1,3-dihydroisoindol-2-yl)propan-2-ol (D39)

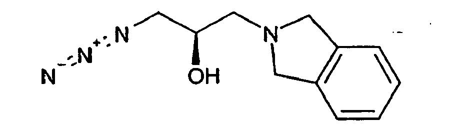

The title compound was prepared from 2,3-dihydro-1*H*-isoindole (595mg;5mmol) using the method outlined in Description 1 (484mg; 48% over 2 steps). MH⁺ 219.

### Description 40

### (S)-1-Amino-3-(1,3-dihydroisoindol-2-yl)propan-2-ol (D40)

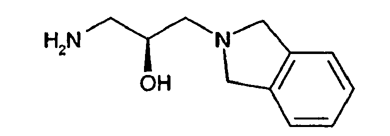

The title compound was prepared from (R)-1-azido-3-(1,3-dihydroisoindol-2-yl)propan-2-ol (D39) (484mg; 2.2mmol) using the method outlined in Description 2 (396mg; 93%). MH⁺ 193.

### Description 41

### (R)-1-Azido-3-(octahydroisoquinolin-1-yl)propan-2-ol (D41)

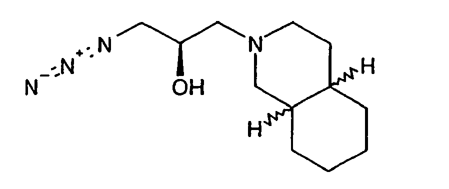

The title compound was prepared from decahydroisoquinoline (695mg; 5mmol) using the method outlined in Description 1 (336mg; 31 % over 2 steps). MH⁺ 239.

### Description 42

### (S)-1-Amino-3-(octahydroisoquinolin-1-yl)propan-2-ol (D42)

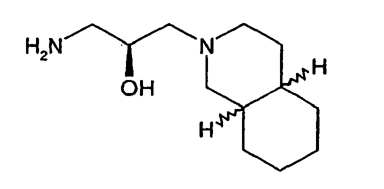

The title compound was prepared from (R)-1-azido-3-(octahydroisoquinolin-1-yl)propan-2-ol (D41) (336mg; 1.41mmol) using the method outlined in Description 2 (267mg; 89%). MH⁺ 213.

### Description 43

### (R)-1-Azido-3-(octahydroquinolin-1-yl)propan-2-ol (D43)

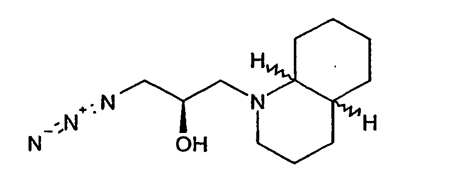

The title compound was prepared from decahydroquinoline (910mg; 6.54mmol) using the method outlined in Description 1 (346mg; 25% over 2 steps). MH⁺ 239.

### Description 44

### (S)-1-Amino-3-(octahydroquinolin-1-yl)propan-2-ol (D44)

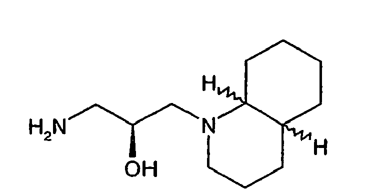

The title compound was prepared from (R)-1-azido-3-(octahydroquinolin-1-yl)propan-2-ol (D43) (346mg; 1.45mmol) using the method outlined in Description 2 (288mg; 93%). MH⁺ 213.

### Description 45

### (R)-1-Azido-3-((1S,5R)-1,3,3-trimethyl-6-azabicydo[3.2.1]oct-6-yl)propan-2-ol (D45)

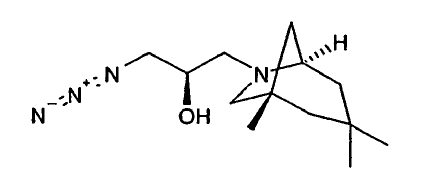

The title compound was prepared from (1S,5R)-1,3,3-trimethyl-6-azabicyclo[3.2.1]octane (0.857ml; 5.05mmol) using the method outlined in Description 1 (213mg; 18% over 2 steps). MH⁺ 253.

### Description 46

### (S)-1-Amino-3-((1S,5R)-1,3,3-trimethyl-6-azabicyclo[3.2.1]oct-6-yl)propan-2-ol (D46)

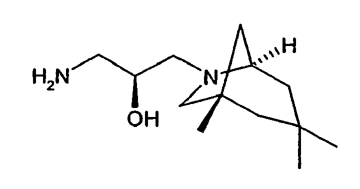

The title compound was prepared from (R)-1-azido-3-((1S,5R)-1,3,3-trimethy)-6-azabicyclo[3.2.1]oct-6-yl)propan-2-ol (D45) (213mg; 0.845mmol) using the method outlined in Description 2 (153mg; 80%). MH⁺ 227.

### Description 47

### (R)-1-Amino-3-(2,4-dimethylpyrrolidin-1-yl)propan-2-ol (D47)

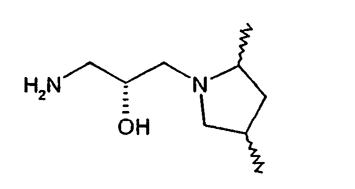

The title compound was prepared from 2,4-dimethylpyrrolidine (193mg;1.94mmol) and (R)-(-)-glycidyl nosylate (460mg; 1.77mmol) using the methods outlined in Descriptions 1 and 2 (71 mg; 23% over three steps). MH⁺ 173.

### Description 48

### 1-(R)-1-Oxiranylmethyl-piperidine (D48)

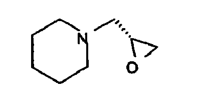

Potassium hydride (527mg; 30% in oil) was weighed into an oven-dried 3-necked flask and THF (5ml) was added under an atmosphere of argon. A solution of piperidine (0.44ml) in THF (5ml) was added to the stirred suspension. The reagents were stirred together at room temperature for 2 hrs, under an atmosphere of argon. S-(-)-Glycidyl nosylate (904mg) was then added dropwise as a solution in THF (5ml) and the reaction mixture stirred at room temperature overnight. The reaction mixture was filtered under an inert atmosphere through Celite^{™} and the liquor reduced to half volume in vacuo, m/z (API⁺) 142 [MH⁺].

### Description 49

### (R)-1-Azido-3-piperidin-1-yl-propan-2-ol (D49)

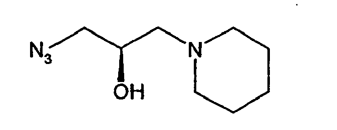

To the product solution from Description 48 was added lithium azide (5.34ml; 20% solution in water) and the reaction mixture heated at 70°C overnight. The THF was then removed in vacuo and the solution partitioned between EtOAc and NaHCO₃ solution. Organics were collected, dried (MgSO₄), and evaporated. The residual oil was chromatographed over silica gel, eluting with a gradient of 0-10% [9:1 MeOH:NH₃] in dichloromethane. The title compound was obtained as a colourless oil (348mg), m/z (API⁺) 185 [MH⁺].

### Description 50

### (S)-1-Amino-3-piperidin-1-yl-propan-2-ol (D50)

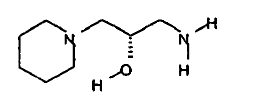

(R)-1-Azido-3-piperidin-1-yl-propan-2-ol (340mg) was dissolved in ethanol (25ml) and hydrogenated at 50 psi and room temperature in the presence of 10% Pd/C catalyst (350mg). After 2hrs the reaction mixture was filtered and evaporated. The title compound was obtained as a colourless oil (117mg), m/z (API⁺) 159 [MH⁺].

### Description 51

### (2R,S)-Methyl-1-[(R)-oxiranylmethyl]piperidine (D51)

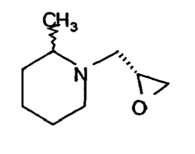

Potassium hydride (527mg; 30% in oil) was weighed into an oven dried 3-necked flask and stirred in THF (5ml) under an inert atmosphere during the addition of a solution of (2R,S)-methylpiperidine (0.52ml) in THF (5ml). The reaction solution was stirred at room temperature for 2 hrs. S-(-)-Glycidyl nosylate (904mg) was then added dropwise as a solution in THF (5ml) and the reaction mixture stirred at room temperature for 16 hours. The reaction mixture was filtered under an inert atmosphere through Celite^{™} and the organic solution reduced to half volume at reduced pressure, m/z 156 (API⁺) [MH⁺].

### Description 52

### 1-Azido-3-[(2R,S)-methylpiperidin-1-yl]-propan-(2R)-ol (D52)

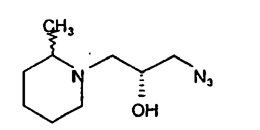

To the product solution from Description 51 was added lithium azide (5.34ml, 20% solution in water) and the reaction mixture heated at 70°C for 16 hours. The solvent was then removed at reduced pressure and the residue was partitioned between EtOAc and NaHCO₃ solution. The organic solution was dried (MgSO₄), and evaporated. The residual oil was chromatographed over silica gel, eluting with a gradient of 0-10% [9:1 MeOH:NH₃] in dichloromethane. The title compound was obtained as a colourless oil (326mg), m/z 199 (API⁺) [MH⁺].

### Description 53

### 1-Amino-3-[(2R,S)-methylpiperidin-1-yl)-propan-(2S)-ol (D53)

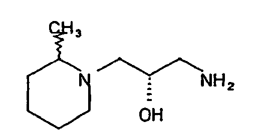

1-Azido-3-[(2R,S)-methylpiperidin-1-yl]-propan-(2R)-ol (320mg) was dissolved in EtOH (25ml) and hydrogenated at 50 psi and room temperature in the presence of 10% Pd/C catalyst (350mg). After 2hrs the reaction mixture was filtered and evaporated. Title compound was obtained as a colourless oil (215mg), m/z 173 (API⁺) [MH⁺].

### Description 54

### (2R,6S)-Dimethyl-1-[(R)-oxiranylmethyl]piperidine (D54)

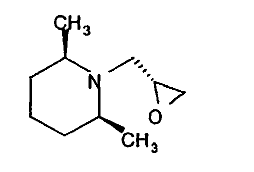

Potassium hydride (860mg; 30% in oil) was weighed into an oven dried 3-necked flask and stirred in THF (5ml) under an inert atmosphere during the addition of (2R,6S)-dimethylpiperidine (0.75ml) as a solution in THF (5ml). The suspension was stirred at room temperature for 2 hrs. S-(-)-Glycidyl nosylate (1.13g) was then added dropwise as a solution in THF (5ml) and the reaction mixture stirred at room temperature overnight. The reaction mixture was filtered under an inert atmosphere through Celite^{™} and the organic solution evaporated to half volume at reduced pressure, m/z 170 (API⁺) [MH⁺].

### Description 55

### (R)-1-Azido-3-[(2R,6S)dimethylpiperidin-1-yl]propan-2-ol (D55)

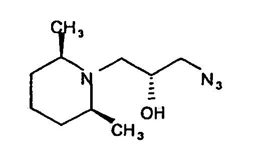

To the product solution from Description 54 was added lithium azide (5.63ml, 20% solution in water) and the reaction mixture heated at 70°C overnight. The solvent was then evaporated in vacuo and the solution partitioned between EtOAc and NaHCO₃ solution. The organic solution was dried (MgSO₄) and evaporated. The residual oil was chromatographed over silica gel, eluting with a gradient of 0-10% [9:1 MeOH:NH₃] in dichloromethane. The title compound was obtained as a colourless oil (80mg), m/z 213 (API⁺) [MH⁺].

### Description 56

### (S)-1-Amino-3-[(2R,6S)dimethylpiperidin-1-yl)-propan-2-ol (D56)

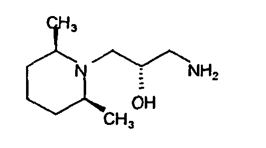

(R)-1-Azido-3-[(2R,6S)dimethylpiperidin-1-yl]propan-2-ol (80mg) was dissolved in ethanol (25ml) and hydrogenated at 50 psi and room temperature in the presence of 10% Pd/C catalyst (80mg). After 2hrs the reaction mixture was filtered and evaporated. Title compound was obtained as a colourless oil (80mg).

### Description 57

### (2S)-Ethyl-1-[(R)-oxiranylmethyl]piperidine (D57)

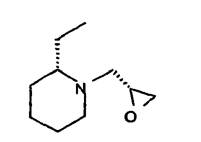

(S)-(+)-2-ethylpiperidine hydrochloride (1.00g, 6.69 mmol) (prepared by the method of J. Cymerman Craig and A. R. Pinder, J. Org. Chem., 1971, 36(23), 3648) was added portionwise to a stirred suspension of potassium hydride (2.05g, 30% in oil, 15.39mmol) in dry THF (45 ml) under argon at room temperature. The suspension was stirred for a further two hours. A solution of the (S)-glycidyl nosylate (1.73g, 6.69 mmol) in dry THF (10 ml) was then added dropwise and the resulting mixture stirred overnight. The suspension was filtered through Celite^{™} and the filtrate evaporated to half-volume under reduced pressure to yield a solution of the title compound.

### Description 58

### 1-Azido-3-[(2S)-ethylpiperidin-1-yl]-propan-(2R)-ol (D58)

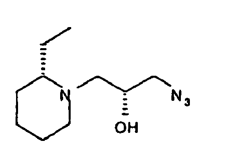

A 20% solution of lithium azide in water (8.2ml, 33.4 mmol) was added to the solution of the product from Description 57 and the mixture stirred at 70° overnight. The cooled solution was evaporated under reduced pressure and the residue partitioned between ethyl acetate and saturated sodium hydrogen carbonate solution. The organic layer was washed with further saturated sodium hydrogen carbonate solution and dried over MgSO₄. Removal of the solvent gave an oil which was chromatographed on silica gel. Elution with CHCl₃ followed by 10% MeOH in CHCl₃ afforded the title product as a yellow oil (500mg, 35%). ¹H NMR δ(CDCl₃) 0.89 (3H, t, J=7 Hz), 1.34-1.69 (9H, overlapping m), 2.1-2.25 (2H, overlapping m), 2.71 (1 H, dd, J=12.4 and 10.4 Hz), 2.93 (1 H, m), 3.19 (1 H, dd, J=12.8 and 5.6 Hz), 3.37 (1H, dd, J=12.8 and 4.0Hz), 3.85 (1 H, m) ppm.

### Description 59

### 1-Amino-3-[(2S)-ethylpiperidin-1-yl)-propan-(2S)-ol (D59)

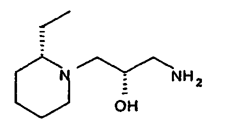

Hydrogenation of the azide from Description 58 (500mg, 2.36 mmol) over 10% palladium on charcoal (100mg) in methanol (25ml) at ambient temperature and pressure for 3 hours and subsequent removal of the catalyst by filtration through celite gave, after removal of the solvent under reduced pressure, the crude amine (2.36 mmol) which was used without further purification.

### Description 60

### (S)-1-Azido-3-(2-methylpyrrolidin-1-yl)propan-2-ol (D60)

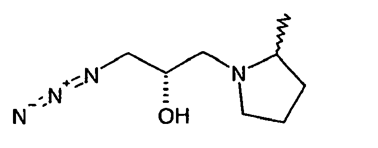

The title compound was prepared from 2-methylpyrrolidine (155mg;1.82mmol) and (R)-(-)-glycidyl nosylate (429mg;1.6mmol) using the method outlined in Description 1 (76mg; 25% over two steps). MH⁺ 185.

### Description 61

### (R)-1-Amino-3-(2-methylpyrrolidin-1-yl)propan-2-ol (D61)

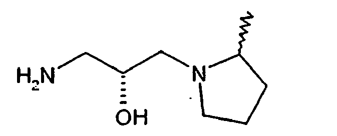

The title compound was prepared from (S)-1-azido-3-(2-methylpyrrolidin-1-yl)propan-2-ol (D60) (63mg; 0.34mmol) using the method outlined in Description 2 (50mg; 92%). MH⁺ 159.

### Description 62

### (S)-1-Azido-3-(3-methylpiperidin-1-yl)propan-2-ol (D62)

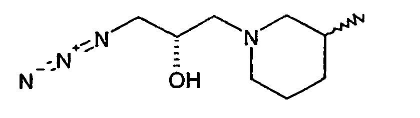

The title compound was prepared from 3-methylpiperidine (366mg; 3.7mmol) and (R)-(-)-glycidyl nosylate (871mg; 3.4mmol) using the methods outlined in Description 1 (220mg; 33% over two steps).

### Description 63

### (R)-1-Amino-3-(3-rnethylpiperidin-1-yl)propan-2-ol (D63)

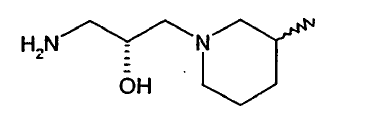

The title compound was prepared from (S)-1-azido-3-(3-methylpiperidin-1-yl)propan-2-ol (D62) (160mg; 0.81 mmol) using the method outlined in Description 2 (130mg; 94%).

### Description 64.

### 2-Oxiranylmethyl-isoindole-1,3-dione (D64)

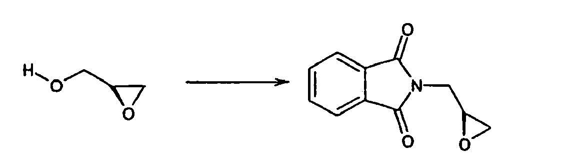

To a stirred solution of phthalimide (735mg) in THF (30ml) was added triphenylphosphine (1.310g) and (S)-glycidol (0.38ml). This solution was cooled using an external ice bath for the dropwise addition of diethylazodicarboxyate (0.8ml). The reaction mixture was stirred at room temperature overnight. The reaction mixture was then concentrated, and stirred in diethyl ether for 2h. White precipitate was filtered and liquor was evaporated. The residual yellow oil was chromatographed over silica gel, eluting with ethyl acetate/hexane (1:1). Title compound was obtained as a white solid (815mg), m/z 204 [MH⁺].

### Description 65.

### 2,6-Diethylpyridine (D65)

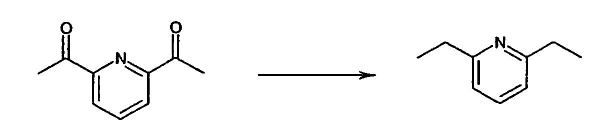

2,6-Diacetylpyridine (2g) was dissolved in diethyleneglycol (50ml) with hydrazine monohydrate (3.6ml). The reaction mixture was heated at 170°C for 30 min. The reaction mixture was then cooled to room temperature for the addition of potassium hydroxide (3.1g), then heated at 200°C for 2h when the reaction mixtue lost all colour. The reaction mixture was then cooled to room temperature and poured onto water. The mixture was extracted with ether, organics combined, washed with brine, dried (MgSO₄) and concentrated to give desired product (1.5g, 93%), δ_{H} (CDCl₃) 1.29 (6H, t, J 7.6Hz), 2.76-2.82 (4H, q, J 7.6Hz), 6.96 (2H, d, J 7.6Hz), 7.50 (1H, t, J 7.6Hz).

### Description 66.

### 2,6-Diethylpiperidine (D66)

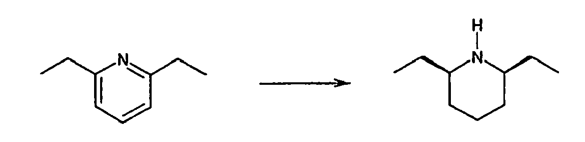

2,6-Diethylpyridine (D65) was hydrogenated in acetic acid with platinum oxide overnight. The reaction mixture was filtered through celite, washed with a small amount of AcOH and basified with KOH and K₂CO₃ to pH 9. The emulsion was extracted with diethyl ether, dried (MgSO₄) and concentrated to give the desired product (1g, 70%), δ_{H} (CDCl₃) 0.91 (6H, m), 1.06 (2H, m), 1.41 (1H, m), 1.44 (4H, m), 1.68 (2H, m), 1.70 (1H, m), 2.44 (2H, m).

### Description 67.

### 2-[(R)-3-(2,6-Diethylpiperidin-1-yl)-2-hydroxy-propyl]-isoindole-1,3-dione (D67)

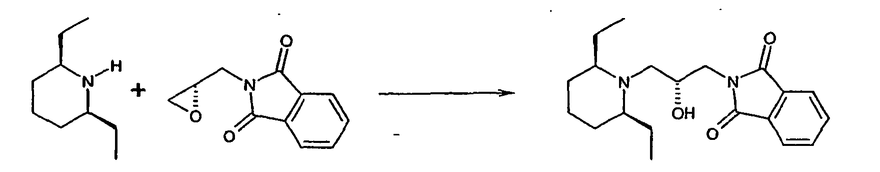

2,6-Diethylpiperidine (D66) (1g) and 2-oxiranylmethylisoindole-1,3-dione (D64) (1.4g) were heated together at 70°C overnight. After cooling to room temperature the crude mixture was chromatographed over silica gel, eluting with a gradient of 0-10% [9:1 MeOH:NH3] in dichloromethane. Title compound was obtained as an oil (90mg), m/z 345 [MH⁺]

### Description 68.

### (S)-1-Amino-3-(2,6-diethylpiperidin-1-yl)-propan-2-ol (D68)

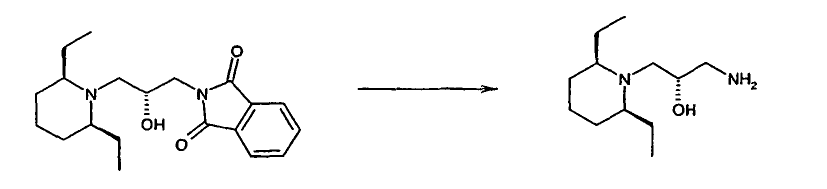

2-[(R)-3-(2,6-Diethylpiperidin-1-yl)-2-hydroxypropyl]-isoindole-1,3-dione (D67) (90 mg) was stirred in ethanol (2ml) with hydrazine monohydrate (0.06ml) overnight. White precipitate was removed by filtration and liquor concentrated. The crude mixture (70mg) containing the title compound was progressed without purification.

### Description 69.

### 2-[(R)-3-[(2R,6S)-Dimethylpiperidin-1-yl]-2-hydroxy-propyl]-isoindole-1,3-dione (D69)

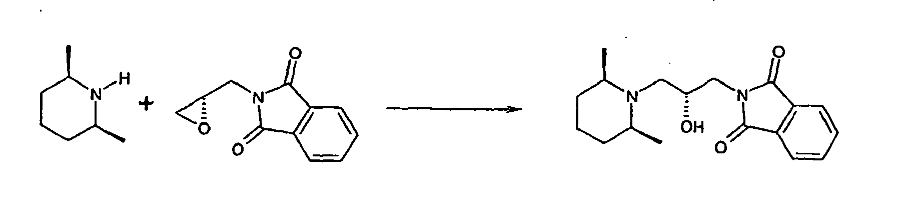

2,6-Dimethylpiperidine was reacted with 2-oxiranylmethylisoindole-1,3-dione (D64) by the procedure of Description 67. The title compound was obtained as a colourless oil.

### Description 70.

### (S)-1-Amino-3-[(2R,6S)-dimethylpiperidin-1-yl]-propan-2-ol (D70)

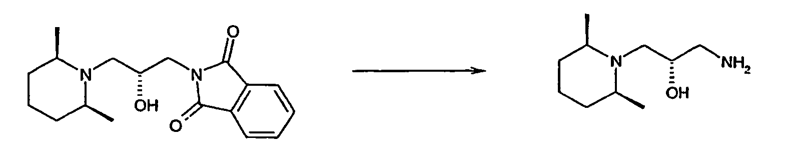

2-[(R)-3-[(2R,6S)-Dimethylpiperidin-1-yl]-2-hydroxy-propyl]-isoindole-1,3-dione (D69) was treated with hydrazine hydrate according to the procedure of Description 68 to yield the title compound as a colourless oil, which was identical to the product of Description 56.

### Description 71.

### Quinoline-5-sulfonyl chloride (D71)

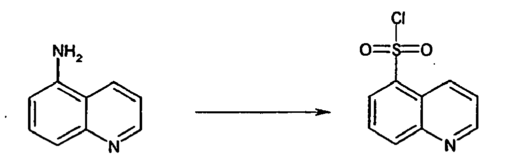

(a) Thionyl chloride (2.1 ml) was added dropwise to water (12.5ml) at 5°C. This mixture was allowed to warm to room temperature and stirred for 16h. Copper I chloride (10mg) was then added and the resulting yellow solution cooled to 0°C.
(b) Concentrated hydrochloric acid (6.75ml) was cooled to 0°C for the portionwise addition of 5-amino quinoline (1g). This was allowed to warm slightly between additions, when the reaction mixture turned red/orange. After complete addition, the reaction mixture was cooled to -5°C for the dropwise addition of a solution of NaNO₂ (0.5g) in water (2ml). After complete addition and at -5°C, the resulting mixture was added slowly to the cooled thionyl chloride/CuCl mixture from part (a).. A solid precipitated and the mixture was stirred at 0°C for 1.5h. The brick red solid was filtered and washed with water (300mg), δ_{H} (CDCl₃) 7.71 (1 H, m), 7.88 (1 H,m), 8.44 (1H,m), 8.52 (1 H,m), 9.1-9.2 (2H, m).

### Description 72.

### 5-Methoxynaphthalen-1-ylamine (D72)

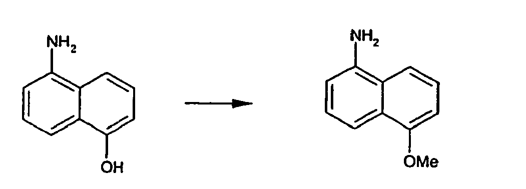

5-Amino-1-naphthol (3.0g) was dissolved in dry DMF (75ml) and cooled to 5°C under an atmosphere of argon. Sodium hydride (0.828g, 60% dispersion in oil)was added portionwise to the stirred, cooled solution over a period of 15 minutes. A solution of methyl iodide (1.17 ml) in dry DMF (5ml) was then added dropwise to the stirred, cooled suspension. The resulting suspension was then stirred at room temperature for 16 hours. The reaction solution was then partitioned between ethyl acetate and saturated sodium bicarbonate solution. The organic solution was washed with brine, dried (MgSO₄) and evaporated. Crystallisation from ethyl acetate/hexane provided the pure title compound as black needles (1.34g), δ_{H} (d₆-DMSO) 3.92 (3H,s), 5.61 (2H, broad s), 6.69 (1 H, d), 6.88 (1 H, d), 7.17 (1 H, t), 7.29 (1 H, t), 7.37 (1H, d), 7.6 (1 H, d).

### Description 73.

### 5-Methoxynaphthalene-1-sulfonyl chloride (D73)

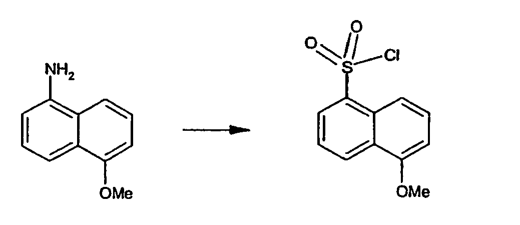

5-Methoxynaphthalen-1-ylamine (D72) was converted to the title compound by the procedure of R. V. Hoffman, Organic Syntheses, **60**, 121. Silica gel column chromatography, eluting with a gradient of 0 to 30% ethyl acetate in hexane gave the title compound as a pale yellow solid, δ_{H} (CDCl₃) 4.09 (3H, s) 7.0 (1 H, d), 7.59 (1 H, t), 7.72 (1 H, t), 8.35 (1 H, d), 8.4 (1 H, d), 8.72 (1 H, d).

### Description 74.

### 5-Cyanonaphthalene-1-sulfonyl chloride (D74)

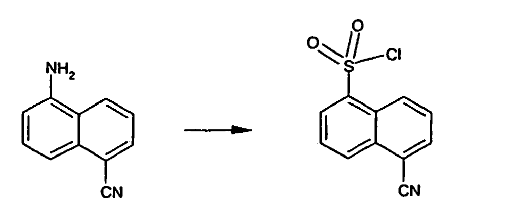

5-Cyanonaphthalen-1-ylamine was converted to the title compound by the procedure of R. V. Hoffman, Organic Syntheses, **60**, 121. Silica gel column chromatography, eluting with a gradient of 0 to 30% ethyl acetate in hexane gave the title compound as a pale yellow solid, δ_{H} (d₆-DMSO) 7.67-7.78 2H, 2 x t), 8.10 (2H, d), 8.18 (1H, d), 9.21 (1 H, d)

### Description 75.

### 4-Cyanonaphthalene-1-sulfonyl chloride (D75)

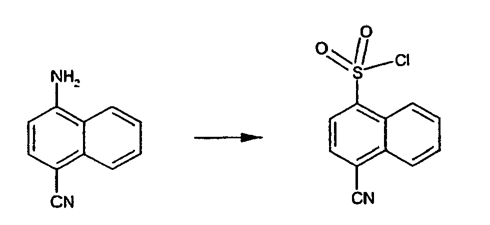

4-Cyanonaphthalen-1-ylamine was converted to the title compound by the procedure of R. V. Hoffman, Organic Syntheses, **60**,121. Silica gel column chromatography, eluting with a gradient of 0 to 50% ethyl acetate in hexane gave the title compound as a white solid, δ_{H} (d₆-DMSO) 7.70 (1 H, t), 7.78(1H, t), 8.06 (1 H, d), 8.11 (2H, d), 9.0 (1 H, d).

### Description 76.

### 4-Bromonaphthalene-1-sulfonyl chloride (D76)

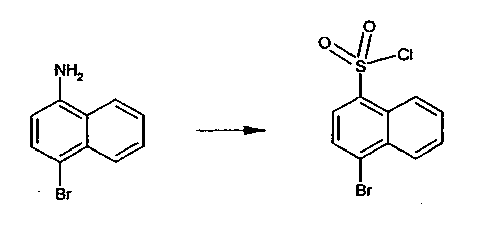

4-Bromonaphthalen-1-ylamine was converted to the title compound by the procedure of R. V. Hoffman, Organic Syntheses, **60**, 121. Silica gel column chromatography, eluting with a gradient of 0 to 30% ethyl acetate in hexane gave the title compound as a pale orange solid, δ_{H} (d₆-CDCl₃) 7.80 (1 H, t), 7.88 (1 H, t), 7.96 (1 H, d), 8.20 (1H, d), 8.48 (1H, d), 8.83 (1H, d).

### Description 77.

### 4-Fluoronaphthalene-1-sulfonyl chloride (D77)

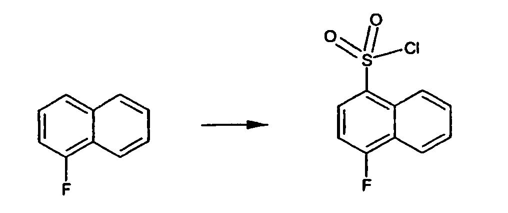

1-Fluoronaphthalene (1.262 ml) was added dropwise over a period of 20 min to an ice-cooled, stirred solution of chlorosulphonic acid (2.3ml). The mixture was stirred for an additional 30min at 5°C and 3h at room temperature. The mixture was then poured onto ice and extracted twice with ethyl acetate. The organic solution was washed with water, brine, dried (MgSO4) and evaporated. The residue was chromatographed over silica gel, eluting with a gradient of 10 to 30% ethyl acetate in hexane. The title compound was obtained as a pale yellow solid (0.86g), δ_{H} (d₆-CDCl₃) 7.27 (1 H, dd, J 8.6, 9.1 Hz),), 7.77 (1 H, ddd, J 0.8, 7.2, 8.2Hz), 7.89 (1 H, ddd, J 1.3, 7.1, 8.6Hz), 8.29 (1 H, d, J 8.4Hz), 8.39 (1 H, dd, J 8.4, 5.1 Hz), 8.80, (1 H, ddd, J 0.9, 0.9, 8.7Hz).

### Description 78

### 2,3-Dimethylbenzenesulphonyl chloride and 3,4-dimethylbenzenesulphonyl chloride (D78a and D78b)

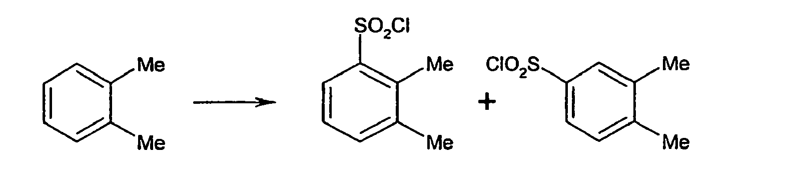

Chlorosulphonic acid (2.18ml, 32.8mmol) was stirred at 0° and treated dropwise with o-xylene (2.0ml, 16.4mmol) added over 20 mins. Stirred for a further 30 mins at 0° followed by 3 hours at ambient temperature. Poured on to ice and extracted with ethyl acetate. Organic extract dried (MgSO₄) and evaporated to give crude product which was chromatographed on silica gel. Elution with hexane gave pure 2,3-dimethylbenzenesulphonyl chloride (D78a) as a solid (220mg) followed by mixed fractions followed by pure 3,4-dimethylbenzenesulphonyl chloride (D78b) as an oil (750mg).

### Description 79

### 7-Trifluoromethyl-quinoline-5-sulfonyl chloride (D79)

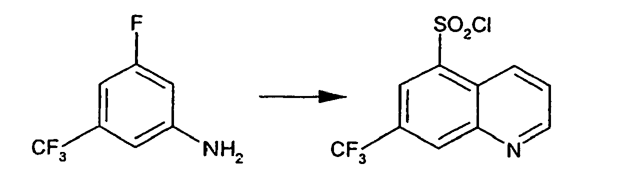

A mixture of 3-fluoro-5-trifluoromethylaniline (5.0g, 27.9mmol), glycerol (6.42g, 69.8mmol) and sodium m-nitrobenzenesulphonate (7.54g, 33.5mmol) was treated with 70% sulphuric acid (24ml) and heated to reflux for 2.5 hours. The mixture was then cooled, diluted with water (50ml) and basified with 50% sodium hydroxide solution. Flitration through kieselguhr gave a solid which was washed with MDC. The aqueous filtrate was extracted twice with MDC and the combined extracts/washings evaporated to give a red oil (4.2g). This mixture of regioisomers in MeOH (30ml) was stirred at room temperature during the addition of sodium methoxide (39mmol) in MeOH (20ml) and then heated to reflux overnight. The MeOH was removed under reduced pressure and the residue partitioned between ethyl acetate and water. Organic layer dried and evaporated to afford crude product which was chromatographed on silica gel. Elution with 25% to 50% EtOAc/hexane gave the desired 5-methoxy-7-trifluoromethylquinoline as a pale solid (1.51 g, 34%) followed by the other regioisomer.

The methoxy compound (1.51g) in 48% aqueous hydrobromic acid (15ml) was heated to reflux for 24 hours. The reaction mixture was evaporated and the residue partitioned between 2M sodium hydroxide and MDC. Aqueous layer neutralised to pH 7 by addition of 5M HCl and the resulting solid (0.97g) collected by filtration.

A solution of 5-hydroxy-7-trifluoromethylquinoline (500mg, 2.35mmol) in dry DMF (15ml) under argon was treated with 60% sodium hydride in oil (94mg, 2.35mmol) and stirred at room temperature for 30 mins. Dimethylthiocarbamoyl chloride (377mg, 3.05mmol) was then added and after 15 mins at room temperature the mixture was heated to 70° over 30 mins and cooled. Diluted with EtOAc and washed 6 times with water, dried and evaporated to afford crude material which was chromatographed on silica gel. Elution with 20% EtOAc in hexane gave O-(quinolyl)-dimethylthiocarbamate (650mg, 92%).

The thiocarbamate (650mg) in acetonitrile (15ml) was heated in a sealed tube under microwave radiation at 200° for 4 hours. After removal of solvent the product was chromatographed on silica gel and elution with 20% EtOAc in chloroform gave the S-(quinolyl)-dimethylthiocarbamate as a yellow solid (122mg).

A solution of the S-(quinolyl)-dimethylthiocarbamate (122mg) in 90% sulphuric acid (1.1ml) was heated to 100° for 40 mins. and cooled, mixed with ice followed by sat. NaHCO₃ solution, and extracted 3 times with MDC. Combined extracts dried and evaporated to afford crude disulphide as a gum (74mg, 81%).

A suspension of the disulphide (74mg) in water (5ml) was stirred at room temperature for 4 hours during which time chlorine was bubbled slowly through the mixture. After basification with sat. NaHCO₃, the organic material was extracted with MDC and evaporated to give the title compound as a yellow oil (80mg, 84%). □_{H} (CDCl₃) 7.83 (1 H, dd, J = 8.8 and 4.0 Hz), 8.58 (1H, s), 8.80 (1 H, s), 9.18 (1 H, d, J = 8.8 Hz), 9.21 (1H, d, J = 4.0 Hz) ppm.

### Description 80

### 7-Fluoro-2-methyl-quinoline-5-sulfonyl chloride (D80)

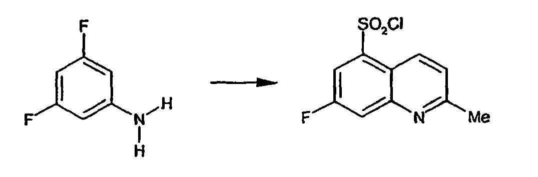

To 3,5-difluoroaniline (4.94g) was added conc. hydrochloric acid followed by p-chloroanil (9.4g) and n-butanol (10ml). The mixture was stirred and heated to reflux. A solution of crotonaldehyde (3.25g) in n-butanol (5 ml) was added slowly over a period of 30 minutes and reflux was continued for a further 20 minutes. After cooling, the reaction mixture was basified by the addition of 50% sodium hydroxide solution. The mixture was extracted with dichloromethane and any insoluble material was removed by filtration. The organic solution was dried (MgSO₄) and evaporated to yield a black solid. This solid was chromatographed over silica gel, eluting with a gradient of 0 to 50% ethyl acetate in hexane. The 5,7-difluoro-2-methyl-quinoline was obtained as a dark green solid (4.88g), m/z 180 [MH⁺].

5,7-Difluoro-2-methyl-quinoline (1.0g) was dissolved in dry DMF (5ml) and stirred at room temperature with sodium thiomethoxide (0.392g). The reaction mixture was then partitioned between ethyl acetate and water. The organic solution was washed with saturated brine, dried (MgSO₄) and evaporated. The residue was chromatographed over silica gel, eluting with a gradient of 0 to 30% ethyl acetate in hexane. The desired 7-fluoro-2-methyl-5-methylthio-quinoline (0.604g) was obtained as a pale green solid, m/z 208 [MH⁺].

7-Fluoro-2-methyl-5-methylthio-quinoline (0.350g) was dissolved in dichloromethane (20ml) and m-chloroperbenzoic acid (0.417g, 70% pure) was added portionwise to the stirred solution. The reaction mixture was then stirred at room temperature for 2 hours. The solution was washed with saturated sodium carbonate solution, saturated brine, dried (MgSO₄) and evaporated. The residue was chromatographed over silica gel, eluting with a gradient of 0 to 100% ethyl acetate in hexane. The pure 7-fluoro-5-methanesulfinyl-2-methyl-quinoline (0.273g) was obtained as a white solid,m/z 224 [MH⁺].

7-Fluoro-5-methanesulfinyl-2-methyl-quinoline (0.500g) was dissolved in acetonitrile (15ml) and cooled to 5°C with stiring, under an atmosphere of argon. 2,6-Lutidine (0.860ml) was added, followed by trifluoroacetic anhydride (0.950ml). The reaction mixture was stirred at 5°C for 1 hour and allowed then to reach room temperature. The solution was evaporated at room temperature and the residue cooled to 5°C. A pre-cooled (5°C) solution of methanol (5ml)/triethylamine (5ml) was added and the mixture stirred at room temperature for 1 hour. The solution was then evaporated at room temperature and the residue partitioned between ethyl acetate and saturated ammonium chloride solutuion. The organic solution was washed with brine, dried (MgSO₄) and evaporated. The residue was chromatographed over silica gel, eluting with a gradient of 0 to 75% ethyl acetate in hexane, to yield bis (7-fluoro-2-methyl-quinolin-5-yl) disulphide as a pale yellow solid (0.158g), m/z 385 [MH⁺].

Bis (7-fluoro-2-methyl-quinolin-5-yl) disulphide (0.205g) was suspended in water and chlorine gas was bubbled through the rapidly stirred suspension for 3 hours. During this time the consistency of the suspension changed. The resulting suspension was collected by filtration, washed with water and dried to yield the 7-fluoro-2-methylquinoline-5-sulfonic acid as a white solid (0.241g), m/z 240 [M-H]⁻.

The 7-fluoro-2-methyl-quinoline-5-sulfonic acid (0.510g) was suspended in methanol (50ml) and powdered sodium hydroxide was added to the stirred suspension, which was stirred at room temperature for 30 minutes. The resulting solution was evaporated at reduced pressure and azeotroped twice from toluene to yield the sodium salt as a white solid. This solid was suspended in phosphorus oxychloride and heated to reflux for 2 hours. After cooling, the reaction mixture was diluted with dichloromethane and added portionwise to saturated sodium hydrogen carbonate solution, with stiring. The resulting two-phase solution was stirred at room temperature for 2 hours until effervescence had ceased. The organic solution was then separated, dried (MgSO₄) and evaporated to yield the 7-fluoro-2-methylquinoline-5-sulfonyl chloride (D80) as an off-white solid (0.141g), δ_{H} (CDCl₃) *inter alia* 2.81 (3H, s), 7.53 (1 H, d, J = 8.9 Hz), 8.03 (1H, ddd, J = 0.5, 2.5 and 9.0 Hz), 8.15 (1 H, dd, J = 2.5 and 7.5 Hz), 8.95 (1 H, d, 8.8 Hz) ppm.

### Examples

### Example 1

Naphthalene-1-sulfonic acid [(R)-2-hydroxy-3-piperidin-1-ylpropyl]-amide (E1)

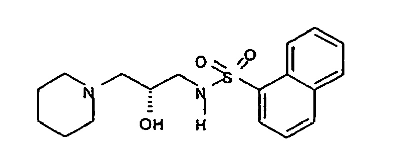

(S)-1-Amino-3-piperidin-1-ylpropan-2-ol (117mg) was dissolved in dichloromethane (6ml) and 1-naphthylsulfonylchloride (185mg) and triethylamine (0.11ml) were added. The reaction mixture was stirred overnight, diluted with dichloromethane and washed with NaHCO₃ solution and brine. The organic solution was dried (MgSO₄) and evaporated. The residual oil was chromatographed over silica gel, eluting with a gradient of 0-10% [9:1 MeOH:NH₃] in dichloromethane. The title compound was obtained as a colourless oil (143mg), m/z (API⁺) 349 [MH⁺].

### Example 2

### Naphthalene-1-sulfonic acid {(2R)-hydroxy-3-[(2R,S)-methylpiperidin-1-yl]-propyl}-amide (E2)

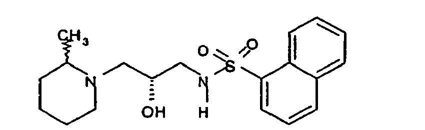

1-Amino-3-[(2R,S)-methylpiperidin-1-yl)-propan-(2S)-ol (215mg) was dissolved in dichloromethane (8ml) and 1-naphthylsuffonylchloride (312mg) and Et₃N (0.2ml) were added. The reaction mixture was stirred overnight, diluted with dichloromethane and washed with NaHCO₃ solution and brine. The organic solution was dried (MgSO₄), and evaporated. The residual oil was chromatographed over silica gel, eluting with a gradient of 0-10% [9:1 MeOH:NH₃] in dichloromethane. The title compound was obtained as a colourless oil (190mg), m/z 363 (API⁺) [MH⁺].

### Example 3

### Naphthalene-1-sulfonic acid {3-[(2R,6S)-dimethylpiperidin-1-yl]-(2R)-hydroxypropyl}-amide (E3)

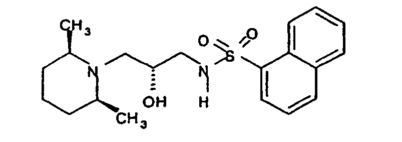

(S)-1-Amino-3-[(2R,6S)dimethylpiperidin-1-yl)-propan-2-ol (80mg) was dissolved in dichloromethane (5ml) and 1-naphthylsulfonylchloride (107mg) and Et₃N (0.07ml) were added to the stirred solution. The reaction mixture was stirred at room temperature for 16 hours. The reaction solution was then diluted with dichloromethane and washed with NaHCO₃ solution, followed by brine. The organic solution was dried (MgSO₄) and evaporated. The residual oil was chromatographed over silica gel, eluting with a gradient of 0-10% [9:1 MeOH:NH₃] in dichloromethane. The title compound was obtained as a colourless oil (27mg), m/z (API⁺) 377 [MH⁺]

### Example 4

### Naphthalene-1-sulfonic acid {3-[(2S)-ethylpiperidin-1-yl]-(2R)-hydroxypropyl}-amide (E4)

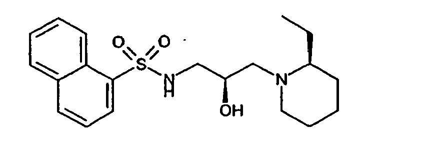

A stirred solution of the amine (2.36 mmol) from Description 59 in dichloromethane (10ml) was treated with triethylamine (496ul, 3.54 mmol) and 1-naphthalenesulphonyl chloride (588mg, 2.60 mmol) and stirred overnight at room temperature. The reaction mixture was diluted with further dichloromethane, washed with saturated NaHCO₃ solution and evaporated to afford the crude product which was chromatographed on silica gel. Gradient elution with (10% ammonia in MeOH) - dichloromethane (4% to 10%) gave the title compound as a colourless gum (460mg, 52%), m/z (API⁺) 377 (MH⁺), (API⁻) 375 (M-H⁻); ¹H NMR δ(CDCl₃) 0.79 (3H, t, J=7.6 Hz), 1.25-1.62 (8H, overlapping m), 2.03-2.13 (3H, overlapping m), 2.58 (1 H, dd, J=12.4 and 10.0 Hz), 2.80 (1 H, m), 2.83 (1 H, dd, J=12.8 and 5.2 Hz), 3.08 (1 H, dd, J=12.8 and 4.0Hz), 3.65 (1 H, m), 7.54 (1H, t, J=8.0 Hz), 7.60 (1H, t, J=8.0 Hz), 7.68 (1 H, t, J=8.0 Hz), 7.95 (1 H, d, J=8.0 Hz), 8.07 (1H, d, J=8.0 Hz), 8.25 (1 H, d, J=8.0 Hz), 8.67 (1 H, d, J=8.0 Hz) ppm.

### Example 5

### Naphthalene-1-sulfonic acid {3-[(2R)-ethylpiperidin-1-yl]-(2R)-hydroxypropyl}-amide (E5)

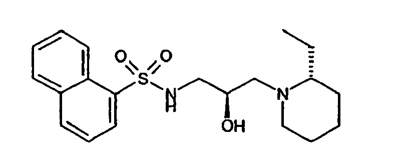

(R)-(-)-2-ethylpiperidine hydrochloride (prepared by the method of J. Cymerman Craig and A. R. Pinder, J. Org. Chem., 1971, 36(23), 3648) was treated as in Descriptions 57, 58, 59 and Example 4 to yield the title compound. Silica gel column chromatography of the crude product [gradient elution with (10% ammonia in MeOH) - dichloromethane, (4% to 10%)] gave the title compound as a colourless gum, m/z (API⁺) 377 (MH⁺), (API⁻) 375 (M-H⁻); ¹H NMR δ(CDCl₃) 0.78 (3H, t, J=7.6 Hz), 1.30-1.60 (8H, overlapping m), 2.14 (1 H, dd, J=12.8 and 10.0 Hz), 2.30 (1H, m), 2.35 (1H, m), 2.44 (1H, dd, J=12.8 and 4.2 Hz), 2.54 (1H, m), 2.82 (1H, dd, J=12.6 and 4.9Hz), 3.07 (1 H, dd, J=12.6 and 4.0 Hz), 3.57 (1H, m), 7.54 (1 H, t, J=8.0 Hz), 7.60 (1H, t, J=8.0 Hz), 7.68 (1 H, t, J=8.0 Hz), 7.95 (1 H, d, J=8.0 Hz), 8.06 (1 H, d, J=8.0 Hz), 8.25 (1 H, d, J=8.0 Hz), 8.67 (1 H, d, J=8.0 Hz) ppm.

### Example 6

### General example exemplified for naphthalene-1-sulfonic acid ((R)-2-hydroxy-3-pyrrolidin-1-ylpropyl)amide trifluoroacetate (E6)

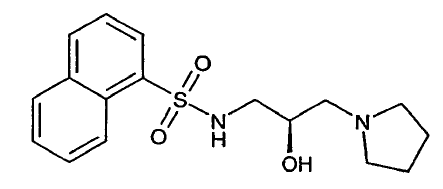

To a solution of naphthalene-1-sulfonyl chloride (55mg;0.24mmol) and (S)-1-amino-3-pyrrolidin-1-ylpropan-2-ol (D2) (32mg; 0.22mmol) in dichloromethane (3ml) was added amberlite IRA-93 resin (30mg), and the reaction was shaken overnight. The resin was removed by filtration and the filtrate evaporated under reduced pressure. Purification by reverse- phase preparative HPLC, eluting with 0.1 % TFA/acetonitrile and 0.1 % TFA/water) gave the title compound as its TFA salt (24mg; 24%). MH⁺ 335. ¹H NMR (250MHz, CDCl₃) □: 10.92 (br s, 1 H), 8.6 (dd,1H), 8.19 (dd,1H), 8.06 (dd,1H), 7.93 (dd1H), 7.71-7.45 (m,3H), 6.21 (br t,1H), 4.2-4.05 (m,1H), 3.9-3.6 (m, 2H), 3.25 (t, 1 H), 3.12-2.69 (m, 5H), 2.15-0.9 (m, 4H) (OH not observed).

### Example 7

### Naphthalene-1-sulfonic acid [(R)-2-hydroxy-3-(2-methylpyrrolidin-1-yl)propyl]amide trifluoroacetate (E7)

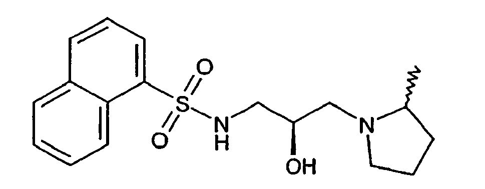

The title compound was prepared from (S)-1-amino-3-(2-methylpyrrolidin-1-yl)propan-2-ol (D4) (35mg; 0.22mmol) using the method outlined in Example 6 (32mg; 31%). MH⁺ 349. ¹H NMR (400MHz, CDCl₃) □: 10.3 (br s, 0.5H), 9.69 (br s, 0.5H), 8.6 (d, 1H), 8.2 (d, 1H). 8.08 (d, 1H). 7.94 (d, 1H). 7.68 (t, 1H). 7.61 (t, 1 H), 7.52 (t, 1H). 6.01 (br s, 1 H), 4.29-3.79 (m, 2H), 3.42-2.78 (m, 6H), 2.25-1.69 (m, 4H), 1.42 (d, 3H) (OH not observed).

### Example 8

### Naphthalene-1-sulfonic acid [(R)-3-(2,4-dimethylpyrrolidin-1-yl)-2-hydroxypropyl]amide trifluoroacetate (E8)

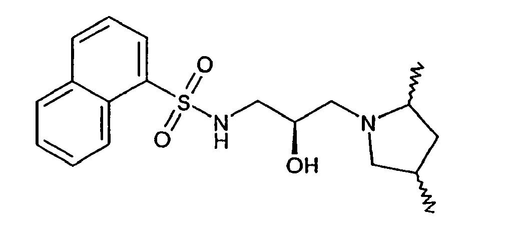

The title compound was prepared from (S)-1-amino-3-(2,4-dimethylpyrrolidin-1-yl)propan-2-ol (D7) (35mg; 0.203mmol) using the method of Example 6 (44mg; 45%). MH⁺ 363. ¹H NMR (400MHz, CDCl₃) δ: 10.9 (br s, 0.5H), 10.34 (br s, 0.5H), 8.6 (dd,1H), 8.19 (dd,1H), 8.06 (dd,1H), 7.94 (dd,1H), 7.68 (dt, 1 H), 7.6 (dt,1H), 7.54 (dt,1H), 6.04-5.89 (m, 1 H), 4.4-3.96 (m, 3H), 3.4-3.1 (m, 3H), 3.06-2.85 (m, 3H), 2.42-2.3 (m, 1 H), 2.26-2.15 (m, 1 H), 1.44-1.37 (m, 3H), 1.11-1.02 (m, 3H). (OH not observed).

### Example 9

### Naphthalene-1-sulfonic acid [(R)-2-hydroxy-3-(2-isopropylpyrrolidin-1-yl)propyl]amide trifluoroacetate (E9)

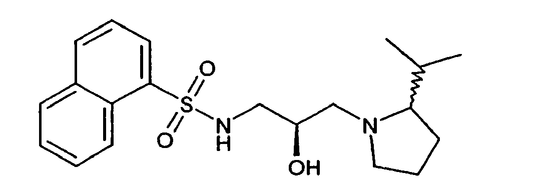

The title compound was prepared from (S)-1-amino-3-(2-isopropylpyrrolidin-1-yl)propan-2-ol (D9) (41 mg; 0.22mmol) using the method outlined in Example 6 (28mg; 26%). MH⁺ 377. The mixture of diastereomers was separated using normal-phase chiral preparative HPLC chromatography to afford both faster- and slower-eluting components as their free-bases.

Faster-eluting diastereomer: Purity >99.9% w/w, >99.8% ee.

¹H NMR (400MHz, CDCl₃) □: 8.66 (dd, 1 H), 8.25 (dd, 1H), 8.08 (dd, 1 H), 7.93 (dd, 1H), 7.68 (dt, 1H), 7.59 (dt, 1 H), 7.51 (dt, 1 H), 5.85-5.38 (br s, 1H), 3.65-3.55 (m, 1H), 3.09 (dd, 1 H), 2.95-2.83 (m, 2H), 2.57-2.49 (m, 1H), 2.38-2.18 (m, 3H), 1.69-1.4 (m, 5H), 0.8 (d, 3H), 0.72 (m, 3H). (OH not observed).

Slower-eluting diastereomer: Purity >99.9% w/w, >99.8% ee.

¹H NMR (400MHz, CDCl₃) □: 8.66 (dd, 1H). 8.25 (dd, 1H). 8.08 (dd, 1 H), 7.93 (dd, 1 H), 7.68 (dt, 1 H), 7.59 (dt, 1H). 7.51 (dt, 1H), 5.32-5.05 (br s, 1H), 3.7-3.6 (m, 1H), 3.11 (dd, 1H), 3.05-2.96 (m, 1H). 2.85 (dd, 1H). 2.56 (t, 1H). 2.29-2.21 (m, 1H). 2.07-1.95 (m, 3H), 1.7-1.42 (m, 5H), 0.80 (d, 3H), 0.72 (d, 3H). (OH not observed).

### Example 10

### Naphthalene-1-sulfonic acid [(R)-3-(2,5-dimethylpyrrolidin-1-yl)-2-hydroxypropyl]amide trifluoroacetate (E10)

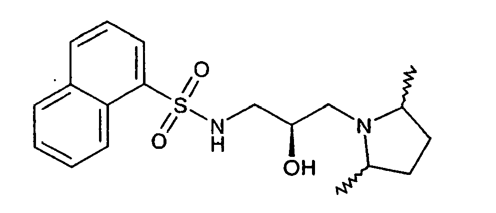

The title compound was prepared from (S)-1-amino-3-(2,5-dimethylpyrrolidin-1-yl)-propan-2-ol (D11) (32mg; 0.22mmol) using the method outlined in Example 6 (41 mg; 39%). MH⁺ 363. ¹H NMR (250MHz, CDCl₃) δ: 10.0 (br s,1H), 8.6 (dd, 1 H), 8.19 (dd, 1 H), 8.05 (dd, 1 H), 7.93 (dd, 1 H), 7.7-7.45 (m, 3H), 5.99 (br t, 1 H), 4.24-4.0 (m, 1 H), 3.35-2.9 (m, 6H), 2.21-2.03 (m, 2H), 2.0-1.79 (m, 2H), 1.46-1.31 (m, 6H). (OH not observed).

### Example 11

### Naphthalene-1-sulfonic acid[(R)-3-(2-cyclohexylpyrrolidin-1-yl)-2-hydroxypropyl]amide (E11)

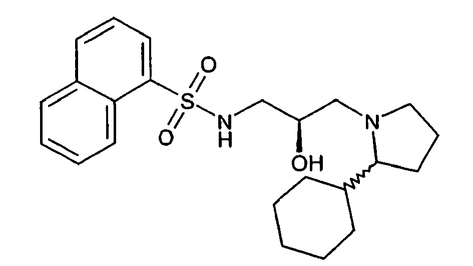

The title compound was prepared from (S)-1-amino-3-(2-cyclohexylpyrrolidin-1-yl)propan-2-ol (D13) (89mg; 0.39mmol) using the method outlined in Example 6. MH⁺ 417. The mixture of diastereomers was separated using normal-phase chiral preparative HPLC chromatography to afford both faster- and slower-eluting components as their free-bases.

Faster-eluting diastereomer: Purity >99.9% w/w, >99.8% ee.

¹H NMR (400MHz, CDCl₃) □: 8.66 (dd, 1H), 8.25 (dd, 1H), 8.06 (dd, 1H), 7.94 (dd, 1H), 7.67 (dt, 1 H), 7.6 (dt, 1H), 7.54 (dt, 1H), 5.81-5.38 (br s, 1H), 3.67-3.59 (m, 1 H), 3.07 (dd, 1H), 2.95-2.85 (m, 2H), 2.58-2.5 (m, 1 H), 2.36-2.19 (m, 3H), 1.72-1.45 (m, 9H), 1.35-1.01 (m, 4H), 0.93-0.81 (m, 2H). (OH not observed).

Slower-eluting diastereomer: Purity 94.5% w/w, 88.9%ee.

¹H NMR (400MHz, CDCl₃) □: 8.66 (dd, 1H), 8.25 (dd, 1H), 8.06 (dd, 1H), 7.94 (dd, 1H), 7.67 (dt, 1H). 7.6 (dt, 1H). 7.54 (dt, 1 H), 5.4-4.91 (br s, 1H), 3.68-3.60 (m, 1H). 3.1 (dd, 1H). 3.0-2.94 (m, 1H), 2.88-2.8 (m, 1H), 2.59 (t, 1H), 2.29-2.21 (m, 1H), 2.07-1.99 (m, 2H), 1.75-1.45 (m, 9H), 1.34-1.03 (m, 4H), 0.96-0.82 (m, 2H). (OH not observed).

### Example 12

### Naphthalene-1-sulfonic acid[(R)-2-hydroxy-3-(2-isobutylpyrrolidin-1-yl)propyl]amide (E12)

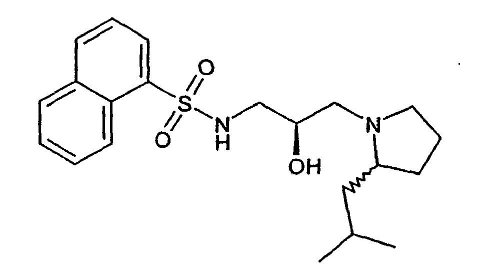

The title compound was prepared from (S)-1-amino-3-(2-isobutylpyrrolidin-1-yl)propan-2-ol (D15) (151 mg; 0.76mmol) using the method outlined in Example 6. MH⁺ 391. The mixture of diastereomers was separated using normal-phase chiral preparative HPLC chromatography to afford both faster- and slower-eluting components.

Faster-eluting diastereomer: Purity 98.3% w/w, 96.7% ee. ¹H NMR (400MHz, CDCl₃) □ : 8.65 (dd, 1H), 8.25 (dd, 1 H), 8.07 (dd, 1 H), 7.94 (dd, 1H), 7.68 (dt, 1H), 7.6 (dt, 1H), 7.53 (dt, 1 H), 3.72-3.65 (m, 2H), 3.05-2.88 (m, 2H), 2.59 (dd, 1H), 2.47-2.24 (m, 3H), 1.95-1.85 (m, 1H), 1.78-1.62 (m, 2H), 1.56-1.45 (m 1H), 1.41-1.31 (m, 1H), 1.29-1.18 (m, 2H), 1.12-1.02 (m, 1H), 0.87 (d, 3H), 0.81 (d, 3H) (OH not observed).

Slower-eluting diastereomer: Purity 99.1 % w/w, 98.2% ee. ¹H NMR (400MHz, CDCl₃) □: 8.66 (dd, 1H), 8.25 (dd, 1 H), 8.06 (dd, 1H), 7.94 (dd, 1 H), 7.68 (dt, 1 H), 7.6 (dt, 1 H), 7.53 (dt, 1H), 3.71-3.63 (m, 1H), 3.12-3.03 (m, 2H), 2.84 (dd,1 1H), 2.69 (t, 1 H), 2.42-2.33 (m, 1H), 2.12-2.02 (m, 2H), 1.96-1.85 (m, 1H), 1.76-1.63 (m, 2H), 1.6-1.5 (m, 1H), 1.44-1.22 (m, 3H), 1.12-1.02 (m,1 H), 0.89 (d, 3H), 0.84 (d, 3H) (OH not observed).

### Example 13

### Naphthalene-1-sulfonic acid[(R)-3-(2-ethylpyrrolidin-1-yl)-2-hydroxypropyl]amide trifluoroacetate (E13)

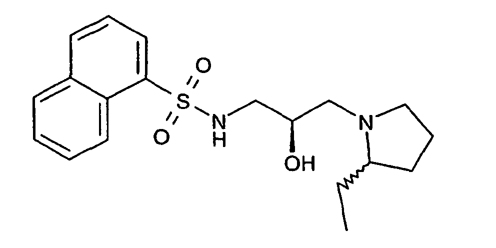

The title compound was prepared from (S)-1-amino-3-(2-ethylpyrrolidin-1-yl)propan-2-ol (D18) (65mg, 0.378mmol) using the method outlined in Example 6 (29mg; 21%). MH⁺ 363. The mixture of diastereomers was separated using normal-phase preparative chiral HPLC to give faster- and slower- eluting components as their free-bases.

Faster-eluting diastereomer: Purity 97.5% w/w, 95% ee.

Slower-eluting diastereomer: Purity >99.9% w/w, >99.8% ee.

### Example 14

### Naphthalene-1-sulfonic acid[(R)-3-(2-tert-butylpyrrolidin-1-yl)-2-hydroxypropyl]amide trifluoroacetate (E14)

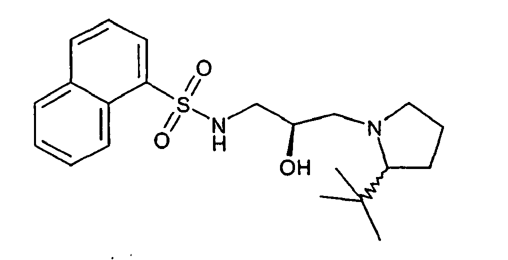

The title compound was prepared from (S)-1-amino-3-(2-tert-butylpyrrolidin-1-yl)propan-2-ol (D21) (56mg; 0.28mmol) using the method outlined in Example 6 (17.6mg; 16%). MH⁺ 391. ¹H NMR (400MHz, CDCl₃) δ: 9.45 (br s, 1 H), 8.61 (dd, 1 H), 8.2 (dd, 1 H), 8.09 (dd, 1 H), 7.95 (dd, 1H), 7.69 (dt, 1 H), 7.61 (dt, 1H), 7.54 (dt, 1 H), 5.98-5.81 (m, 1H), 4.49-4.04 (m, 1H), 3.65-3.45 (m, 2H), 3.25-2.94 (m, 5H), 2.15-1.82 (m, 4H), 1.05 (s, 9H) (OH not observed).

The diastereomers were separated using normal-phase preparative chiral HPLC to give faster- and slower- eluting components as their free-bases.

Faster-eluting diastereomer: Purity >99.9% w/w, >99.8 ee.

Slower-eluting diastereomer: Purity 95.6% w/w, 91.2% ee.

### Example 15

### Naphthalene-1-sulfonic acid[(R)-3-(2-cyclopropylpyrrolidin-1-yl)-2-hydroxypropyl]amide trifluoroacetate (E15)

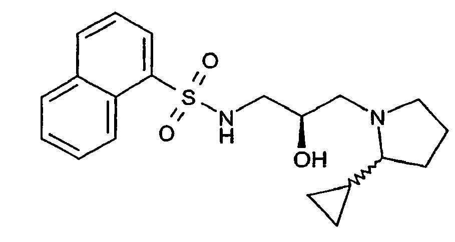

The title compound was prepared from (S)-1-amino-3-(2-cyclopropylpyrrolidin-1-yl)propan-2-ol (D24) (100mg; 0.54mmol) using the method outlined in Example 6 (170mg; 84%). MH⁺ 375.

The diastereomers were separated using normal-phase preparative chiral HPLC to give faster- and slower- eluting components as their free-bases.

Faster-eluting diastereomer: Purity >99.9% w/w, >99.8 ee. ¹H NMR (400MHz, CDCl₃) □: 8.67 (dd, 1H), 8.26 (dd, 1H), 8.07 (dd, 1H), 7.95 (dd, 1H), 7.68 (dt, 1H), 7.60 (dt, 1H), 7.54 (dt, 1H), 6.0-5.5 (br s, 1 H), 3.85-3.71 (m, 1H), 3.07 (dd, 1H), 2.98-2.81 (m, 2H), 2.72-2.59 (m, 1 H), 2.49-2.24 (m, 2H), 1.92-1.5 (m, 5H), 0.61-0.49 (m, 1H), 0.45-0.29 (m, 2H), 0.14- -0.9 (m, 2H) (OH no observed).

Slower-eluting diastereomer: Purity 98.3% w/w, 96.6% ee*.* ¹H NMR (400MHz, CDCl₃) □: 8.67 (dd, 1H), 8.26 (dd, 1 H), 8.07 (dd, 1 H), 7.95 (dd, 1 H), 7.68 (dt, 1H), 7.61 (dt, 1 H), 7.54 (dt, 1H), 5.68-4.96 (br s, 1 H), 3.69 (m, 1H), 3.15-2.95 (m, 3H), 2.87 (dd, 1H), 2.13-2.00 (m, 2H), 1.92-1.49 (m, 5H), 0.6-0.47 (m, 2H), 0.4-0.3 (m, 1 H), 0.18-0.07 (m, 1H), 0.05- -0.1 (m, 1 H) (OH not observed).

### Example 16

### Naphthalene-1-sulfonic acid[(R)-2-hydroxy-3-(3-methylpiperidin-1-yl)propyl]amide trifluoroacetate (E16)

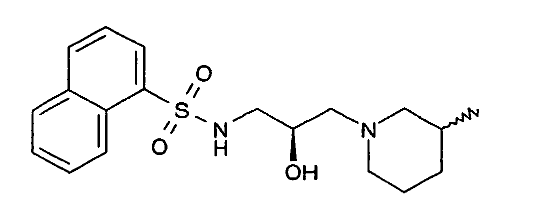

The title compound was prepared from (S)-1-amino-3-(3-methylpiperidin-1-yl)propan-2-ol (D26) (38mg; 0.22mmol) using the method outlined in Example 6 (28mg; 38%). MH⁺ 363. ¹H NMR (400MHz, CDCl₃) δ: 10.05 (br s, 1H), 8.6 (dd, 1 H), 8.2 (dd, 1 H), 8.08 (dd, 1H), 7.94 (dd, 1 H), 7.68 (dt, 1 H), 7.6 (dt, 1 H), 7.52 (dt, 1 H), 6.19 (br s, 1 H), 4.21 (br s, 1H), 3.69-3.4 (m, 2H), 3.22-2.9 (m, 4H), 2.60-2.43 (m, 1 H), 2.31-2.12 (m, 1H), 2.08-1.75 (m, 4H), 1.06-0.87 (m, 4H) (OH not observed).

### Example 17

### Naphthalene-1-sulfonic acid[(R)-2-hydroxy-3-(5-ethyl-2-methylpiperidin-1-yl)propyl]amide (E17)

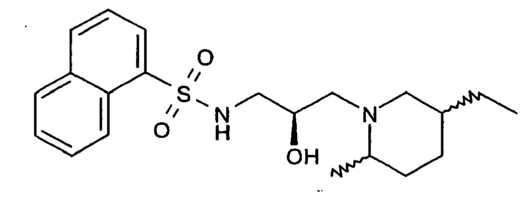

The title compound was prepared from (S)-1-amino-3-(5-ethyl-2-methylpiperidin-1-yl)propan-2-ol (D28) (44mg; 0.22mmol) using the method outlined in Example 6 (34mg; 31%) MH⁺ 391

### Example 18

### Naphthalene-1-sulfonic acid[(R)-2-hydroxy-3-(2-ethylpiperidin-1-yl)propyl]amide trifluoroacetate (E18)

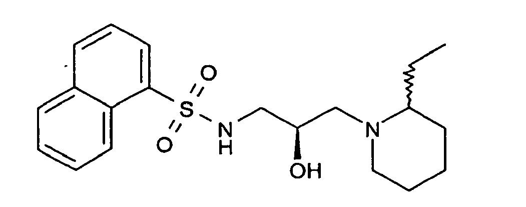

The title compound was prepared from (S)-1-amino-3-(2-ethylpiperidin-1-yl)propan-2-ol (D30) (60mg; 0.323mmol) using the method outlined in Example 6 (71 mg; 45%). MH⁺ 377. The diastereomers were separated using normal-phase preparative chiral HPLC to give faster- and slower- eluting components as their free-bases.

Faster-eluting diastereomer: Purity 99.1% w/w, 98.2% ee. ¹H NMR (400MHz, CDCl₃) δ: 8.66 (dd, 1 H), 8.25 (dd, 1 H), 8.07 (dd, 1 H), 7.95 (dd, 1H). 7.68 (dt, 1H). 7.61 (dt, 1 H), 7.54 (dt, 1 H), 5.7- 4.9 (br s, 1 H), 3.71-3.52 (m, 1 H), 3.07 (dd, 1 H), 2.84 (dd, 1 H), 2.67-2.1 (m, 5H), 1.66-1.15 (m, 8H), 0.95-0.7 (m, 3H) (OH not observed).

Slower-eluting diastereomer: Purity 96% w/w, 92% ee. ¹H NMR (400MHz, CDCl₃) δ: 8.66 (dd, 1H), 8.25 (dd, 1 H), 8.07 (dd, 1 H), 7.95 (dd, 1H), 7.68 (dt, 1H), 7.61 (dt, 1H), 7.54 (dt, 1H), 5.6-5.0 (br s, 1H), 3.75-3.64 (m, 1H), 3.07 (dd, 1H), 2.84 (dd, 2H), 2.7-2.55 (m, 1H), 2.22-2.0 (m, 3H), 1.7-1.18 (m, 8H), 0.8 (t, 3H) (OH not observed).

### Example 19

### Naphthalene-1-sulfonic acid[(R)-2-hydroxy-3-(2-isopropylpiperidin-1-yl)propyl]amide trifluoroacetate (E19)

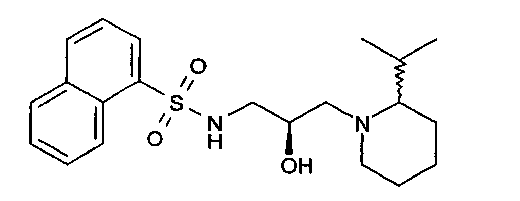

The title compound was prepared from (S)-1-amino-3-(2-isopropylpiperidin-1-yl)propan-2-ol (D33) (50mg; 0.25mmol) using the method outlined in Example 6 (31 mg; 25%). MH⁺ 391. The diastereomers were separated using normal-phase preparative chiral HPLC to give faster- and slower- eluting components as their free-bases.

Faster-eluting diastereomer: Purity >99.9% w/w, >99.8% ee. ¹H NMR (400MHz, CDCl₃) δ: 8.65 (d, 1 H), 8.25 (d, 1 H), 8.07 (d, 1 H), 7.95 (d, 1H), 7.68 (t, 1 H), 7.6 (t, 1 H), 7.52 (t, 1 H), 5.56-5.02 (br s, 1H), 3.74-3.62 (m, 1 H), 3.14-2.94 (m, 2H), 2.88 (dd, 1 H), 2.8-2.7 (m, 1 H), 2.53-2.05 (m, 3H), 1.9-1.77 M, 1H), 1.74-1.53 (m, 2H), 1.51-1.15 (m, 3H), 0.9 (d, 3H), 0.8 (d, 3H) (OH not observed).

Slower-eluting diastereomer: Purity 96.1% w/w, 92.2% ee. ¹H NMR (400MHz, CDCl₃) δ: 8.63 (d, 1H), 8.21 (d, 1H), 8.06 (d, 1H), 7.93 (d, 1 H), 7.68 (t, 1 H), 7.6 (t, 1H), 7.53 (t, 1H), 5.7-5.2 (br s, 1 H), 4.01-3.89 (m, 1 H), 3.59-3.5 (m, 1H), 3.35-3.22 (m, 1 H), 3.08 (dd, 1H), 3.01-2.85 (m, 2H), 2.8-2.22 (m, 2H), 2.1-1.95 (m, 1H), 1.81-1.5 (m, 3H), 1.49-1.12 (m, 4H), 0.95-0.78 (m, 6H) (OH not observed).

### Example 20

### Naphthalene-1-sulfonic acid[(R)-(sec-butylpropylamino)hydroxypropyl]amide trifluoroacetate (E20)

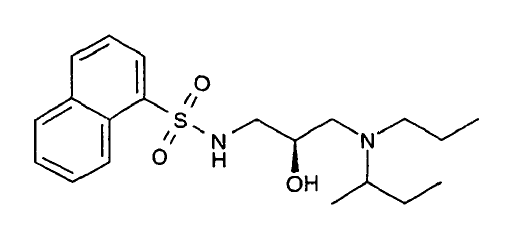

The title compound was prepared from (S)-amino-(*sec-*butylpropylamino)propan-2-ol (D35) (38mg; 0.202mmol) using the method outlined in Example 6 (29.2mg; 29%). MH⁺ 379. ¹H NMR (400MHz, CDCl3) δ: 9.29-8.65 (m, 1H), 8.59 (dd, 1H), 8.2 (dd, 1H), 8.1 (dd, 1H), 7.95 (dd, 1H), 7.7 (t, 1 H), 7.62 (t, 1 H), 7.55 (t, 1 H), 5.75 (br s, 1 H), 4.18 (br s, 1H), 3.48-3.32 (m, 1H). 3.25-2.8 (m, 6H), 1.89-1.62 (m, 3H), 1.54-1.17 (m, 4H), 1.04-0.9 (m, 6H) (OH not observed).

### Example 21

### Naphthalene-1-sulfonic acid[(R)-(tert-butylpropylamino)hydroxypropyl]amide trifluoroacetate (E21)

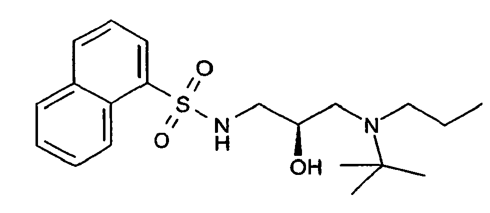

The title compound was prepared from (S)-amino-(*tert*-butylpropylamino)propan-2-ol (D38) (32mg; 0.17mmol) using the method outlined in Example 6 (24mg; 29%). MH⁺ 379. ¹H NMR (400MHz, CDCl3) δ: 8.86 (br s, 0.5H), 8.63 (br s, 0.5H), 8.61 (dd, 1H), 8.2 (dd, 1 H), 8.09 (dd, 1H), 7.95 (dd, 1H), 7.70 (t, 1H), 7.63 (t, 1H), 7.55 (t, 1 H), 5.79-5.62 (m, 1 H), 4.19-4.02 (m, 1H), 3.46-3.32 (m, 1H), 3.27-3.14 (m, 1H), 3.09-2.99 (m, 2H), 2.96-2.84 (m, 1 H), 2.80-2.64 (m, 1H), 2.03-1.57 (m, 2H), 1.39 (s, 9H), 0.94 (t, 3H) (OH not observed).

### Example 22

### Naphthalene-1-sulfonic acid[(R)-3-(1,3-dihydroisoindol-2-yl)-2-hydroxypropyl]amide trifluoroacetate (E22)

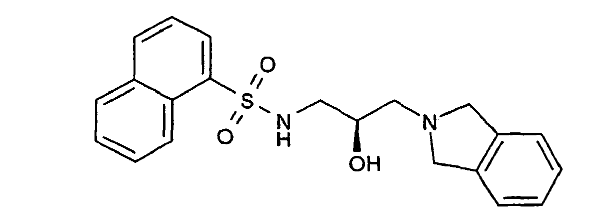

The title compound was prepared from (S)-1-amino-3-(1,3-dihydroisoindol-2-yl)propan-2-ol (D40) (42mg; 0.218mmol) using the method outlined in Example 6 (31 mg, 28%). MH⁺ 383. ¹H NMR (400MHz, CDCl3) δ: 8.6 (d, 1 H), 8.18 (d, 1 H), 8.05 (d, 1H), 7.91 (d, 1 H), 7.61 (t, 1H), 7.55 (t, 1H), 7.5 (t, 1H), 7.33 (m, 2H), 7.25 (m, 2H), 6.35 (br s, 1 H), 5.15-4.1 (m, 5H), 3.46-3.23 (m, 2H), 3.12-2.95 (m, 2H) (OH not observed).

### Example 23

### Naphthalene-1-sulfonic acid[(R)-2-hydroxy-3-(octahydroisoquinolin-2-yl)propyl]amide trifluoroacetate (E23)

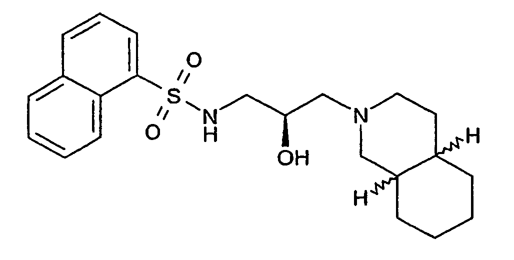

The title compound was prepared from (S)-1-amino-3-(octahydroisoquinolin-1-yl)propan-2-ol (D42) (43mg; 0.202mmol) using the method outlined in Example 6 (28mg; 27%). MH⁺ 403.

### Example 24:

### Naphthalene-1-sulfonic acid[(R)-2-hydroxy-3-(octahydroquinolin-2-yl)propyl]amide (E24)

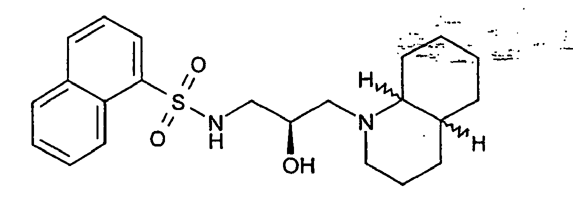

The title compound was prepared from (S)-1-amino-3-(octahydroquinolin-1-yl)propan-2-ol (D44) (144mg; 0.68mmol) using the method outlined in Example 6 (227mg; 83%). MH⁺ 403.

### Example 25

### Naphthalene-1-sulfonic acid[(R)-2-hydroxy-3-((1 S,5R)-1,3,3-trimethyl-6-azabicyclo[3.2.1]oct-6-yl)-propyl]amide trifluoroacetate (E25)

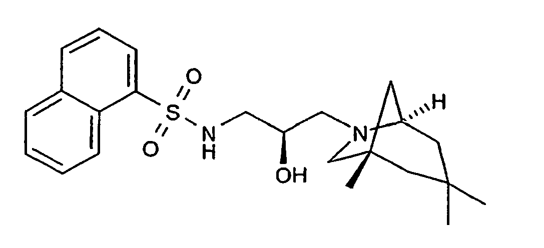

The title compound was prepared from (S)-1-amino-3-((1S,5R)-1,3,3-trimethyl-6-azabicyclo[3.2.1]oct-6-yl)propan-2-ol (D46) (50mg; 0.22mmol) using the method outlined in Example 6 (42mg; 36%). MH⁺ 417.

### Example 26

### Naphthalene-1-sulfonic acid[(S)-3-(2,4-dimethylpyrrolidin-1-yl)-2-hydroxypropyl]amide trifluoroacetate (E26)

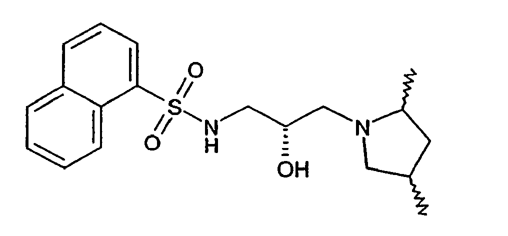

The title compound was prepared from (R)-1-amino-3-(2,4-dimethylpyrrolidin-1-yl)propan-2-ol (D47) (35mg;0.203mmol) using the method outlined in Example 6 (15mg;16%). MH⁺ 363.

### Example 27

### Naphthalene-1-sulfonic acid [(S)-2-hydroxy-3-(2-methylpyrrolidin-1-yl)propyl]amide trifluoroacetate (E27)

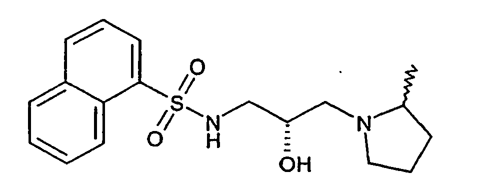

The title compound was prepared from (R)-1-amino-3-(2-methylpyrrolidin-1-yl)propan-2-ol (D61) (47mg; 0.297mmol) using the method outlined in Example 6 (30mg;22%). MH⁺ 349.

### Example 28

### Naphthalene-1-sulfonic acid[(S)-2-hydroxy-3-(3-methylpiperidin-1-yl)propyl]amide trifluoroacetate (E28)

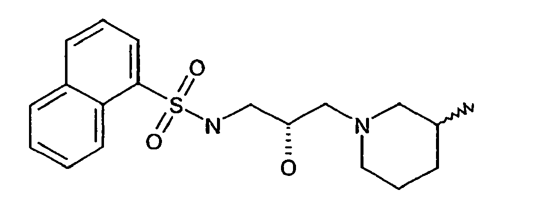

The title compound was prepared from (R)-1-amino-3-(3-methylpiperidin-1-yl)propan-2-ol (D63) (24mg; 0.14mmol) using the method outlined in Example 6 (25mg;38%).

### Example 29

### Naphthalene-1-sulfonic acid[3-(2,4-dimethylpyrrolidin-1-yl)-2-hydroxypropyl]amide (E29)

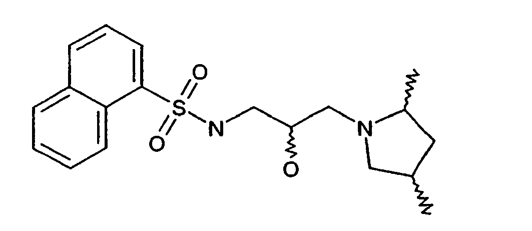

A diastereomeric mixture of the title compound was separated by normal-phase preparative chiral HPLC to give four diastereomers.

A1 20.7mg (>99.9% ee); A2 20.7mg (>99.9% ee); A3 6.3 mg (70.7% ee); A4 7.6 mg (99.9% ee).

### Examples 30 to 71

A solution of (S)-1-amino-3-(2,4-dimethylpyrrolidin-1-yl)propan-2-ol (D7) (800mg;4.65mmol) in anhydrous dichloromethane (38ml) was prepared, and aliquots (0.5ml; 0.06mmol) were added to solutions of 50 sulfonyl chlorides (see Table 1) (0.075mmol each) dissolved in anhydrous dichloromethane (1ml each).
Triethylamine (0.021 ml; 0.15mmol) was added to each solution, which were shaken overnight at ambient temperature. The reaction mixtures were shaken with polymer-bound tris amine (~45mg) for 3h., then filtered and purified by preparative HPLC to afford the trifluoroacetate salts. The following compounds passed 90% purity criterion.

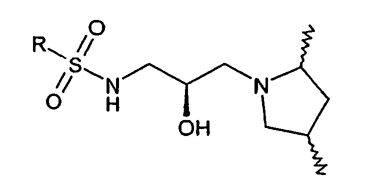

**Table 1**

| **Example no.** | **R** | **MH⁺** |
|---|---|---|
| 30 | 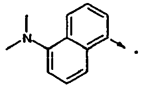 | 406 |
| 31 | 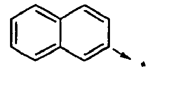 | 363 |
| 32 | 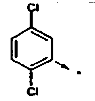 | 382 |
| 33 | 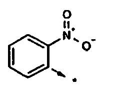 | 358 |
| 34 | 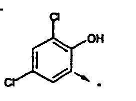 | 398 |
| 35 | 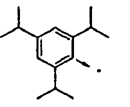 | 439 |
| 36 | 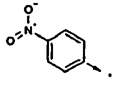 | 358 |
| 37 | 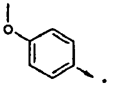 | 343 |
| 38 | 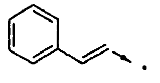 | 339 |
| 39 | 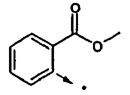 | 371 |
| 40 | 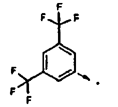 | 449 |
| 41 | 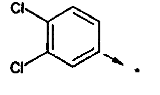 | 382 |
| 42 | 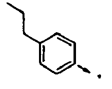 | 355 |
| 43 | 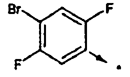 | 428 |
| 44 | 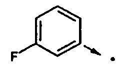 | 331 |
| 45 | 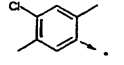 | 375 |
| 46 | 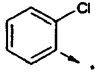 | 347 |
| 47 | 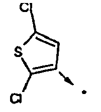 | 388 |
| 48 | 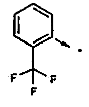 | 381 |
| 49 | 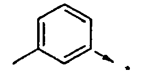 | 327 |
| 50 | 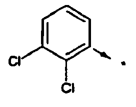 | 382 |
| 51 | 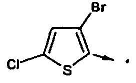 | 432 |
| 52 | 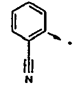 | 338 |
| 53 | 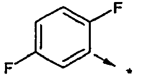 | 349 |
| 54 | 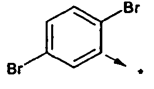 | 471 |
| 55 | 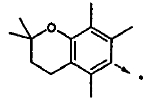 | 439 |
| 56 | 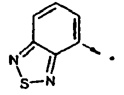 | 371 |
| 57 | 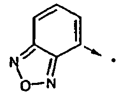 | 355 |
| 58 | 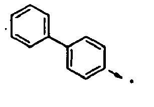 | 389 |
| 59 | 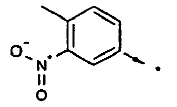 | 372 |
| 60 | 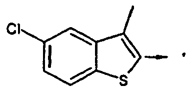 | 418 |
| 61 | 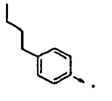 | 369 |
| 62 | 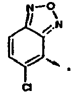 | 389 |
| 63 | 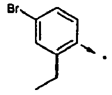 | 420 |
| 64 | 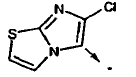 | 393 |
| 65 | 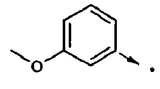 | 343 |
| 66 | 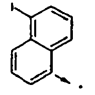 | 489 |
| 67 | 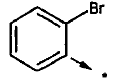 | 392 |
| 68 | 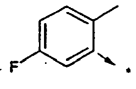 | 345 |
| 69 | 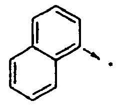 | 363 |
| 70 | 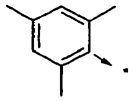 | 355 |
| 71 | 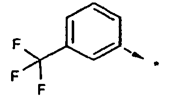 | 381 |

### Example 72.

### Naphthalene-1-sulfonic acid [(R)-3-(2,6-diethylpiperidin-1-yl)-2-hydroxy-propyl]-amide (E72)

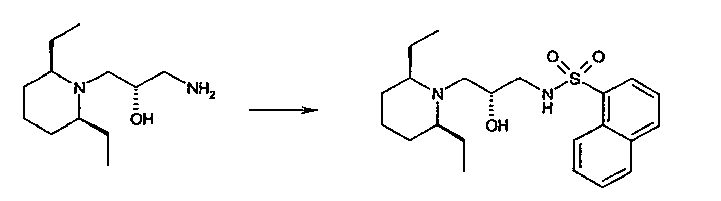

(S)-1-Amino-3-(2,6-diethyl-piperidin-1-yl)-propan-2-ol (D67) (70mg) was stirred with 1-naphthalenesulfonylchloride (75mg) and triethylamine (0.05ml) in DCM (5ml) at room temperature for 72h. Reaction mixture was diluted with DCM, washed with NaHCO₃ solution, dried (MgSO₄), and evaporated. The residual oil was chromatographed over silica gel eluting with a gradient of 0-10% [9:1 MeOH:NH₃] in DCM . Title compound was obtained as a colourless oil (60mg), m/z 405 [MH⁺]; δ_{H} (CDCl₃) 0.77-0.83 (6H, overlapping, m), 1.15-1.18 (4H, overlapping, m), 1.34-1.45 (5H, overlapping, m), 1.68 (1H. m), 2.22 (1 H, m), 2.38 (3H, m), 2.82 (1H. dd, J 12.8 and 5.2Hz), 3.06 (1H. dd, J 12.8 and 4Hz), 3.80 (1 H, m), 7.51-7.69 (3H, overlapped, m), 7.93 (1 H, d, J 8Hz), 8.05 (1 H, d, J= 8.4Hz), 8.25 (1 H, d, J 7.2Hz), 8.67 (1 H, d, J 8.8Hz).

### Example 73.

### 2,3-Dichloro-N-[(R)-3-((2S,6R)-2,6-dimethyl-piperidin-1-yl)-2-hydroxy-propyl]-benzenesulfonamide (E73)

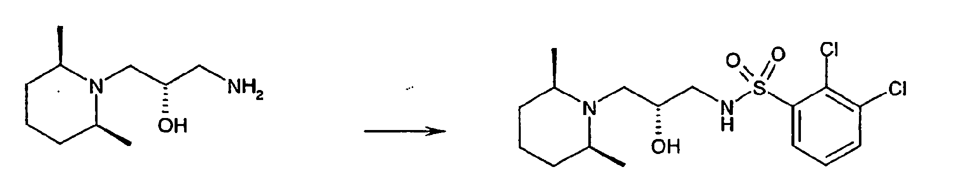

(S)-1-Amino-3-((2S,6R)-2,6-dimethyl-piperidin-1-yl)-propan-2-ol (D70) (0.5 mmol) was stirred with 2,3-dichlorophenylsulfonylchloride (0.55 mmol) and triethylamine (0.08ml) in DCM (5ml) at room temperature overnight. Reaction mixture was diluted with DCM, washed with NaHCO₃ solution, dried (MgSO₄), and evaporated. The residual oil was chromatographed over silica gel eluting with a gradient of 0-10% [9:1 MeOH:NH₃] in DCM . Title compound was obtained as a colourless oil (40mg), m/z 395 [MH⁺]

### Example 74.

### 2,3,4-Trichloro-N-[(R)-3-((2S,6R)-2,6-dimethyl-piperidin-1-yl)-2-hydroxy-propyl]-benzenesulfonamide (E74)

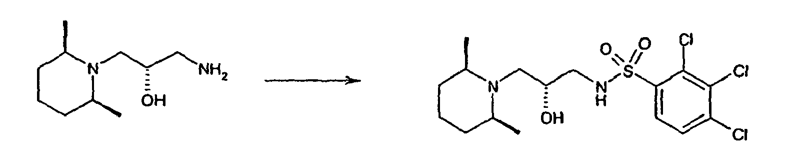

The title compound, prepared by the procedure of Example 73 was obtained as a colourless oil (69mg), m/z 430 [MH⁺]

### Example 75.

### 2,5-Dichlorothiophene-3-sulfonic acid [(R)-3-((2S,6R)-2,6-dimethyl-piperidin-1-yl)-2-hydroxy-propyl]-amide (E75)

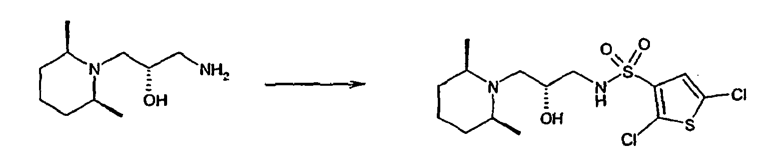

The title compound, prepared by the procedure of Example 73 was obtained as a colourless oil (105mg), m/z 401 [MH⁺]

### Example 76.

### 4,5-Dibromo-thiophene-2-sulfonic acid [(R)-3-((2R,6S)-2,6-dimethyl-piperidin-1-yl)-2-hydroxy-propyl]amide (E76)

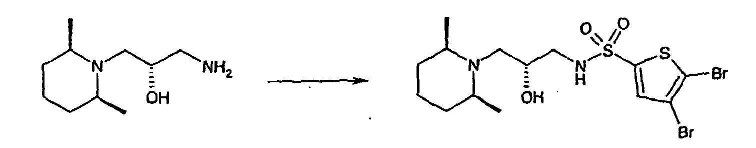

The title compound, prepared by the procedure of Example 73 was obtained as a colourless oil (112mg), m/z 491 [MH⁺].

### Example 77.

### 4-Bromo-2,5-dichloro-thiophene-3-sulfonic acid [(R)-3-((2S,6R)-2,6-dimethyl-piperidin-1-yl)-2-hydroxy-propyl]-amide (E77)

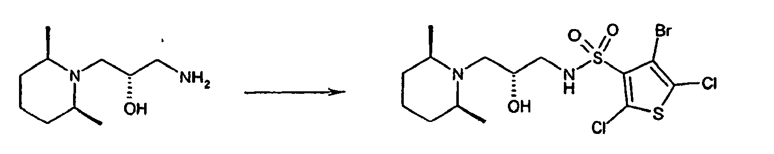

The title compound, prepared by the procedure of Example 73 was obtained as a colourless oil (91 mg), m/z 481 [MH⁺].

### Example 78.

### 5-Chloro-naphthalene-1-sulfonic acid [(R)-3-((2S,6R)-2,6-dimethyl-piperidin-1-yl)-2-hydroxy-propyl]-amide (E78)

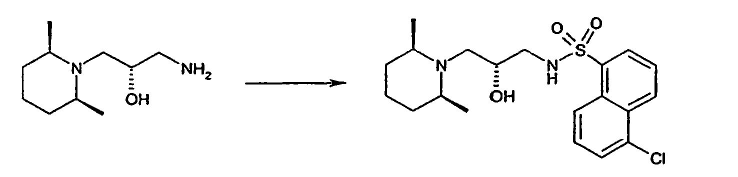

The title compound, prepared by the procedure of Example 73 was obtained as a yellow oil (72mg), m/z 411 [MH⁺]; δ_{H} (CDCl₃) 0.95-0.99 (6H, overlapping, m), 1.14-1.37 (4H, overlapping, m), 1.47 (2H, t, broad, J=16Hz), 1.64 (1H. m), 2.33 (1H. dd, J=14 and 5Hz), 2.50 (3H, m), 2.82 (1 H, dd, J=13 and 5Hz), 3.05 (1 H, dd, J=13 and 4Hz), 3.47 (1 H, m), 7.63 (3H, overlapped, m), 8.31 (1H. dd, J=7 and 1 Hz), 8.57 (1H. dd, J= 8 and 1Hz). 8.64 (1 H, dd, J=8.5 and 1 Hz).

### Example 79.

### 5-Chloro-naphthalene-2-sulfonic acid [(R)-3-((2S,6R)-2,6-dimethyl-piperidin-1-yl)-2-hydroxy-propyl]-amide (E79)

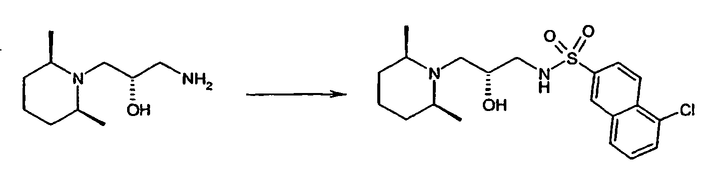

The title compound, prepared by the procedure of Example 73 was obtained as a yellow oil (72mg), m/z 411 [MH⁺]

### Example 80.

### Naphthalene-2-sulfonic acid [(R)-3-((2S,6R)-2,6-dimethyl-piperidin-1 -yl)-2-hydroxypropyl]-amide (E80)

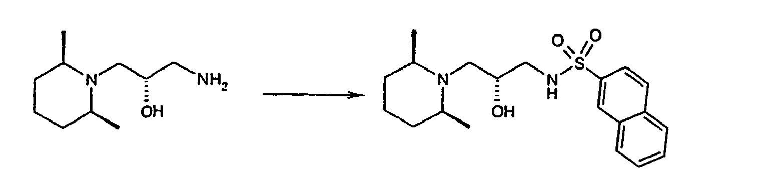

The title compound, prepared by the procedure of Example 73 was obtained as a yellow oil(71mg), m/z 377 [MH⁺].

### Example 81.

### N-[(R)-3-((2S,6R)-2,6-Dimethyl-piperidin-1-yl)-2-hydroxy-propyl]-benzenesulfonamide (E81)

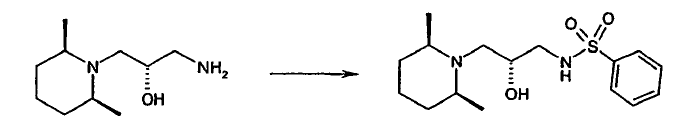

The title compound, prepared by the procedure of Example 73 was obtained as a yellow solid (19mg), m/z 327 [MH⁺]

### Example 82.

### 4'-Chloro-biphenyl-4-sulfonic acid [(R)-3-((2S,6R)-2,6-dimethyl-piperidin-1-yl)-2-hydroxy-propyl]-amide (E82)

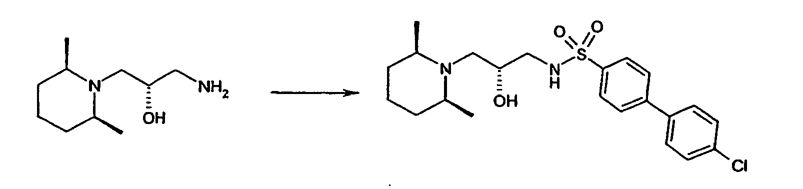

The title compound, prepared by the procedure of Example 73 was obtained as a yellow solid (78mg), m/z 437 [MH⁺].

### Example 83.

### Biphenyl-4-sulfonic acid [(R)-3-((2S,6R)-2,6-dimethyl-piperidin-1-yl)-2-hydroxypropyl]-amide (E83)

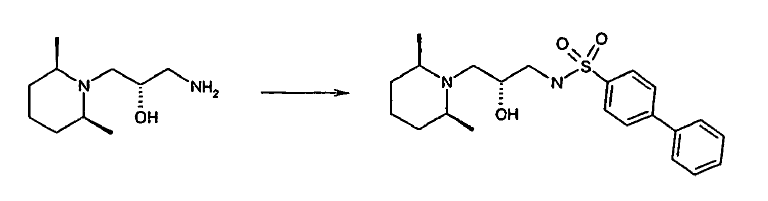

The title compound, prepared by the procedure of Example 73 was obtained as a yellow solid (39mg), m/z 403 [MH⁺].

### Example 84.

### N-[(R)-3-((2S,6R)-2,6-Dimethyl-piperidin-1-yl)-2-hydroxy-propyl]-4-phenoxy-benzenesulfonamide (E84)

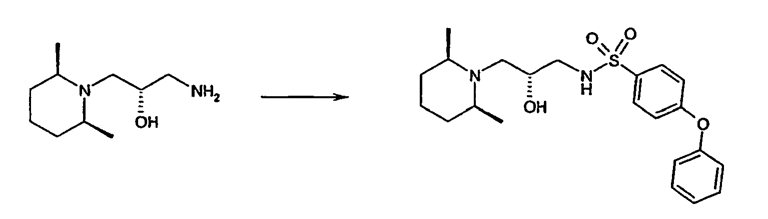

The title compound, prepared by the procedure of Example 73 was obtained as a yellow solid (51 mg), m/z 419 [MH⁺].

### Example 85.

### 3,4-Dichloro-N-[(R)-3-((2S,6R)-2,6-dimethyl-piperidin-1-yl)-2-hydroxy-propyl]-benzenesulfonamide (E85)

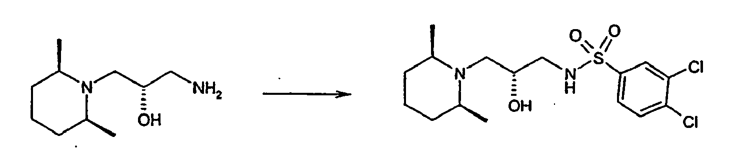

The title compound, prepared by the procedure of Example 73 was obtained as a yellow solid (54mg), m/z 395 [MH⁺].

### Example 86.

### Quinoline-5-sulfonic acid [(R)-3-((2S,6R)-2,6-dimethyl-piperidin-1-yl)-2-hydroxypropyl]-amide (E86)

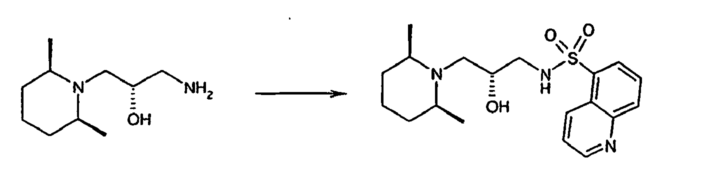

The title compound, prepared by the procedure of Example 73 from the products of Descriptions 56 and 71 was obtained as a pink oil (120mg), m/z 378 [MH⁺] ; δ_{H} (CDCl₃) 0.94-0.98 (6H, overlapping, m), 1.10-1.75 (6H, overlapping, m), 2.35 (1H, dd, J=18 and 5Hz), 2.47 (3H, m), 2.84 (1 H, dd, J=13 and 6Hz), 3.10 (1 H, dd, J=12 and 4Hz), 3.47 (1 H, m), 7.57 (3H, dd, J=9 and, 4Hz), 7.77 (1H, dd, J=8 and 7.5Hz), 8.28 (1H, dd, J= 7 and 1Hz), 8.33 (1H, d, J=8.5Hz), 9.00 (1 H, dd, J=4.5 and 1.5Hz), 9.07 (1H, dd, J=8.5 and 1 Hz).

### Example 87.

### Quinoline-8-sulfonic acid [(R)-3-((2S,6R)-2,6-dimethyl-piperidin-1-yl)-2-hydroxypropyl]-amide (E87)

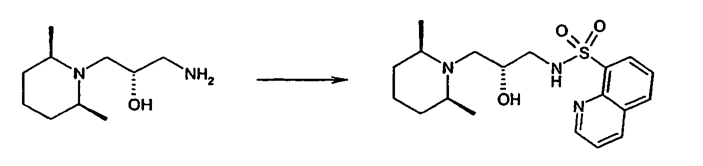

The title compound, prepared by the procedure of Example 73 was obtained as a colourless oil (74mg) m/z 378 [MH⁺].

### Example 88.

### 5-lodo-naphthalene-1-sulfonic acid [(R)-3-((2S,6R)-2,6-dimethyl-piperidin-1-yl)-2-hydroxy-propyl]-amide (E88)

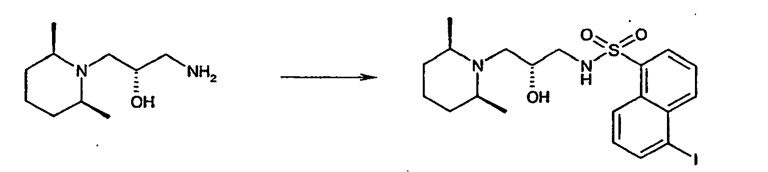

The title compound, prepared by the procedure of Example 73 was obtained as a cream foam (260mg), m/z 503 [MH⁺]

### Example 89.

### 5-Acetyl-naphthalene-1-sulfonic acid [(R)-3-((2S,6R)-2,6-dimethyl-piperidin-1-yl)-2-hydroxy-propyl]-amide (E89)

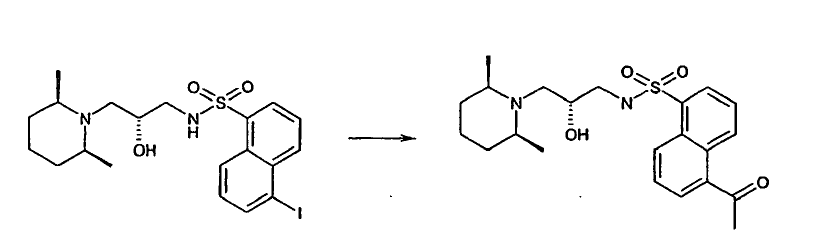

5-lodo-naphthalene-1-sulfonic acid [(R)-3-((2S,6R)-2,6-dimethyl-piperidin-1-yl)-2-hydroxy-propyl]-amide (E88) (100mg) and Pd(PPh₃)₄ (6mg) were stirred in 1,4-dioxane at room temperature for the addition of 1-ethoxyvinyltributyltin (0.1 ml). The mixture was then heated at reflux for 24h. After cooling to room temperature, water (1ml) and 5N HCI (5 drops) were added and the reaction mixture stirred for a further 2h at room temperature. The mixture was concentrated and partitioned between NaHCO₃ solution and ethyl acetate. The organic solution was washed with brine, dried (MgSO₄), and evaporated. The residual oil was chromatographed over silica gel eluting with a gradient of 0-10% [9:1 MeOH:NH₃] in DCM . The title compound was obtained as an orange oil (20mg), m/z 419 [MH⁺]

### Example 90.

### Isoquinoline-5-sulfonic acid [(R)-3-((S)-2-ethyl-piperidin-1-yl)-2-hydroxy-propyl]-amide (E90)

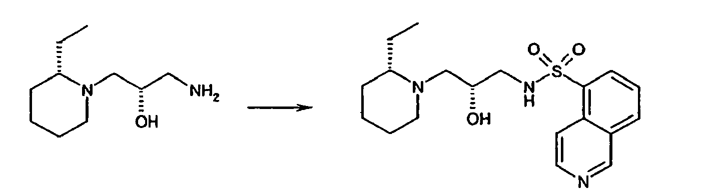

(S)-1-Amino-3-((S)-2-ethylpiperidin-1-yl)-propan-2-ol (D59) (0.54 mmol) was stirred with isoquinoline sulfonylchloride (0.6 mmol) and triethylamine (0.1ml) in DCM (5ml) at room temperature overnight. Reaction mixture was diluted with DCM, washed with NaHCO₃ solution, dried (MgSO₄), and evaporated. The residual oil was chromatographed over silica gel eluting with a gradient of 0-10% [9:1 MeOH:NH₃] in DCM. Title compound was obtained as a colourless oil (123mg), m/z 378 [MH⁺]

### Example 91.

### Quinoline-8-sulfonic acid [(R)-3-((S)-2-ethyl-piperidin-1-yl)-2-hydroxy-propyl]-amide (E91)

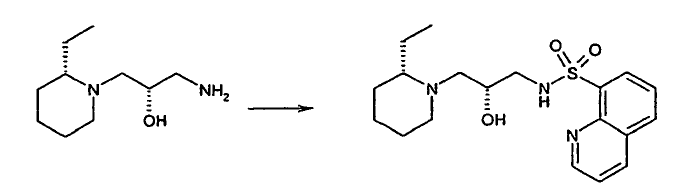

The title compound, prepared by the procedure of Example 90 was obtained as a colourless oil (151 mg), m/z 378 [MH⁺]

### Example 92.

### 5-Chloro-3-methyl-benzo[b]thiophene-2-sulfonic acid [(R)-3-((S)-2-ethyl-piperidin-1-yl)-2-hydroxy-propyl]-amide (E92)

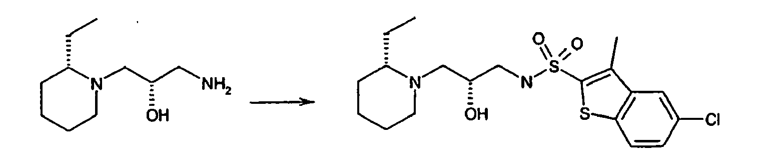

The title compound, prepared by the procedure of Example 90 was obtained as a colourless oil (52mg), m/z 431 [MH⁺]

### Example 93.

### N-[(R)-3-((S)-2-Ethylpiperidin-1-yl)-2-hydroxy-propyl]-1-phenyl-methanesulfonamide (E93)

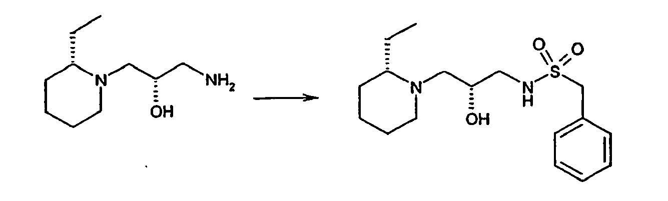

The title compound, prepared by the procedure of Example 90 was obtained as a colourless oil (21 mg) m/z 341 [MH⁺]

### Example 94.

### 2,3-Dichloro-N-[(R)-3-((S)-2-ethyl-piperidin-1-yl)-2-hydroxy-propyl]-benzenesulfonamide (E94)

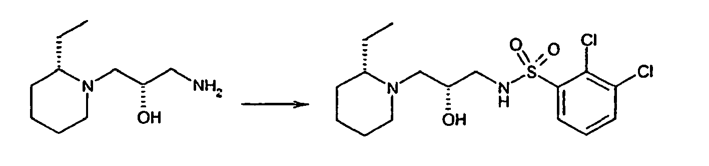

The title compound, prepared by the procedure of Example 90 was obtained as a colourless oil (80mg), m/z 395 [MH]⁺

### Example 95.

### Thiophene-2-sulfonic acid [(R)-3-((S)-2-ethyl-piperidin-1-yl)-2-hydroxy-propyl]-amide (E95)

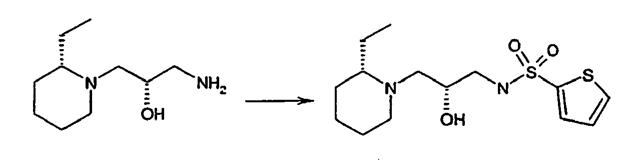

The title compound, prepared by the procedure of Example 90 was obtained as a colourless oil (78mg), m/z 333 [MH⁺].

### Example 96.

### 2,5-Dichlorothiophene-3-sulfonic acid [(R)-3-((S)-2-ethyl-piperidin-1-yl)-2-hydroxypropyl]-amide (E96)

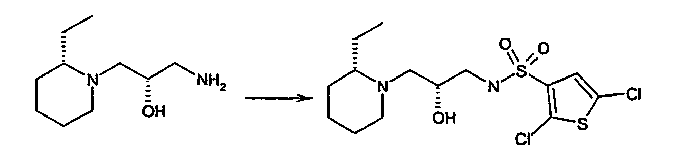

The title compound, prepared by the procedure of Example 90 was obtained as a colourless oil (85mg), m/z 401 [MH⁺].

### Example 97.

### 5-Methoxynaphthalene-1-sulfonic acid [(R)-3-((2R,6S)-2,6-dimethyl-piperidin-1-yl)-2-hydroxy-propyl]-amide (E97)

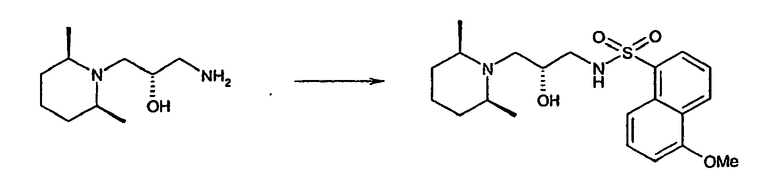

The title compound, prepared by the procedure of Example 73 from the products of Descriptions 56 and 73, was obtained as a colourless oil, m/z 407 [MH⁺].

### Example 98.

### 5-Cyanonaphthalene-1-sulfonic acid [(R)-3-((2R,6S)-2,6-dimethyl-piperidin-1-yl)-2-hydroxy-propyl]-amide (E98)

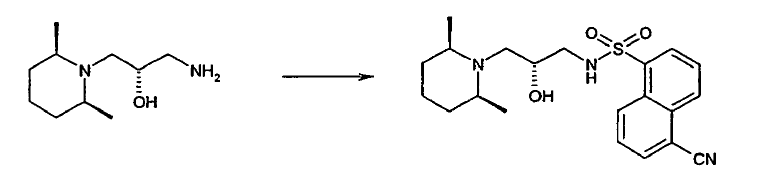

The title compound, prepared by the procedure of Example 73 from the products of Descriptions 56 and 74, was obtained as a colourless oil, m/z 402 [MH⁺].

### Example 99.

### 4-Cyanonaphthalene-1-sulfonic acid [(R)-3-((2R,6S)-2,6-dimethyl-piperidin-1-yl)-2-hydroxy-propyl]-amide (E99)

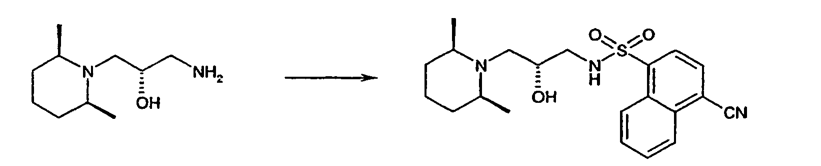

The title compound, prepared by the procedure of Example 73 from the products of Descriptions 56 and 75, was obtained as a colourless oil, m/z 402 [MH⁺].

### Example 100.

### 4-Bromonaphthalene-1-sulfonic acid [(R)-3-((2R,6S)-2,6-dimethyl-piperidin-1-yl)-2-hydroxy-propyl]-amide (E100)

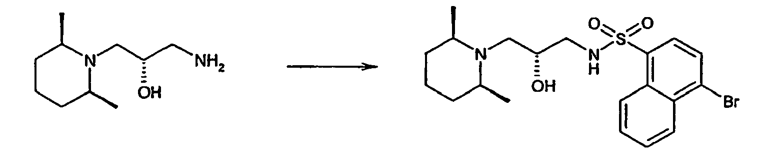

The title compound, prepared by the procedure of Example 73 from the product of Description 56 was obtained as a colourless oil, m/z 455, 457 [MH⁺].

### Example 101.

### 4-Fluoronaphthalene-1-sulfonic acid [(R)-3-((2R,6S)-2,6-dimethyl-piperidin-1-yl)-2-hydroxy-propyl]-amide (E101)

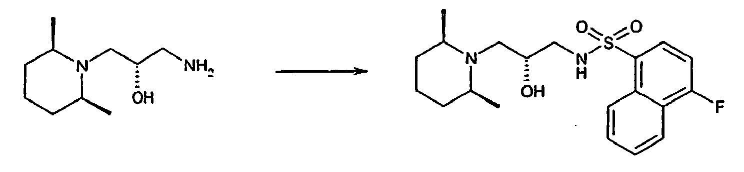

The title compound, prepared by the procedure of Example 73 from the product of Description 56 was obtained as a colourless oil, m/z 395 [MH⁺].

### Example 102

### N-[(R)-3-((2R,6S)-2,6-Dimethyl-piperidin-1-yl)-2-hydroxy-propyl]-2,3-dimethyl benzenesulfonamide (E102)

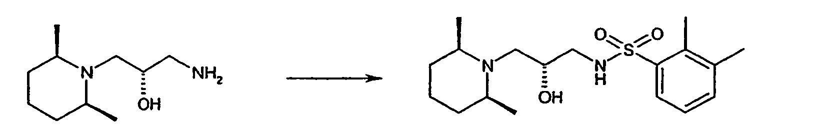

The title compound, prepared by the procedure of Example 73 from the product of Description 56 and 2,3-dimethylbenzenesulphonyl chloride (D78a), was obtained as a colourless oil, m/z 355 [MH⁺].

### Example 103.

### N-[(R)-3-((2R,6S)-2,6-Dimethyl-piperidin-1-yl)-2-hydroxy-propyl]-3,4-dimethyl-benzenesulfonamide (E103)

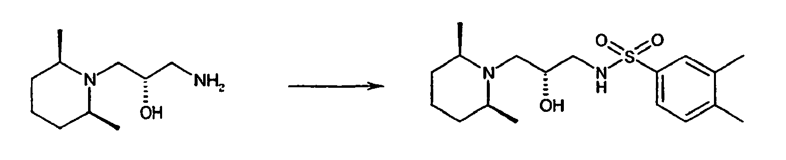

The title compound, prepared by the procedure of Example 73 from the product of Description 56 and 3,4-dimethylbenzenesulphonyl chloride (D78b), was obtained as a colourless oil, m/z 355 [MH⁺].

### Example 104.

### N-[(R)-3-((2R,6S)-2,6-Dimethyl-piperidin-1-yl)-2-hydroxy-propyl]-2,3-dimethoxy-benzenesulfonamide (E104)

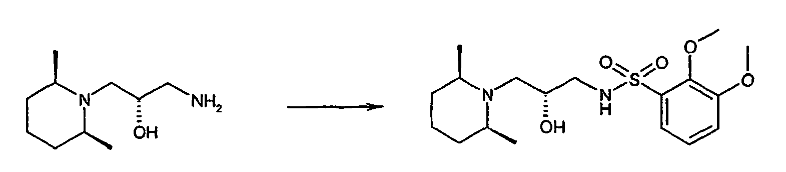

The title compound, prepared by the procedure of Example 73 from the product of Description 56 and 2,3-dimethoxybenzenesulphonyl chloride (US Patent 6,342,504), was obtained as a colourless oil, m/z 387 [MH⁺].

### Example 105.

### 7-Trifluoromethyl-quinoline-5-sulfonic acid [(R)-3-((2R,6S)-2,6-dimethyl-piperidin-1-yl)-2-hydroxy-propyl]-amide (E105)

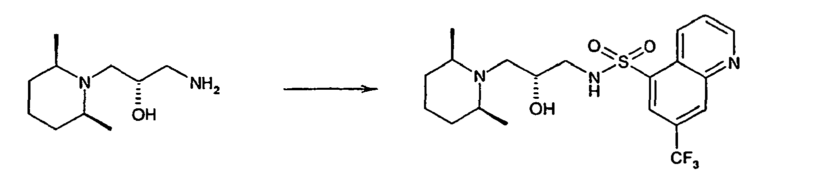

The title compound, prepared by the procedure of Example 73 from the product of Description 56 and 7-trifluoromethyl-quinoline-5-sulfonyl chloride (D79), was obtained as a colourless oil, m/z 446 [MH⁺]. δ_{H} (CDCl3) 1.02 (6H, br s), 1.2 - 1.7 (6H, br overlapping m), 2.44 (1 H, br m), 2.57 (3H, overlapping br m), 2.90 (1 H, dd, J = 12.5 and 5.2 Hz), 3.15 (1 H, dd, J = 12.5 and 4.0 Hz), 3.55 (1 H, br s), 7.70 (1 H, dd, J = 8.8 and 4.4 Hz), 8.45 (1 H, s), 8.63 (1 H, s), 9.08 (1 H, d, J = 8.8 Hz), 9.12 (1 H, d, J = 4.4 Hz) ppm.

### Example 106

### 7-Fluoro-2-methyl-quinoline-5-sulfonic acid [(R)-3-((2R,6S)-2,6-dimethyl-piperidin-1-yl)-2-hydroxy-propyl]-amide (E106)

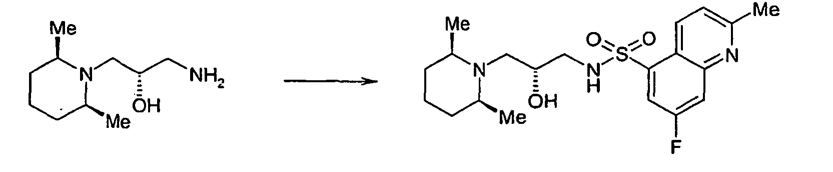

The title compound, prepared by the procedure of Example 73 from the products of Descriptions 56 and 80, was obtained as a colourless oil, m/z 410 [MH⁺]. δ_{H} (CDCl3) 1.01 (6H, t), 1.05-1.75 (6H, br overlapping m), 2.39 (1 H, dd, J = 14.4 and 5.2 Hz), 2.49 - 2.62 (3H, overlapping br m), 2.78 (3H, s), 2.87 (1 H, dd, J = 12.8 and 5.6 Hz), 3.11 (1H, dd, J = 12.8 and 4.0 Hz), 3.48-3.58 (1H, br m), 7.41 (1H, d, J = 8.8 Hz), 7.87 (1H, dd, J = 9.2 and 2.8 Hz), 8.01 (1H, dd, J = 8.0 and 2.8 Hz), 8.89 (1 H, d, J = 9.2 Hz) ppm.

## Claims

1. Use of a compound of formula (I): 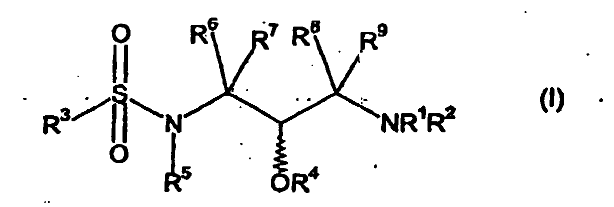 or a salt, solvate, ester or amide thereof, for the manufacture of a medicament for treating disorders mediated by GlyT1, wherein :
R¹ and R² are Independently selected from hydrogen, C₁-C₆ alkyl and C₃-C₆ cycloalkyl, with the proviso that R¹ and R² do net both represent hydrogen, or
R¹ and R² together with the nitrogen atom to which they are attached are linked to form a 4-. 5-, 6- or 7-membered saturated ring, wherein one or more of the carbon atoms is optionally replaced by a heteroatom Independently selected from N, O and
S, said saturated ring being optionally substituted by one or more groups. Independently selected from C₁-C₆ alkyl, C₃-C₆ cycloalkyl, C₃-C₆ cycloalkylC₁-C₄ alkyl, aryl and arylC₁-C₄ alkyl, and said saturated ring being further optionally bridged by a C₁-C₆ alkylene group, and said saturated ring being further optionally fused to a C₅-C₇ alicyclic or 5- or 6-membered aromatic or heteroaromatic ring optionally substituted by one or more groups independently selected from C₁-C₆ alkyl and C₃-C₆cycloalkyl;
R³ is 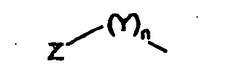 wherein
Y is C₁-C₂ alkylene. C₂ alkenylene or C₂ alkynylene,
and
n is 0 or 1, and
Z is a 5- to 8-membered monocyclic or 6- to 10-membered bicyclic aromatic ring system wherein one or more of the carbon atoms is optional replaced by a heteroatom independently selected from N, O and S, and said ring system being optionally substituted by one or more groups independently selected from -hal, R¹⁰, -CF₃, -C₁₋₆alkylsulphonyl, -OR¹¹, -COOR¹², -CN, -NO₂, -NR¹³R¹⁴, -C(O)NR¹⁵R¹⁶, -NR¹⁷C(O)R¹⁸, -C(O)R¹⁹, -C(NR²⁰)NR²¹R²², -C(NOR²³)R²⁸,
wherein
hal is F, Cl, Br or l,
R¹⁰ is C₁-C₆ alkyl, C₃-C₆ cycloalkyl, C₃-C₆ cycloalkylC₁-C₄ alkyl, aryl, aryloxy or aryl C₁-C₄ alkyl, optionally substituted by one or more groups Independently selected from hal, C₁-C₆ alkyl, -OR¹¹, -COOR¹²-CN, -NO₂ and -NR¹³R¹⁴,
R¹¹, R¹², R¹³, R¹⁴, R¹⁵, R¹⁶, R¹⁷, R¹⁸, R²⁰, R²¹, R²², R²³ and R²⁸ are independently selected from hydrogen and C₁-C₆ alkyl;
R⁴ and R¹⁹ are independently selected from hydrogen, C₁-C₆ alkyl, C₃-C₆ cycloalkyl, aryl or arylC₁-C₄ alkyl, optionally substituted by one or more groups independently selected from hal, C₁-C₆ alkyl, -OR²⁴, -COOR²⁵, CN, -NO₂ and -NR²⁶R²⁷;
R⁶, R⁷, R⁸ and R⁹ are independently selected from hydrogen, C₁-C₆ alkyl or arylC₁-C₄ alkyl, or R⁶ and R⁷ together form a C₃-C₆ cycloalkyl group, or R⁸ and R⁹ together form a C₃-C₆ cycloalkyl group; wherein the C₁-C₆ alkyl. arylC₁-C₄ alkyl group. the C₃-C₆ cycloalkyl group formed by R⁶ and R⁷. and the C₃-C₆ cycloalkyl group formed by R⁸ and R⁹ are optionally substituted by one or more groups independently selected from hal. C₁-C₆ alkyl, -OR²⁴, -COOR²⁵, -CN, -NO₂ and -NR²⁶R²⁷,
wherein R²⁴, R²⁵, R²⁶ and R²⁷ are independently selected form hydrogen and C₁-C₆ alkyl: and
R⁵ is independently selected from hydrogen. C₁-C₆ alkyl, C₃-C₆ cycloalkyl, aryl and aryl C₁-C₄ alkyl, optionally substituted by one or more groups independently selected from hall, C₁-C₆ alkyl-OR²⁴, -COOR²⁵, CN, -NO₂ and -NR²⁶R²⁷,
wherein R²⁴, R²⁵, R²⁶ and R²⁷ are, as herein before defined.
and wherein the term "aryl" refers to a 5- to 7-membered aromatic or heteroaromatic ring system wherein the heteroatomic ring contains at least one heteroatom selected from N. O and S.

2. The use as claimed in claim 1, wherein the compound has the following stereochemical configuration:

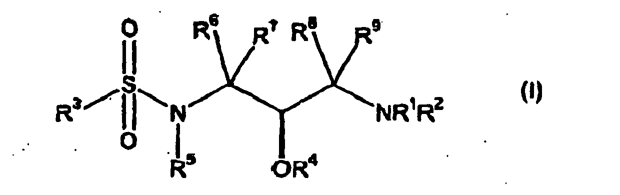

3. The use as claimed in claim 1 or claim 2, wherein:
(a) R¹ and R² together with the nitrogen atom to which they are attached are linked to form an optionally substituted 5- or 6-membered ring, wherein one or more of the carbon atoms is optionally replaced by a heteroatom independently selected from N, O and S, said ring being further optionally fused to a C₅-C₇ alicyclic or 5- or 6-membered aromatic or heteroaromatic ring: or
(b) R¹ and R² together with the nitrogen atom to which they are attached are linked to form a 5- or 6-membered heterocyclic ring, wherein the sate heteroatom is the nitrogen atom to which R¹ and R² are attached, said ring being optionally substituted by one or more groups independently selected from C₁-C₆ alkyl and C₃-C₆ cycloalkyl, more Preferably by one or more groups independently selected from C₁-C₄ alkyl, most preferably methyl, ethyl or isopropyl: or
(c) R¹ and R² are independently selected from C₁-C₆ alkyl, preferably C₃-C₆ alkyl.

4. The use as claimed in any of claims 1-3, wherein n is 0.

5. The use as claimed in any of claims 1-4, wherein Z is a 5- or 6-membered monocyclic ring system or Z is a 6- to 10-membered bicyclic ring system.

6. The use as claimed in claim 5 wherein Z is phenyl, thienyl, naphthyl, naphthyridinyl, quinolyl, isoquinolyl, benzothienyl, chromanyl, chromenyl, imidazoleisothiazolyl, benzothiadiazolyl or benzofuryl, optionally substituted by one or more groups independently selected from -hal, C₁-C₆ alkyl, C₁-C₆ alkoxy, CF₃, - CN and C₃-C₆ cycloalkyl.

7. The use as claimed in any of claims 1-6, wherein R⁴ is hydrogen or C₁-C₆ alkyl.

8. The use as claimed in any of claims 1-7, wherein R⁵ is selected from hydrogen, C₁-C₆ alkyl, aryl and benzyl, optionally substituted by one or more groups independently selected from hal, C₁-C₆ alkyl and OR²⁴.

9. The use as claimed in any of claims 1-8, wherein R⁶, R⁷, R⁸ and R⁹ are independently selected from hydrogen and C₁-C₆ alkyl.

10. Use of a compound of formula (Ia): 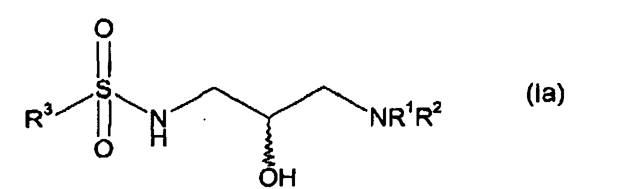 or a salt, solvate, ester or amide thereof, for the manufacture of a medicament for treating disorders mediated by GlyT1, wherein:
R¹ and R² are independently selected from C₃-C₆ alkyl, or
R¹ and R² together with the nitrogen atom to which they are attached are linked to form a 5-, 6-, or 7- membered heterocyclic ring, wherein the sole heteroatom is the nitrogen atom to which R¹ and R² are attached, said ring being optionally substituted by one or more groups independently selected from C₁-C₄ alkyl and C₃-C₆ cycloalkyl, and said ring being further optionally fused to a C₆ alicyclic or aromatic ring, and said ring being further optionally bridged by a methylene group;
R³ is 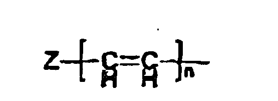 wherein
n is 0 or 1. and
Z is a 5- or 6-membered monocyclic or 8- to 10-membered bicyclic aromatic ring system,
wherein one or more of the carbon atoms is optionally replaced by a heteroatom independently selected from N, O and S, and said ring system being optionally substituted by one or more groups independently selected from -hal, -R¹⁰, -OR¹¹, -COOR¹², -CN, -NO₂, -NR¹³R¹⁴. -CF₃, and -C₁-₆alkylsulphonyl.
wherein
hal is F, Cl Br or I,
R¹⁰ is C₁-C₄ alkyl, phenyl or phenyloxy, optionally substituted by one or more had groups, and
R¹¹, R¹², R¹³ and R¹⁴ are independently selected from hydrogen and
methyl
and wherein the term "aryl" refers to a 5- to 7-membered aromatic or heteroaromatic ring system wherein the heteroatomic ring contains at least one heteroatom selected from N, O and S.

11. The use as claimed in claim 10, wherein when n is 1, the C₂ alkenylene group is in the *trans* configuration.

12. The use as claimed in claim 10 or claim 11, wherein the compound of formula (la) has the following stereochemical configuration:

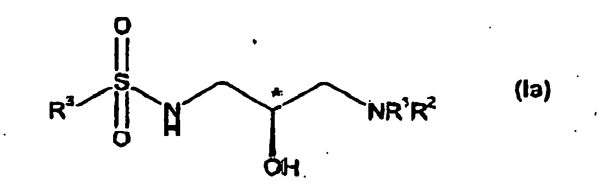

13. The use as claimed in any of claims 10, 11 or 12. wherein R¹ and R² together with the nitrogen atom to which they are attached are linked to form a pyrrolidinyl ring or a piperidinyl ring optionally substituted by one of more groups independently selected from C₁-C₄ alkyl, preferably methyl, ethyl or isopropyl.

14. The use as claimed in claim any of claims 10-13, wherein n is 0.

15. The use as claimed in any of claims 10-14. wherein Z is selected from 2- or 3-thienyl, phenyl, 5-quinolinyl, 1-naphthyl and 2-naphthyl, optionally substituted by one or more groups independently selected from -hal, -R¹⁰, -OR¹¹, -COOR¹², -CN,-NO₂, -NR¹³R¹⁴, -CF₃ and -C₁₋₆alkylsulphonyl.

16. The use as claimed in any of claims 10-15, wherein R³ is 1-naphthyl or 5-quinolinyl.

17. The use as claimed in any of claims 1-16, wherein the compound is selected from the group consisting of:
Naphthalene-1-sulfonic acid [(R)-2-hydroxy-3-piperidin-1-ylpropyl]-amide
Naphthalene-1-sulfonic acid {(2R)-hydroxy-3-[(2R,S)-methylpiperidin-1-yl]-propyl}-amide
Naphthalene-1-sulfonic acid {3-[(2R,6S)-dimethylpiperidin-1-yl]-(2R)-hydroxypropyl}-amide
Naphthalene-1-sulfonic acid {3-[(2S)-ethylpiperidin-1-yl]-(2R)-hydroxypropyl}-amide)
Naphthalene-1-sulfonic acid {3-[(2R)-ethylpiperidin-1-yl]-(2R)-hydroxypropyl}-amide
Naphthalene-1-sulfonic acid ((R)-2-hydroxy-3-pyrrolidin-1-ylpropyl)amide trifluoroacetate
Naphthalene-1-sulfonic acid [(R)-2-hydroxy-3-(2-methylpyrrolidin-1-yl)propyl]amide trifluoroacetate
Naphthalene-1-sulfonic acid [(R)-3-(2,4-dimethylpyrrolidin-1-yl)-2-hydroxypropyl]amide trifluoroacetate
Naphthalene-1-sulfonic acid [(R)-2-hydroxy-3-(2-isopropylpyrrolidin-1-yl)propyl]amide trifluoroacetate
Naphthalene-1-sulfonic acid [(R)-3-(2,5-dimethylpyrrolidin-1-yl)-2-hydroxypropyl]amide trifluoroacetate
Naphthalene-1-sulfonic acid[(R)-3-(2-cyclohexylpyrrolidin-1-yl)-2-hydroxypropyl]amide
Naphthalene-1-sulfonic acid[(R)-2-hydroxy-3-(2-isobutylpyrrolidin-1-yl)propyl]amide
Naphthalene-1-sulfonic acid[(R)-3-(2-ethylpyrrolidin-1-yl)-2-hydroxypropyl]amide trifluoroacetate
Naphthalene-1-sulfonic acid[(R)-3-(2-*tert*-butylpyrrolidin-1-yl)-2-hydroxypropyl]amide trifluoroacetate
Naphthalene-1-sulfonic acid[(R)-3-(2-cyclopropylpyrrolidin-1-yl)-2-hydroxypropyl]amide trifluoroacetate
Naphthalene-1-sulfonic acid[(R)-2-hydroxy-3-(3-methylpiperidin-1-yl)propyl]amide trifluoroacetate
Naphthalene-1-sulfonic acid[(R)-2-hydroxy-3-(5-ethyl-2-methylpiperidin-1-yl)propyl]amide
Naphthalene-1-sulfonic acid[(R)-2-hydroxy-3-(2-ethylpiperidin-1-yl)propyl]amide trifluoroacetate
Naphthalene-1-sulfonic acid[(R)-2-hydroxy-3-(2-isopropylpiperidin-1-yl)propyl]amide trifluoroacetate
Naphthalene-1-sulfonic acid[(R)-(sec-butylpropylamino)hydroxypropyl]amide trifluoroacetate
Naphthalene-1-sulfonic acid[(R)-(*tert*-butylpropylamino)hydroxypropyl]amide trifluoroacetate
Naphthalene-1-sulfonic acid[(R)-3-(1,3-dihydroisoindol-2-yl)-2-hydroxypropyl]amide trifluoroacetate
Naphthalene-1-sulfonic acid[(R)-2-hydroxy-3-(octahydroisoquinolin-2-yl)propyl]amide trifluoroacetate
Naphthalene-1-sulfonic acid[(R)-2-hydroxy-3-(octahydroquinolin-2-yl)propyl]amide
Naphthalene-1-sulfonic acid[(R)-2-hydroxy-3-((1S,5R)-1,3,3-trimethyl-6-azabicyclo[3.2.1]oct-6-yl)-propyl]amide trifluoroacetate
Naphthalene-1-sulfonic acid[(S)-3-(2,4-dimethylpyrrolidin-1-yl)-2-hydroxypropyl]amide trifluoroacetate
Naphthalene-1-sulfonic acid [(S)-2-hydroxy-3-(2-methylpyrrolidin-1-yl)propyl]amide trifluoroacetate
Naphthalene-1-sulfonic acid[(S)-2-hydroxy-3-(3-methylpiperidin-1-yl)propyl]amide trifluoroacetate
Naphthalene-1-sulfonic acid[3-(2,4-dimethylpyrrolidin-1-yl)-2-hydroxypropyl]amide 5-Dimethylamino-naphthalene-1-sulfonic acid [(R)-3-(2,4-dimethyl-pyrrolidin-1-yl)-2-hydroxy-propyl]-amide
Naphthalene-2-sulfonic acid [(R)-3-(2,4-dimethyl-pyrrolidin-1-yl)-2-hydroxy-propyl]-amide
2,5-Dichloro-N-[(R)-3-(2,4-dimethyl-pyrrolidin-1-yl)-2-hydroxy-propyl]-benzenesulfonamide
N-[(R)-3-(2,4-Dimethyl-pyrrolidin-1-yl)-2-hydroxy-propyl]-2-nitro-benzenesulfonamide
3,5-Dichloro-*N*-[(R)-3-(2,4-dimethyl-pyrrolidin-1-yl)-2-hydroxy-propyl]-2-hydroxy-benzenesulfonamide
*N*-[(R)-3-(2,4-Dimethyl-pyrrolidin-1-yl)-2-hydroxy-propyl]-2,4,6-triisopropyl-benzenesulfonamide
*N*-[(R)-3-(2,4-Dimethyl-pyrrolidin-1-yl)-2-hydroxy-propyl]-4-nitro-benzenesulfonamide
*N*-[(R)-3-(2,4-Dimethyl-pyrrolidin-1-yl)-2-hydroxy-propyl]-4-methoxy-benzenesulfonamide
(E)-2-Phenyl-ethenesulfonic acid [(R)-3-(2,4-dimethyl-pyrrolidin-1-yl)-2-hydroxypropyl]-amide
2-[(R)-3-(2,4-Dimethyl-pyrrolidin-1-yl)-2-hydroxy-propylsulfamoyl]-benzoic acid methyl ester
*N*-[(R)-3-(2,4-Dimethyl-pyrrolidin-1-yl)-2-hydroxy-propyl]-bis-trifluoromethyl-benzenesulfonamide
3,4-Dichloro-*N*-[(R)-3-(2,4-dimethyl-pyrrolidin-1-yl)-2-hydroxy-propyl]-benzenesulfonamide
*N*-[(R)-3-(2,4-Dimethyl-pyrrolidin-1-yl)-2-hydroxy-propyl]-4-propyl-benzenesulfonamide
4-Bromo-*N*-[(R)-3-(2,4-dimethyl-pyrrolidin-1-yl)-2-hydroxy-propyl]-2,5-difluoro-benzenesulfonamide
*N*-[(R)-3-(2,4-Dimethyl-pyrrolidin-1-yl)-2-hydroxy-propyl]-3-fluoro-benzenesulfonamide
4-Chloro-*N*-[(R)-3-(2,4-Dimethyl-pyrrolidin-1-yl)-2-hydroxy-propyl]-3-fluoro-benzenesulfonamide
2-Chloro-*N*-[(R)-3-(2,4-dimethyl-pyrrolidin-1-yl)-2-hydroxy-propyl]-benzenesulfonamide
2,5-Dichloro-thiophene-3-sulfonic acid [(R)-3-(2,4-dimethyl-pyrrolidin-1-yl)-2-hydroxypropyl]-amide
*N*-[(R)-3-(2,4-Dimethyl-pyrrolidin-1-yl)-2-hydroxy-propyl]-*C*-trifluoromethyl-benzenesulfonamide
*N*-[(R)-3-(2,4-Dimethyl-pyrrolidin-1-yl)-2-hydroxy-propyl]-3-methyl-benzenesulfonamide
2,3-Dichloro-*N*-[(R)-3-(2,4-dimethyl-pyrrolidin-1-yl)-2-hydroxy-propyl]-benzenesulfonamide
3-Bromo-5-chloro-thiophene-2-sulfonic acid [(R)-3-(2,4-dimethyl-pyrrolidin-1-yl)-2-hydroxy-propyl]-amide
2-Cyano-*N*-[(R)-3-(2,4-dimethyl-pyrrolidin-1-yl)-2-hydroxy-propyl]-benzenesulfonamide
*N*-[(R)-3-(2,4-Dimethyl-pyrrolidin-1-yl)-2-hydroxy-propyl]-2,5-difluoro-benzenesulfonamide
5-Bromo-2-chloro-*N*-[(R)-3-(2,4-dimethyl-pyrrolidin-1-yl)-2-hydroxy-propyl]-benzenesulfonamide
2,2,5,7,8-Pentamethyl-chroman-6-sulfonic acid [(R)-3-(2,4-dimethyl-pyrrolidin-1-yl)-2-hydroxy-propyl]-amide
Benzo[1,2,5]thiadiazole-4-sulfonic acid [(R)-3-(2,4-dimethyl-pyrrolidin-1 -yi)-2-hydroxy-propyl]-amide
Benzo[1,2,5]oxadiazole-4-sulfonic acid [(R)-3-(2,4-dimethyl-pyrrolidin-1-yl)-2-hydroxy-propyl]-amide
Biphenyl-4-sulfonic acid [(R)-3-(2,4-dimethyl-pyrrolidin-1-yl)-2-hydroxy-propyl]-amide
*N*-[(R)-3-(2,4-Dimethyl-pyrrolidin-1-yl)-2-hydroxy-propyl]-4-methyl-3-nitrobenzenesulfonamide
5-Chloro-3-methyl-benzo[b]thiophene-2-sulfonic acid [(R)-3-(2,4-dimethyl-pyrrolidin-1-yl)-2-hydroxy-propyl]-amide
4-Butyl-*N*-[(R)-3-(2,4-dimethyl-pyrrolidin-1-yl)-2-hydroxy-propyl]-benzenesulfonamide
5-Chloro-benzo[1,2,5]oxadiazole-4-sulfonic acid [(R)-3-(2,4-dimethyl-pyrrolidin-1-yl)-2-hydroxy-propyl]-amide
4-Butyl-*N*-[(R)-3-(2,4-dimethyl-pyrrolidin-1-yl)-2-hydroxy-propyl]-benzenesulfonamide
6-Chloro-imidazo[2,1-b]thiazole-5-sulfonic acid [(R)-3-(2,4-dimethyl-pyrrolidin-1-yl)-2-hydroxy-propyl]-amide
*N*-[(R)-3-(2,4-Dimethyl-pyrrolidin-1-yl)-2-hydroxy-propyl]-3-methoxy-benzenesulfonamide
5-lodo-naphthalene-1-sulfonic acid [(R)-3-(2,4-dimethyl-pyrrolidin-1-yl)-2-hydroxypropyl]-amide
2-Bromo-*N*-[(R)-3-(2,4-dimethyl-pyrrolidin-1-yl)-2-hydroxy-propyl]-benzenesulfonamide
*N*-[(R)-3-(2,4-Dimethyl-pyrrolidin-1-yl)-2-hydroxy-propyl]-5-fluoro-2-methyl-benzenesulfonamide
Naphthalene-1-sulfonic acid [(R)-3-(2,4-dimethyl-pyrrolidin-1-yl)-2-hydroxy-propyl]-amide
*N-*[(R)-3-(2,4-Dimethyl-pyrrolidin-1-yl)-2-hydroxy-propyl]-2,4,6-trimethyl-benzenesulfonamide
*N*-[(R)-3-(2,4-Dimethyl-pyrrolidin-1-yl)-2-hydroxy-propyl]-trifluoromethyl-benzenesulfonamide
Naphthalene-1-sulfonic acid [(R)-3-(2,6-diethylpiperidin-1-yl)-2-hydroxy-propyl]-amide
2,3-Dichloro-N-[(R)-3-((2S,6R)-2,6-dimethyl-piperidin-1-yl)-2-hydroxy-propyl]-benzenesulfonamide
2,3,4-Trichloro-*N*-[(R)-3-((2S,6R)-2,6-dimethyl-piperidin-1-yl)-2-hydroxy-propyl]-benzenesulfonamide
2,5-Dichlorothiophene-3-sulfonic acid [(R)-3-((2S,6R)-2,6-dimethyl-piperidin-1-yl)-2-hydroxy-propyl]-amide
4,5-Dibromo-thiophene-2-sulfonic acid [(R)-3-((2R,6S)-2,6-dimethyl-piperidin-1-yl)-2-hydroxy-propyl]amide
4-Bromo-2,5-dichloro-thiophene-3-sulfonic acid [(R)-3-((2S,6R)-2,6-dimethyl-piperidin-1-yl)-2-hydroxy-propyl]-amide
5-Chloro-naphthalene-1-sulfonic acid [(R)-3-((2S,6R)-2,6-dimethyl-piperidin-1-yl)-2-hydroxy-propyl]-amide
5-Chloro-naphthalene-2-sulfonic acid [(R)-3-((2S,6R)-2,6-dimethyl-piperidin-1-yl)-2-hydroxy-propyl]-amide
Naphthalene-2-sulfonic acid [(R)-3-((2S,6R)-2,6-dimethyl-piperidin-1-yl)-2-hydroxypropyl]-amide
N-[(R)-3-((2S,6R)-2,6-Dimethyl-piperidin-1-yl)-2-hydroxy-propyl]-benzenesulfonamide
4'-Chloro-biphenyl-4-sulfonic acid [(R)-3-((2S,6R)-2,6-dimethyl-piperidin-1-yl)-2-hydroxy-propyl]-amide
Biphenyl-4-sulfonic acid [(R)-3-((2S,6R)-2,6-dimethyl-piperidin-1-yl)-2-hydroxypropyl]-amide
*N*-[(R)-3-((2S,6R)-2,6-Dimethyl-piperidin-1-yl)-2-hydroxy-propyl]-4-phenoxy-benzenesulfonamide
3,4-Dichloro-*N*-[(R)-3-((2S,6R)-2,6-dimethyl-piperidin-1-yl)-2-hydroxy-propyl]-benzenesulfonamide
Quinoline-5-sulfonic acid [(R)-3-((2S,6R)-2,6-dimethyl-piperidin-1-yl)-2-hydroxypropyl]-amide
Quinoline-8-sulfonic acid [(R)-3-((2S,6R)-2,6-dimethyl-piperidin-1-yl)-2-hydroxypropyl]-amide
5-lodo-naphthalene-1-sulfonic acid [(R)-3-((2S,6R)-2,6-dimethyl-piperidin-1-yl)-2-hydroxy-propyl]-amide
5-Acetyl-naphthalene-1-sulfonic acid [(R)-3-((2S,6R)-2,6-dimethyl-piperidin-1-yl)-2-hydroxy-propyl]-amide
Isoquinoline-5-sulfonic acid [(R)-3-((S)-2-ethyl-piperidin-1-yl)-2-hydroxy-propyl]-amide
Quinoline-8-sulfonic acid [(R)-3-((S)-2-ethyl-piperidin-1-yl)-2-hydroxy-propyl]-amide
5-Chloro-3-methyl-benzo[b]thiophene-2-sulfonic acid [(R)-3-((S)-2-ethyl-piperidin-1-yl)-2-hydroxy-propyl]-amide
*N*-[(R)-3-((S)-2-Ethylpiperidin-1-yl)-2-hydroxy-propyl]-1-phenyl-methanesulfonamide
2,3-Dichloro-*N*-[(R)-3-((S)-2-ethyl-piperidin-1-yl)-2-hydroxy-propyl]-benzenesulfonamide
Thiophene-2-sulfonic acid [(R)-3-((S)-2-ethyl-piperidin-1-yl)-2-hydroxy-propyl]-amide 2,5-Dichlorothiophene-3-sulfonic acid [(R)-3-((S)-2-ethyl-piperidin-1-yl)-2-hydroxypropyl]-amide
5-Methoxynaphthalene-1-sulfonic acid [(R-3-((2R,6S)-2,6-dimethyl-piperidin-1-yl)-2-hydroxy-propyl]-amide
5-Cyanonaphthalene-1-sulfonic acid [(R)-3-((2R,6S)-2,6-dimethyl-piperidin-1-yl)-2-hydroxy-propyl]-amide
4-Cyanonaphthalene-1-sulfonic add [(R)-3-((2R,6S)-2,6-dimethyl-piperidin-1-yl)-2-hydroxy-propyl]-amide
4-Bromonaphthalene-1-sulfonic acid [(R)-3-((2R,6S)-2,6-dimethyl-piperidin-1-yl)-2-hydroxy-propyl]-amide
4-Fluoronaphthalene-1-suffonic acid [(R)-3-((2R,6S)-2,6-dimethyl-piperidin-1-yl)-2-hydroxy-propyl]-amide
N-[(R)-3-((2R,6S)-2,6-Dimethyl-piperidin-1-yl)-2-hydroxy-propyl]-2,3-dimethyl-benzenesulfonamide
N-[(R)-3-((2R,6S)-2,6-Dimethyl-piperidin-1-yl)-2-hydroxy-propyl]-3,4-dimethyl-benzenesulfonamide
N-[(R)-3-((2R,6S)-2,6-Dimethyl-piperidin-1-yl)-2-hydroxy-propyl]-2,3-dimethoxy-benzenesulfonamide
7-Trifluoromethyl-quinoline-5-sulfonic acid [(R)-3-((2R,6S)-2,6-dimethyl-piperidin-1-yl)-2-hydroxy-propyl]-amide
7-Fluoro-2-methyl-quinoline-5-sulfonic acid [(R)-3-((2R,6S)-2,6-dimethyl-piperidin-1-yl)-2-hydroxy-propyl]-amide
and salts, solvates esters and amides thereof.

18. A compound as defined in claim 17 or a salt, solvate ester or amide thereof for use in therapy, but not including
N-[3-(2,4-dimethyl-1-pyrrolidinyl)-2-hydroxypropyl]-1-naphthalenesulfonamide,
N-[2-hydroxy-3-(2-methyl-1-piperidinyl)propyl]-1-naphthalenesulfonamide,
N-[2-hydroxy-3-(1-piperidinyl)propyl]-1-naphthalenesulfonamide, and
N-[2-hydroxy-3-(1-pyrrolidinyl)propyl]-1-naphthalenesulfonamide,
and salts thereof.

19. A pharmaceutical composition comprising as active ingredient a compound as defined in claim 17 or a salt, solvate, ester or amide thereof, but not including
N-[3-(2,4-dimethyl-1-pyrrolidinyl)-2-hydroxypropyl]-1-naphthalenesulfonamide,
N-[2-hydroxy-3-(2-methyl-1-pipendinyl)propyl]-1-naphthalenesulfonamide,
N-[2-hydroxy-3-(1-piperidinyl)propyl]-1-naphthalenesulfonamide, and
N-[2-hydroxy-3-(1-pyrrolidinyl)propyl]-1-naphthalenesulfonamide, and salts thereof,
and at least one pharmaceutically acceptable carrier, diluent or excipient.

20. A compound as defined in claim 17 or a salt, solvate ester or amide thereof, but not including
4-amino-N-(2-hydroxy-3-piperidin-1-yl-propyl)-benzenesulfonamide,
N-[3-(2,4-dimethyl-1-pyrrolidinyl)-2-hydroxypropyl]-1-naphthalenesulfonamide,
N-[2-hydroxy-3-(2-methyl-1-piperidinyl)propyl]-1-naphthalenesulfonamide,
N-[2-hydroxy-3-(1-plperidinyt)propyl]-1-naphthalenesulfonamlde, and
N-[2-hydroxy-3-(1-pyrrolidinyl)propyl]-1-naphthalenesulfonamide, and salts thereof.

## Patentansprüche

1. Verwendung einer Verbindung der Formel (I): 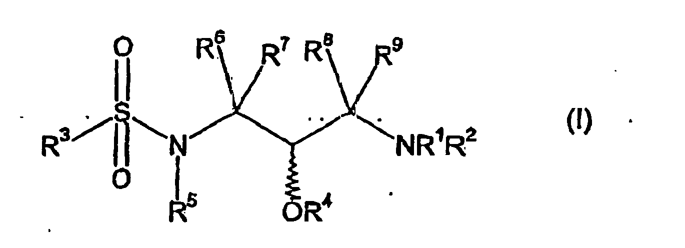 oder eines Salzes, Solvats, Esters oder Amids davon, zur Herstellung eines Medikaments zur Behandlung von durch GlyT1 vermittelten Störungen, wobei:
R¹ und R² unabhängig ausgewählt sind aus Wasserstoff, C₁-C₆-Alkyl und C₃-C₆-Cycloalkyl, mit der MaBgabe, dass R¹ und R² nicht beide Wasserstoff darstellen, oder
R¹ und R² zusammen mit dem Stickstoffatom, an das sie gebunden sind, verbunden sind, um einen 4-, 5-, 6- oder 7-gliedrigen gesättigten Ring zu bilden, wobei eines oder mehrere der Kohlenstoffatome gegebenenfalls durch ein Heteroatom ersetzt ist, unabhängig ausgewählt aus N, O und S, wobei der gesättigte Ring gegebenenfalls mit einem oder mehreren Resten substituiert ist; unabhängig ausgewählt aus C₁-C₆-Alkyl, C₃-C₆-Cycloalkyl, C₃-C₆-Cycloalkyl-C₁-C₄-alkyl, Aryl und Aryl-C₁-C₄-alkyl, und der gesättigte Ring ferner gegebenenfalls durch einen C₁-C₃-Alkylenrest verbrückt ist und der gesättigte Ring ferner gegebenenfalls an einen C₅-C₇ alicyclischen Ring oder einen 5-der gesättigte Ring ferner gegebenenfalls an einen C₅-C₇ Ring oder einen 5-oder 6-gliedrigen aromatischen oder heteroaromatischen Ring kondensiert ist, welcher gegebenenfalls mit einem oder mehreren Resten, unabhängig ausgewählt aus C₁-C₆-Alkyl und C₃-C₆-Cycloalkyl, substituiert ist;
R³ 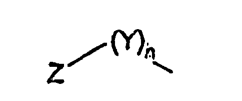 ist, wobei
Y C₁-C₂-Alkylen, C₂-Alkenylen oder C₂-Alkinylen ist,
und
n 0 oder 1 ist, und
Z ein 5- bis 8-gliedriges monocyclisches oder 6- bis 10-gliedriges bicyclisches aromatisches Ringsystem ist, wobei eines oder mehrere der Kohlenstoffatome gegebenenfalls durch ein Heteroatom ersetzt ist, unabhängig ausgewählt aus N, O und S, und wobei das Ringsystem gegebenenfalls mit einem oder mehreren Resten substituiert ist, unabhängig ausgewählt aus -hal, -R¹⁰, -CF₃, -C₁₋₆-Alkylsulfonyl, -OR¹¹, -COOR¹², -CN, -NO₂, -NR¹³R¹⁴, -C(O)NR¹⁵R¹⁶, -NR¹⁷C(O)R¹⁸, -C(O)R¹⁹, -C(NR²⁰)NR²¹R²², -C(NOR²³)R²⁸,
wobei
hal F, Cl, Br oder I ist,
R¹⁰ C₁-C₆-Alkyl, C₃-C₆-Cycloalkyl, C₃-C₆-Cycloalkyl-C₁-C₄-alkyl, Aryl, Aryloxy oder Aryl-C₁-C₄-alkyl ist, gegebenenfalls substituiert mit einem oder mehreren Resten, unabhängig ausgewählt aus hal, C₁-C₆-Alkyl, -OR¹¹, -COOR¹², -CN, -NO₂ und -NR¹³R¹⁴,
R¹¹, R¹², R¹³, R¹⁴, R¹⁵, R¹⁶, R¹⁷, R¹⁸, R²⁰, R²¹, R²², R²³ und R²⁸ unabhängig ausgewählt sind aus Wasserstoff und C₁-C₆-Alkyl;
R⁴ und R¹⁹ unabhängig ausgewählt sind aus Wasserstoff, C₁-C₆-Alkyl, C₃-C₆-Cycloalkyl, Aryl oder Aryl-C₁-C₄-alkyl, gegebenenfalls substituiert mit einem oder mehreren Resten, unabhängig ausgewählt aus hal, C₁-C₆-Alkyl, -OR²⁴, -COOR²⁵, -CN, -NO₂ und -NR²⁶R²⁷;
R⁶, R⁷ R⁸ und R⁹ unabhängig ausgewählt sind aus Wasserstoff, C₁-C₆-Alkyl oder Aryl-C₁-C₄-Alkyl, oder R⁶ und R⁷ zusammen einen C₃-C₆-Cycloalkylrest bilden, oder R⁸ und R⁹ zusammen einen C₃-C₆-Cycloalkylrest bilden, wobei der C₁-C₆-Alkylrest, Aryl-C₁-C₄-alkylrest, der durch R⁶ und R⁷ gebildete C₃-C₆-Cycloalkylrest und der durch R⁸ und R⁹ gebildete C₃-C₆-Cycloakylrest gegebenenfalls mit einem oder mehreren Resten substituiert sind, unabhängig ausgewählt aus hal, C₁-C₆-Alkyl, -OR²⁴, -COO²⁵, -CN, - NO₂ und -NR²⁶R²⁷,
wobei R²⁴, R²⁵, R²⁶ und R²⁷ unabhängig ausgewählt sind aus Wasserstoff und C₁-C₆-Alkyl;
und
R⁵ unabhängig ausgewählt ist aus Wasserstoff, C₁-C₆-Alkyl, C₃-C₆-Cycloalkyl, Aryl und Aryl-C₁-C₄-alkyl, gegebenenfalls substituiert mit einem oder mehreren Resten, unabhängig ausgewählt aus hal, C₁-C₆-Alkyl, -OR²⁴, -COOR²⁵, -CN, -NO₂ und -NR²⁶R²⁷,
wobei R²⁴, R²⁵, R²⁶ und R²⁷ wie vorstehend definiert sind,
und wobei der Begriff "Aryl" ein 5- bis 7-gliedriges aromatisches oder heteroaromatisches Ringsystem betrifft, wobei der heteroaromatische Ring mindestens ein Heteroatom enthält, ausgewählt aus N, O und S.

2. Verwendung gemäß Anspruch 1, wobei die Verbindung die folgende stereochemische Konfiguration aufweist:

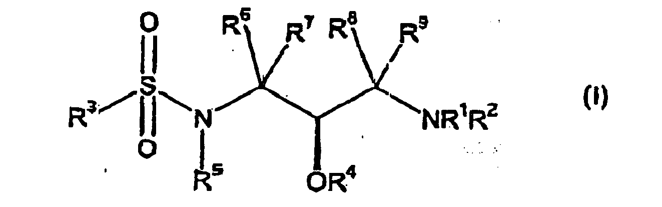

3. Verwendung gemäß Anspruch 1 oder Anspruch 2, wobei:
(a) R¹ und R² zusammen mit dem Stickstoffatom, an das sie gebunden sind, verbunden sind, um einen gegebenenfalls substituierten 5- oder 6-gliedrigen Ring zu bilden, wobei eines oder mehrere der Kohlenstoffatome gegebenenfalls durch ein Heteroatom ersetzt ist, unabhängig ausgewählt aus N, O und S, wobei der Ring ferner gegebenenfalls an einen C₅-C₇ alicyclischen Ring oder einen 5- oder 6-gliedrigen aromatischen oder heteroaromatischen Ring kondensiert ist; oder
(b) R¹ und R² zusammen mit dem Stickstoffatom, an das sie gebunden sind, verbunden sind, um einen 5- oder 6-gliedrigen heterocyclischen Ring zu bilden, wobei das einzige Heteroatom das Stickstoffatom ist, an das R¹ und R² gebunden sind, wobei der Ring gegebenenfalls mit einem oder mehreren Resten, unabhängig ausgewählt aus C₁-C₆-Alkyl und C₃-C₆-Cycloalkyl, stärker bevorzugt mit einem oder mehreren Resten, unabhängig ausgewählt aus C₁-C₄-Alkyl, besonders bevorzugt Methyl, Ethyl oder Isopropyl, substituiert ist; oder
(c) R¹ und R² unabhängig ausgewählt sind aus C₁-C₆-Alkyl, vorzugsweise C₃-C₆-Alkyl.

4. Verwendung gemäß einem der Ansprüche 1 bis 3, wobei n 0 ist.

5. Verwendung gemäß einem der Ansprüche 1 bis 4, wobei Z ein 5- oder 6-gliedriges monocyclisches Ringsystem ist oder Z ein 6- bis 10-gliedriges bicyclisches Ringsystem ist.

6. Verwendung gemäß Anspruch 5, wobei Z Phenyl, Thienyl, Naphthyl, Naphthyridinyl, Chinolyl, Isochinolyl, Benzothienyl, Chromanyl, Chromenyl, Imidazolisothiazolyl., Benzothiadiazolyl oder Benzofuryl ist, gegebenenfalls substituiert mit einem oder mehreren Resten, unabhängig ausgewählt aus -hal, C₁-C₆-Alkyl, C₁-C₆-Alkoxy, CF₃, -CN und C₃-C₆-Cycloalkyl.

7. Verwendung gemäß einem der Ansprüche 1 bis 6, wobei R⁴ Wasserstoff oder C₁-C₆-Alkyl ist.

8. Verwendung gemäß einem der Ansprüche 1 bis 7, wobei R⁵ ausgewählt ist aus Wasserstoff, C₁-C₆-Alkyl, Aryl und Benzyl, gegebenenfalls substituiert mit einem oder mehreren Resten, unabhängig ausgewählt aus hal, C₁-C₆-Alkyl und OR²⁴.

9. Verwendung gemäß einem der Ansprüche 1 bis 8, wobei R⁶, R⁷, R⁸ und R⁹ unabhängig ausgewählt sind aus Wasserstoff und C₁-C₆-Alkyl.

10. Verwendung einer Verbindung der Formel (la): 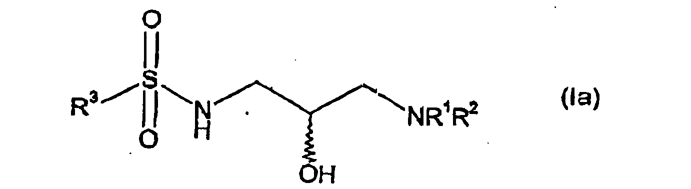 oder eines Salzes, Solvats, Esters oder Amids davon, zur Herstellung eines Medikaments zur Behandlung von durch GlyT1 vermittelten Störungen, wobei:
R¹ und R² unabhängig ausgewählt sind aus C₃-C₆-Alkyl, oder
R¹ und R² zusammen mit dem Stickstoffatom, an das sie gebunden sind, verbunden sind, um einen 5-, 6- oder 7-gliedrigen heterocyclischen Ring zu bilden, wobei das einzige Heteroatom das Stickstoffatom ist, an das R¹ und R² gebunden sind, wobei der Ring gegebenenfalls mit einem oder mehreren Resten substituiert ist, unabhängig ausgewählt aus C₁-C₄-Alkyl und C₃-C₆-Cycloalkyl, und der Ring ferner gegebenenfalls an einen C₆ alicyclischen oder aromatischen Ring kondensiert ist, und wobei der Ring ferner gegebenenfalls durch eine Methylengruppe verbrückt ist;
R³ 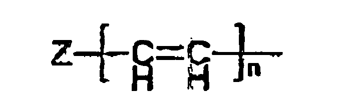 ist,
wobei
n 0 oder 1 ist, und
Z ein 5- oder 6-gliedriges monocyclisches oder 8- bis 10-gliedriges bicyclisches aromatisches Ringsystem ist,
wobei eines oder mehrere Kohlenstoffatome gegebenenfalls durch ein Heteroatom ersetzt ist, unabhängig ausgewählt aus N, O und S, und das Ringsystem gegebenenfalls mit einem oder mehreren Resten substituiert ist, unabhängig ausgewählt aus -hal, -R¹⁰, -OR¹¹, -COOR¹², -CN, -NO₂, -NR¹³R¹⁴, -CF₃ und -C₁₋₆-Alkylsulfonyl,
wobei
hal F, Cl, Br oder I ist,
R¹⁰ C₁-C₄-Alkyl, Phenyl oder Phenyloxy ist, gegebenenfalls substituiert mit einem oder mehreren hal-Resten, und
R¹¹, R¹², R¹³ und R¹⁴ unabhängig ausgewählt sind aus Wasserstoff und Methyl,
und wobei der Begriff "Aryl" ein 5- bis 7-gliedriges aromatisches oder heteroaromatisches Ringsystem betrifft, wobei der heteroaromatische Ring mindestens ein Heteroatom enthält, ausgewählt aus N, O und S.

11. Verwendung gemäß Anspruch 10, wobei, wenn n 1 ist, der C₂-Alkenylenrest in traus-Konfiguration vorliegt.

12. Verwendung gemäß Anspruch 10 oder Anspruch 11, wobei die Verbindung der Formel (Ia) die folgende stereochemische Konfiguration aufweist:

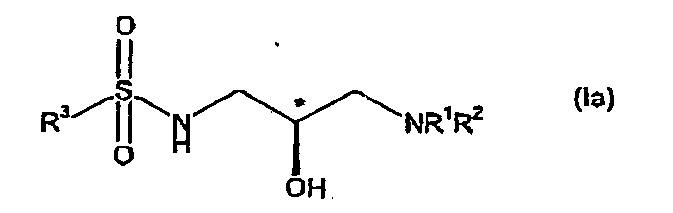

13. Verwendung gemäß einem der Ansprüche 10, 11 oder 12, wobei R¹ und R² zusammen mit dem Stickstoffatom, an das sie gebunden sind, verbunden sind, um einen Pyrrolidinylring oder einen Piperidinylring zu bilden, gegebenenfalls substituiert mit einem oder mehreren Resten, unabhängig ausgewählt aus C₁-C₄-Alkyl, vorzugsweise Methyl, Ethyl oder Isopropyl.

14. Verwendung gemäß einem der Ansprüche 10 bis 13, wobei n 0 ist.

15. Verwendung gemäß einem der Ansprüche 10 bis 14, wobei Z ausgewählt ist aus 2- oder 3-Thienyl, Phenyl, 5-Chinolinyl, 1-Naphthyl und 2-Naphthyl, gegebenenfalls substituiert mit einem oder mehreren Resten, unabhängig ausgewählt aus -hal, -R¹⁰, -OR¹¹, -COOR¹², -CN, NO₂, NR¹³R¹⁴, -CF₃ und C₁₋₆-Alkylsulfonyl.

16. Verwendung gemäß einem der Ansprüche 10 bis 15, wobei R³ 1-Naphthyl oder 5-Chinolinyl ist.

17. Verwendung gemäß einem der Ansprüche 1 bis 16, wobei die Verbindung ausgewählt ist aus:
Naphthalin-1-sulfonsäure-[(R)-2-hydroxy-3-piperidin-1-ylpropyl]amid,
Naphthalin-1-sulfonsäure-{(2R)-hydroxy-3-[(2R,S)-methylpiperidin-1-yl]propyl}amid,
Naphthalin-1-sulfonsäure-{3-[(2R,6S)-dimethylpiperidin-1-yl]-(2R)-hydroxypropyl}amid,
Naphthalin-1-sulfonsäure-{3-[(2S)-ethylpiperidin-1-yl]-(2R)-hydroxypropyl}amid),
Naphthaün-1-sulfonsäure-{3-[(2R)-ethylpiperidin-1-yl]-(2R)-hydroxypropyl}amid,
Naphthalin-1-sulfonsäure-((R)-2-hydroxy-3-pyrrolidin-1-ylpropyl)amidtrifluoracetat,
Naphthalin-1-sulfonsäure-[(R)-2-hydroxy-3-(2-methylpyrrolidin-1-yl)propyl]amidtrifluoracetat,
Naphthalin-1-sulfonsäure-[(R)-3-(2,4-dimethylpyrrolidin-1-yl)-2-hydroxypropyl]amidtrifluoracetat,
Naphthalin-1-sulfonsäure-[(R)-2-hydroxy-3-(2-isopropylpyrrolidin-1-yl)propyl]amidtrifluoracetat,
Naphthalin-1-sulfonsäure-[(R)-3-(2,5-dimethylpyrrolidin-1-yl)-2-hydroxypropyl]amidtrifluoracetat,
Naphthalin-1-sulfonsäure-[(R)-3-(2-cyclohexylpyrrolidin-1-yl)-2-hydroxypzopyl]amid,
Naphthalin-1-sulfonsäure-[(R)-2-hydroxy-3-(2-isobutylpyrrolidin-1-yl)propyl]amid,
Naphthalin-1-sulfonsäure-[(R)-3-(2-ethylpyrrolidin-1-yl)-2-hydroxypropyl]amidtrifluoracetat,
Naphthalin-1-sulfonsäure-[(R)-3-(2-tert-butylpyrrolidin-1-yl)-2-hydroxypropyl]amidtrifluoracetate,
Naphthalin-1-sulfonsäure-[(R)-3-(2-cyclopropylpyrrolidin-1-yl)-2-hydroxypropyl]amidtrifluoracetat,
Naphthatin-1-sulfonsäure-[(R)-2-hydroxy-3-(3-methylpiperidin-1-yl)propyl]amidtrifluoracetat,
Naphthalin-1-sulfonsäute-[(R)-2-hydroxy-3-(5-ethyl-2-methylpiperidin-1-yl)propyl]-amid,
Naphthalin-1-sulfonsäure-[(R)-2-hydroxy-3-(2-ethylpiperidin-1-yl)propyl]amidtrifluoracetat,
Naphthalin-1-sulfonsäure-[(R)-2-hydroxy-3-(2-isopropylpiperidin-1-yl)propyl]amidtrifluoracetat,
Naphthalin-1-sulfonsäure-[(R)-(sec-butylpropylamino)hydroxypropyl]amidtrifluoracetat,
Naphthalin-1-sulfonsäure-[(R)-(tert-butylpropylamino)hydroxypropyl]amidtrifluoracetat,
Naphthalin-1-sulfonsäure-[(R)-3-(1,3-dihydroisoindol-2-yl)-2-hydroxypropyl]amidtrifluoracetat,
Naphthalin-1-sulfonsäure-[(R)-2-hydroxy-3-(octahydroisochinolin-2-yl)propyl]amidtrifluoracetat,
Naphthalin-1-sulfonsäure-[(R)-2-hydroxy-3-(octahydrochinolin-2-yl)propyl]amid,
Naphthalin-1-sulfonsäure-[(R)-2-hydroxy-3-((1S,5R)-1,3,3-trimethyl-6-azabicyclo-[3.2.1]-oct-6-yl)propyl]amidtrifluoracetat,
Naphthalin-1-sulfonsäure-[(S)-3-(2,4-dimethylpyrrolidin-1-yl)-2-hydroxypropyl]amidtrifluoracetat,
Naphthalin-1-sulfonsäure[(S)-2-hydroxy-3-(2-methylpyrrolidin-1-yl)propyl]amidtrifluoracetat,
Naphthalin-1-sulfonsäure-[(S)-2-hydroxy-3-(3-methylpiperidin-1-yl)propyl]amidtrifluoracetat,
Naphthalin-1-sulfonsäure-[3-(2,4-dimethylpyrrolidin-1-yl)-2-hydroxypropyl]amid,
5-Dimethylaminonaphthalin-1-sulfonsäure-[(R)-3-(2,4-dimethylpyrrolidin-1-yl)-2-hydroxypropyl]amid,
Naphthalin-2-sulfonsäure-[(R)-3-(2,4-dimethylpyrrolidin-1-yl)-2-hydroxypropyl]amid,
2,5-Dichlor-N-[(R)-3-(2,4-dimethylpyrrolidin-1-yl)-2-hydroxypropyl]benzelsulfonamid,
N-[(R)-3-(2,4-Dimethylpyrrolidin-1-yl)-2-hydroxypropyl]-2-nitrobenzolsulfonamid,
3,5-Dichlor-N-[(R)-3-(2,4-dimethylpyrrolidin-1-yl)-2-hydroxypropyl]-2-hydroxybenzolsulfonamid,
N-[(R)-3-(2,4-Dimethylpyrrolidin-1-yl)-2-hydroxypropyl]-2,4,6-triisopropylbenzolsulfonamid,
N-[(R)-3-(2,4-Dimethylpyrrolidin-1-yl)-2-hydroxypropyl]-4-nitrobenzolsulfonamid,
N-[(R)-3-(2,4-Dimethylpyrrolidin-1-yl)-2-hydroxypropyl]-4-methoxybenzolsulfonamid,
(E)-2-Phenylethensulfonsäure-[(R)-3-(2,4-dimethylpyrrolidin-1-yl)-2-hydroxypropyl]-amid,
2-[(R)-3-(2,4-Dimethylpyrrolidin-1-yl)-2-hydroxypropylsulfamoyl]benzoesäuremethylester,
N -[(R)- 3-(2,4-Dimethylpyrrolidin-1-yl)-2-hydroxypropyl]-bis-trifluormethylbenzolsulfonamid,
3,4-Dichlor-N-[(R)-3-(2,4-dimethylpyrrolidin-1-yl)-2-hydroxypropyl]benzolsulfonamid,
N-[(R)-3-(2,4-Dimethylpyrrolidin-1-yl)-2-hydroxypropyl]-4-propylbenzolsulfonamid,
4-Brom-N-[(R)-3-(2,4-dimethylpyrrolidin-1-yl)-2-hydroxypropyl]-2,5-difluor-benzolsulfonamid,
N-[(R)-3-(2,4-Dimethylpyrrolidin-1-yl)-2-hydroxypropyl]-3-fluorbenzolsulfonamid,
5-Iodnaphthalin-1-sulfonsäure-[(R)-3-(2,4-dimethylpyrrolidin-1-yl)-2-hydroxypropyl]-amid,
2-Brom-N-[(R)-3-(2,4-dimethylpyrrolidin-1-yl)-2-hydroxypropyl]benzolsulfonamid,
N-[(R)-3-(2,4-Dimethylpyrrolidin-1-yl)-2-hydroxypropyl]-5-fluor-2-methylbenzolsulfonamid,
Naphthalin-1-sulfonsäure-[(R)-3-(2,4-dimethylpyrrolidin-1-yl)-2-hydroxypropyl]amid,
N-[(R)-3-(2,4-Dimethylpyrrolidin-1-yl)-2-hydroxypropyl]-2,4,6-trimethylbenzolsulfonamid,
N-[(R)-3-(2,4-Dimethylpyrrolidin-1-yl)-2-hydroxypropyl]trifluormethylbenzolsulfonamid,
Naphthalin-1-sulfonsäure-[(R)-3-(2,6-diethylpiperidin-1-yl)-2-hydroxypropyl]amid,
2,3-Dichlor-N-[(R)-3-((2S,6R)-2,6-dimethylpiperidin-1-yl)-2-hydroxypropyl]benzolsulfonamid,
2,3,4-Trichlor-N-[(R)-3-((2S,6R)-2,6-dimethylpiperidin-1-yl)-2-hydroxypropyl]benzolsulfonamid,
2,5-Dichlorthiophen-3-sulfonsäure-[(R)-3-((2S,6R)-2,6-dimethylpiperidin-1-yl)-2-hydroxypropyl]amid,
4,5-Dibromthiophen-2-sulfonsäure-[(R)-3-((2R,6S)-2,6-dimethylpiperidin-1-yl)-2-hydroxypropyl]amid,
4-Brom-2,5-dichlorthiophen-3-sulfonsäure-[(R)-3-((2S,6R)-2,6-dimethylpiperidin-1-yl)-2-hydroxypropyl]amid,
5-Chlornaphthalin-1-sulfonsäure-[(R)-3-((2S,6R)-2,6-dimethylpiperidin-1-yl)-2-hydroxypropyl]amid,
5-Chlornaphthalin-2-sulfonsäure-[(R)-3-((2S,6R)-2,6-dimethylpiperidin-1-yl)-2-hydroxypropyl]amid,
Naphthalin-2-sulfonsäure-[(R)-3-((2S,6R)-2,6-dimethylpiperidin-1-yl)-2-hydroxypropyl]-amid,
N-[(R)-3-((2S,6R)-2,6-Dimethylpiperidin-1-yl)-2-hydroxypropyl]benzolsulfonamid,
4'-Chlorbiphenyl-4-sulfonsäure-[(R)-3-((2S,6R)-2,6-dimethylpiperidin-1-yl)-2-hydroxypropyl]amid,
Biphenyl-4-sulfonsäure-[(R)-3-((2S,6R)-2,6-dimethylpiperidin-1-yl)-2-hydroxypropyl]-amid,
N-[(R)-3-((2S,6R)-2,6-Dimethylpiperidin-1-yl)-2-hydroxypropyl]-4-phenoxybenzolsulfonamid,
3 ,4-Dichlor-N-[(R)-3-((2S,6R)-2,6-dimethylpiperidin-1-yl)- 2-hydroxypropyl]-benzolsulfonamid,
Chinolin-5-sulfonsäure-[(R)-3-((2S,6R)-2,6-dimethylpiperidin-1-yl)-2-hydroxypropyl]-amid,
Chinolin-8-sulfonsäure-[(R)-3-((2S,6R)-2,6-dimethylpiperidin-1-yl)-2-hydroxypropyl]-amid,
5-lodnaphthalin-1-sulfonsäure-[(R)-3-((2S,6R)-2,6-dimethylpiperidin-1-yl)-2-hydroxypropyl]amid,
5-Acetylnaphthalin-1-sulfonsäure-[(R)-3-((2S,6R)-2,6-dimethylpiperidin-1-yl)-2-hydroxypropyl]amid,
Isochinolin-5-sulfonsäure-[(R)-3-((S)-2-ethylpiperidin-1-yl)-2-hydroxypropyl]amid,
Chinolin-8-sulfonsäure-[(R)-3-((S)-2-ethylpiperidin-1-yl)-2-hydroxypropyl]amid,
5-Chlor-3-methylbenzo[b]thiophen-2-sulfonsäure-[(R)-3-((S)-2-ethylpiperidin-1-yl)-2-hydroxypropyl]amid,
N-[(R)-3-((S)-2-Ethylpiperidin-1-yl)-2-hydroxypropyl]-1-phenylmethansulfonamid,
2,3-Dichlor-N-[(R)-3-((S)-2-ethylpiperidin-1-yl)-2-hydroxypropyl]benzolsulfonamid,
Thiophen-2-sulfonsäure-[(R)-3-((S)-2-ethylpiperidin-1-yl)-2-hydroxypropyl]amid,
2,5-Dichlorthiophen-3-sulfonsäure-[(R)-3-((S)-2-ethylpiperidin-1-yl)-2-hydroxypropyl]-amid,
5-Methoxynaphthalin-1-sulfousäure-[(R)-3-((2R,6S)-2,6-dimethylpiperidin-1-yl)-2-hydroxypropyl]amid,
5-Cyanonaphthalin-1-sulfonsäure-[(R)-3-((2R,6S)-2,6-dimethylpiperidin-1-yl)-2-hydroxypropyl]amid,
4-cyanonaphthalin-1-sulfonsäure-[(R)-3-((2R,6S)-2,6-dimethylpiperidin-1-yl)-2-hydroxypropyl] amid,
4-Bromnaphthalin-1-sulfonsäure-[(R)-3-((2R,6S)-2,6-dimethylpipendin-1-yl)-2-hydroxypropyl]amid,
4-Fluomaphthalin-1-sulfonsäure-[(R)-3-((2R,6S)-2,6-dimethylpiperidin-1-yl)-2-hydroxypropyl]amid,
N-[(R)-3-((2R,6S)-2,6-Dimethylpipendin-1-yl)-2-hydroxy-propyl]-2,3-dimethylbenzolsulfonamid,
N-[(R)-3-((2R,6S)-2,6-Dimethylpiperidin-1-yl)-2-hydroxypropyl]-3,4-dimethylbenzolsulfonamid,
N-[(R)-3-((2R,6S)-2,6-Dimethylpiperidin-1-yl)-2-hydroxypropyl]-2,3-dimethoxybenzolsulfonamid,
7-Trifluormethylchinolin-5-sulfonsäure-[(R)-3-((2R,6S)-2,6-dimethylpiperidin-1-yl)-2-hydroxypropyl]amid,
7-Fluor-2-methylchinolin-5-sulfonsäure-[(R)-3-((2R,6S)-2,6-dimethylpiperidin-1-yl)-2-hydroxypropyl]amid
und Salzen, Solvaten, Estern und Amiden davon.

18. Verbindung wie in Anspruch 17 definiert oder ein Salz, Solvat, Ester oder Amid davon, zur Verwendung in der Therapie, aber nicht einschließlich
N-[3-(2,4-Dimethyl-1-pyrrolidinyl)-2-hydroxypropyl]- 1-naphthalinsulfonamid,
N-[2-Hydroxy-3-(2-methyl-1-piperidinyl)propyl]-1-naphthalinsulfönamid,
N-[2-Hydroxy-3-(1-piperidinyl)propyl]-1-naphthalinsulfonamid, und
N-[2-Hydroxy-3-(1-pyrrolidinyl)propyl]-1-naphthalinsulfonamid,
und Salze davon.

19. Arzneimittel, umfassend als Wirkstoff eine Verbindung wie in Anspruch 17 definiert, oder ein Salz, Solvat, Ester oder Amid davon, aber nicht einschließlich
N-[3-(2,4-Dimethyl-1-pyrrolidinyl)-2-hydroxypropyl]-1-naphthalinsulfonamid,
N-[2-Hydroxy-3-(2-methyl-1-piperidinyl)propyl]-1-naphthalinsulfonamid,
N-[2-Hydroxy-3-(1-piperidinyl)propyl]-1-naphthalinsulfonamid, und
N-[2-Hydroxy-3-(1-pyrrolidinyl)propyl]-1-naphthalinsulfonamid, und Salze davon,
und mindestens ein(en) pharmazeutisch verträgliches(n) Träger, Verdünnungsmittel oder Exzpienten.

20. Verbindung wie in Anspruch 17 definiert, oder ein Salz, Solvat, Ester oder Amid davon, aber nicht einschließlich
4-Amino-N-(2-hydroxy-3-piperidin-1-ylpropyl)benzolsulfonamid,
N-[3-(2,4-Dimethyl-1-pyrrolidinyl)-2-hydroxypropyl]-1-naphthalinsulfonamid,
N-[2-Hydroxy-3-(2-methyl-1-piperidinyl)propyl]-1-naphthalinsulfonamid,
N-[2-Hydroxy-3-(1-piperidinyl)propyl]-1-naphthalinsulfonamid, und
N-[2-Hydroxy-3-(1-pyrrolidinyl)propyl]-1-naphthalinsulfonamid, und Salze davon.

## Revendications

1. Utilisation d'un composé de formule (I) : 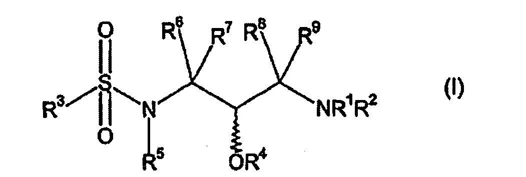 ou d'un de ses sels, produits de solvatation , esters ou amides, pour la production d'un médicament destiné au traitement de troubles à médiation par GlyTl, formule dans laquelle .
R¹ et R ² sont choisis indépendamment entre un atome d'hydrogène, des groupes alkyle en C ₁ à C ₆ et cycloalkyle en C ₃ à C ₆, sous réserve que R ¹ et R² ne représentent ni l'un ni l'autre un atome d'hydrogène, ou bien
R¹ et R ², conjointement avec l'atome d'azote auquel ils sont fixés, sont liés pour former un noyau tétra -, penta-, hexa - ou heptagonal saturé dans lequel un ou plusieurs des atomes de carbone sont facultativement remplacés par un hétéroatome choisi indépendamment entre N, O et S, ledit noyau saturé étant facult ativement substitué avec un ou plusieurs groupes choisis indépendamment entre des groupes alkyle en C ₁ à C ₆, cycloalkyle en C ₃ à C ₆, (cycloalkyle en C ₃ à C ₆) (alkyle en C ₁ à C ₄), aryle et aryl(alkyle en C ₁ à C ₄), et ledit noyau saturé étant en outre facultativement ponté par un groupe alkylène en C ₁ à C₃, et ledit noyau saturé étant en outre facultativement condensé avec un noyau alicyclique en C ₅ à C₇ ou un noyau aromatique ou hétéroaromatique penta - ou hexagonal facultativement substitué avec un ou plusieurs groupes choisis indépendamment entre des groupes alkyle C ₁ à C₆ et cycloalkyle en C₃ à C₆ ;
R³ représente un groupe 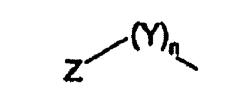 dans lequel
Y représente un groupe alkylène en C ₁ ou C ₂, alcénylène en C₂ ou alcynylène en C₂,
et
n est égal à 0 ou 1, et
Z représente un système de noyau aromatique monocyclique penta- à octogonal ou aromatique bicyclique hexa- à décagonal, danslequel un ou plusieurs des atomes de carbone sont facultativement remplacés par un hétéroatome choisi indépendamment entre N, O et S, et ledit système de noyau est facultativement substitué avec un ou plusieurs groupes choisis indépendamment entre des groupes choisis, -R¹⁰, -CF₃, - (alkyle en C ₁ à C ₆)sulfonyle -OR¹¹, -COOR¹², -CN, -NO₂, -NR¹³R¹⁴, -C(O)NR¹⁵R¹⁶, -NR¹⁷C (O) R¹⁸, -C (O) R¹⁹, -C (NR²⁰) NR²¹R²², -C (NOR²³) R²⁸,
dans lesquels
hal représente un groupe F, Cl, Br ou I,
R¹⁰ représente un groupe alkyle en C₁ à C₆, cycloalkyle en C₃ à C ₆, (cycloalkyle en C ₃ à C ₆) (alkyle en C ₁ à C ₄), aryle, aryloxy ou aryl(alkyle en C ₁ à C₄), facultativement substitué avec un ou plusieurs groupes choisis indépendamment entre des groupes hal, alkyle en C ₁ à C₆, -OR¹¹, -COOR¹², - CN,
-NO₂ et -NR¹³R¹⁴,
R¹¹, R¹², R¹³, R¹⁴, R¹⁵, R¹⁶, R¹⁷, R¹⁸, R²⁰, R²¹, R²², R²³ et R²⁶ sont choisis indépendamment entre un atome d'hydrogène et un groupe alkyle en C₁ à C₆ ;
R⁴ et R¹⁹ sont choisis indépendamment entre un atome d'hydrogène, des groupes alkyle en C ₁ à C₆, cycloalkyle en C₃ à C₆, aryle et aryl (alkyle en C ₁ à C₄), facultativement substitué avec un ou plusieurs groupes choisis indépendamment entre des groupes hal, alkyle en C ₁ à C ₆, -OR²⁴, -COOR²⁵, -CN, -NO₂ et -NR²⁶R²⁷ ;
R⁶, R⁷, R⁸ et R⁹ sont choisis indépendamment entre un atome d'hydrogène, des groupes alkyle en C ₁ à C ₆ et aryl (alkyle en C ₁ à C ₄), ou bien R ⁶ et R⁷ forment conjointement un groupe cycloalkyle en C ₃ à C₆, ou bien R⁸ et R⁹ forment conjointement un groupe cycloalkyle en C ₃ à C₆ ; où le groupe alkyle en C ₁ à C₆, le groupe aryl(alkyle en C₁ à C₄), le groupe cycloalkyle en C ₃ à C₆ formé par R⁶ et R⁷ et le groupe cycloalkyle en C₃ à C₆ formé par R⁸ et R⁹ sont facultativement substitués avec un ou plusieurs groupes choisis indépendamment entre des groupes hal, alkyle en C₁ à C₆, -OR²⁴, -COOR²⁵, -CN, -NO₂ et -NR²⁶R²⁷ ;
dans lesquels R ²⁴, R ²⁵, R ²⁶ et R ²⁷ sont choisis indépendamment entre un atome d'hydrogène et un groupe alkyle en C₁ à C₆ ;
et
R⁵ est choisi indépendamment entre un atome d'hydrogène, des groupes alkyle en C ₁ à C₆, cycloalkyle en C₃ à C₆, aryle et aryl (alkyle en C ₁ à C₄), facultativement substitué avec un ou plusieurs groupes choisis indépendamment entre des groupes hal, alkyle en C₁ à C ₆, -OR²⁴, -COOR²⁵, -CN, -NO₂ et -NR²⁶R²⁷,
dans lesquels les groupes R²⁴, R²⁵, R²⁶ et R²⁷ sont tels que définis ci-dessus,
et dans laquelle le terme "aryle" désigne un système de noyau aromatique ou hétéroaromatique penta - à heptagonal, le noyau hétéroaromatique contenant au moins un hétéroatome choisi entre N, O et S.

2. Utilisation suivant la revendication 1, dans laquelle le composé a la configuration stéréochimique suivante :

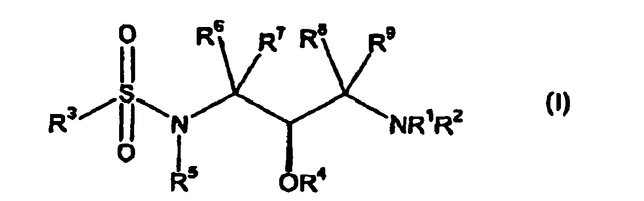

3. Utilisation suivant la revendication 1 ou la revendication 2, dans laquelle :
(a) R¹ et R ², conjointement avec l'atome d'azote auquel ils sont fixés, sont liés pour former un noyau penta- ou hexagonal facultativement substitué, dans lequel un ou plusieurs atomes de carbone sont facultativement remplacés par un hétéroatome choisi indépendamment entre N, O et S, ledit noyau étant en outre facultativement condensé avec un noyau alicyclique en C₅ à C₇ ou un noyau aromatique ou hétéroaromatique penta- ou hexagonal ; ou bien
(b) R¹ et R², conjointement avec l'atome de carbone auquel ils sont fixés, sont liés pour former un noyau hétérocyclique penta - ou hexagonal, dans lequel le seul hétéroatome est l'atome d'azote auquel R¹ et R² sont fixés, ledit noyau étant facultativement substitué avec un ou plusieurs groupes choisis entre des groupes alkyle en C ₁ à C₆ et cycloalkyle en C ₃ à C₆, plus avantageusement avec un ou plusieurs groupes choisis indépendamment entre des groupes alkyle en C₁ à C₄, de préférence méthyle, éthyle ou isopropyle ; ou bien
(c) R¹ et R ² sont choisis indépendamment entre des groupes alkyle en C₁ à C₆, de préférence alkyle en C₃ à C₆.

4. Utilisation suivant l'une quelconque des revendications 1 à 3, dans laquelle n est égal à 0.

5. Utilisation suivant l'une quelconque des revendications 1 à 4, dans laquelle Z représente un système de noyau monocyclique penta - ou hexagonal ou Z représente un système de noyau bicyclique hexa- à décagonal.

6. Utilisation suivant la revendication 5, dans laquelle Z représente un groupe phényle, thiényle, naphtyle, naphtyridinyle, quinolyle, isoquinolyle, benzothiényle, chromannyle, chroményle,imidazoleisothiazolyle, benzothiazolyle
ou benzofuryle, facultativement substitué avec ou plusieurs groupes choisis indépendamment entre -hal, alkyle en C ₁ à C₆, alkoxy en C₁ à C₆, CF₃, -CN et cycloalkyle en C₃ à C₆.

7. Utilisation suivant l'une quelconque des revendications 1 à 6, dans laquelle R ⁴ représente un atome d'hydrogène ou un groupe alkyle en C₁ à C₆.

8. Utilisation suivant l'une quelconque des revendications 1 à 7, dans laquelle R ⁵ est choisi entre un atome d'hydrogène, des groupes alkyle en C₁ à C₆, aryle et benzyle, facultativement substitué avec un ou plusieurs groupes choisis indépendamment entre des groupes h al, alkyle en C₁ à C₆ et OR²⁴.

9. Utilisation suivant l'une quelconque des revendications 1 à 8, dans laquelle R ⁶, R⁷, R⁸ et R⁹ sont choisis indépendamment entre un atome d'hydrogène et un groupe alkyle en C₁ à C₆.

10. Utilisation d'un composé de formule (Ia) : 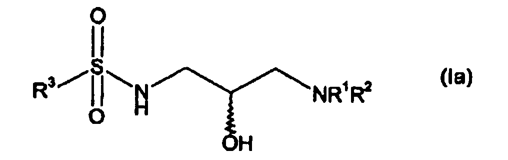 ou d'un de ses sels, produits de solvatation, esters ou amides, pour la production d'un médicament destiné au traitement de troubles à médiation par GlyTl, formule dans laquelle :
R¹ et R² sont choisis indépendamment parmi des groupes alkyle en C₃ à C₆, ou bien
R¹ et R², conjointement avec l'atome d'azote auquel ils sont fixé s, sont liés pour forme r un noyau hétérocyclique penta-, hexa- ou heptagonal, dans lequel le seul hétéroatome est l'atome d'azote auquel R¹ et R² sont fixés, ledit noyau étant facultativement substitué avec un
ou plusieurs groupes choisis indépendamment entre des groupes alkyle en C₁ à C₄ et cycloalkyle en C₃ à C₆, ledit noyau étant en outre facultativement condensé avec un noyau alicyclique aromatique en C₆, et ledit noyau étant en outre facultativement ponté par un groupe méthylène ;
R³ représente un groupe 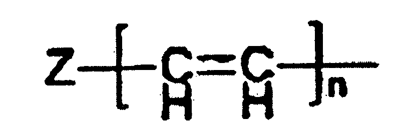 dans lequel
n est égal à 0 ou 1, et
Z représente un système de noyau aromatique monocyclique penta - ou hexagonal ou bicyclique octo - à décagonal,
dans lequel un ou plusieurs des atomes de carbone sont facultativement remplacés par un hétéroatome choisi indépendamment entre N, O et S, et ledit système de noyau est facultativement substitué avec un ou plusieurs groupes choisis indépendamment entre des groupes -hal, -R¹⁰, -OR¹¹, -COOR¹², -CN, -NO₂, -NR¹³R¹⁴, -CF₃ et - (alkyle en C₁ à C₆) sulfonyle,
dans lesquels
hal représente F, Cl, Br ou I,
R¹⁰ représente un groupe alkyle en C₁ à C₄, phényle ou phényloxy, facultativement substitué avec un ou plusieurs groupes hal, et
R¹¹, R¹², R¹³ et R¹⁴ sont choisis indépendamment entre un atome d'hydrogène et un groupe méthyle ;
et dans laquelle le terme "aryle" désigne un système de noyau aromatique ou hétéroaromatique penta - à heptagonal, le noyau hétéroaromatique contenant au moins un hétéroatome choisi entre N, O et S.

11. Utilisation suivant la revendication 10, dans laquelle, lorsque n est égal à 1, le groupe alcénylène en C₂ est en configuration *trans.*

12. Utilisation suivant la revendication 10 ou la revendication 11, dans laquelle le composé de formule (Ia) a la configuration stéréochimique suivante :

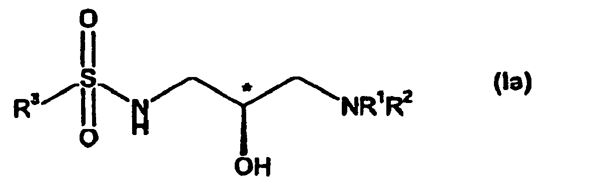

13. Utilisation suivant l'une quelconque des revendications 10, 11 et 12, dans laquelle R ¹ et R ², conjointement avec l'atome d'azote auquel ils sont fixés, sont liés pour former un noyau pyrrolidinyle ou un noyau pipéridinyle, facultativement substitué avec un ou plusieurs groupes choisis indépendamment entre des groupes alkyle en C ₁ à C ₄, de préférence méthyle, éthyle et isopropyle.

14. Utilisation suivant l'une quelconque des revendications 10 à 13, dans laquelle n est égal à 0.

15. Utilisation suivant l'une quelconque des revendications 10 à 14, dans laquelle Z est choisi entre des groupes 2- ou 3-thiényle, phényle, 5 -quinolinyle, 1-naphtyle et 2-naphtyle, facultativement substitué avec un ou plusieurs groupes choisis indépendamment entre des groupes -hal, -R¹⁰, -OR¹¹, -COOR¹², -CN, -NO₂, -NR¹³R¹⁴, -CF₃ et -(alkyle en C₁ à C₆)sulfonyle.

16. Utilisation suivant l'une quelconque des revendications 10 à 15, dans laquelle R³ représente un groupe 1-naphtyle ou 5-quinolinyle.

17. Utilisation suivant l'une quelconque des revendications 1 à 16, dans laquelle le composé est choisi dans le groupe consistant en :
le [(R)-2-hydroxy-3-piperidine-1-ylpropyl]-amide d'acide naphtalène-1-sulfonique,
le {(2R)-hydroxy-3-[(2R,S)-méthylpipéridine-1-yl]-propyl}amide d'acide naphtalène-1-sulfonique,
le {3-[(2R,6S)-diméthylpipérine-1-yl]-(2R)-hydroxypropyl} amide d'acide naphtalène-1-sulfonique,
le {3-[(2S)-éthylpipéridine-1-yl]-(2R)-hydroxypropyl}amide) d'acide naphtalène-1-sulfonique,
le {3-[(2R)-éthylpipéridine-1-yl]-(2R)-hydroxypropyl}amide d'acide naphtalène-1-sulfonique,
le trifluoracétate de ((R) -2-hydroxy-3-pyrrolidine-1-ylpropyl)amide d'acide naphtalène-1-sulfonique,
le trifluoracétate de [-(2R)-hydroxy3-(2-méthylpyrrolidine-1-yl)propyl]amide d'acide naphtalène-1-sulfonique,
le trifluoracétate de [(R)-3-(2,4-diméthylpyrrolidine-1-yl)-2-hydroxypropyl]amide d'acide naphtalène-sufonique,
le trifluoracétate de [(R)-2-hydroxy-3-(2-isopropylpyrrolidine-1-yl)propyl]amide d'acide naphtalène-1-sulfonique,
le trifluoracétate de [(R)-3-(2,5-diméthylpyrrolidine-1-yl)-2-hydroxypropyl]amide d'acide naphtalène-1-sulfonique,
le [(R) -3-(2-cyclohexylpyrrolidine-yl)-2-hydroxypropyl] amide d'acide naphtalène-1-sulfonique,
le [(R) -2-hydroxy-3-(2-isobuylpyrrolidine-yl)propyl] amide d'acide naphtalène-1-sulfonique,
le trifluoracétate de [(R)-3-(2-éthylpyrrolidine-1-yl)-2-hydroxypropyl]amide d'acide naphtalène-1-sulfonique,
le trifluoracétate de [-(R-)(2-tertiobutylpyrrolidine-1-yl)-2-hydroxypropyl]amide d'acide naphtalène-1-sulfonique,
le trifluoracétate de [-(R-)(2-cyclopropylpyrrolidine-1-yl)-2-hydroxypropyl]amide d'acide naphtalène-1-sulfonique,
le trifluoracétate de [-(R)-hydroxy-3-(3-méthylpipéridine-1-yl)propyl]amide d'acide naphtalène-1-sulfonique,
le [(R)-2-hydroxy-3-(5-éthyl-2-méthylpipéridine-1-yl)propyl-] amide d'acide naphtalène-1-sulfonique,
le trifluoracétate de [(R2)-hydroxy-3-(2-éthylpipéridine-1-yl)propyl]amide d'acide naphtalène-1-sulfonique,
le trifluoracétate de [-(2R)-hydroxy-3-(2-isopropylpipéridine-1-yl)propyl]amide d'acide naphtalène-1-sulfonique,
le trifluoracétate de [(R)-(*sec*.-butylpropylamino)-hydroxypropyl]amide d'acide naphtalène-1-sulfonique,
le trifluoracétate de [(R) -(tertiobutylpropylamino)-hydroxypropyl]amide d'acide naphtalène-1-sulfonique,
le trifluoracétate de [ (R)-3-(1,3-dihydro-iso-indole-2-yl)-2-hydroxypropyl]amide d'acide naphtalène-1-sulfonique,
le trifluoracétate de [(R) -2-hydroxy-3-(octahydro-iso-quinoléine-2-yl)propyl]amide d'acide naphtalène-1-sulfonique,
le [(R)-2-hydroxy-3-(octahydroquinoléine-2-yl)propyl]-amide d'acide naphtalène-1-sulfonique,
le trifluoracétate de [(R)-2-hydroxy-3-((1S,5R)-1,3,3-triméthyl-6-azabicyclo[3.2.1]oct-6-yl)-propyl]amide d'acide naphtalène-1-sulfonique,
le trifluoracétate de S[3-(2,4-diméthylpyrrolidine-1-yl)-2-hydroxypropyl]amide d'acide naphtalène-1-sulfonique,
le trifluoracétate de S[(-2-hydroxy-3-(2-méthylpyrrolidine-1-yl)propyl]amide d'acide naphtalène-1-sulfonique,
le trifluoracétate de [(S)-2-hydroxy-3-(3-méthylpipéridine-1-yl)propyl]amide d'acide naphtalène-1-sulfonique,
le [3-(2,4-diméthylpyrrolidine-1-yl) -2-hydroxypropyl] amide d'acide naphtalène-1-sulfonique,
le [(R)-3-(2,4-diméthylpyrrolidine-1-yl)-2-hydroxypropyl]-2-hydroxypropyl] - amide d'acide 5-diméthylamino-naphtalène-1-sulfonique,
le [(R)-3-(2,4-diméthylpyrrolidineyl)-2-hydroxypropyl]-2-hydroxypropyl]-amide d'acide naphtalène-2-sulfonique,
le 2, 5 -dichloro-N-[(R)-3- (2, 4-diméthyl-pyrrolidine-1-yl)-2-hydroxypropyl]-benzènesulfonamide,
le *N*-[(R)-3-(2,4-diméthylpyrrolidine-1-yl)-2-hydroxypropyl]-2-nitro-benzènesulfonamide,
le 3,5-dichlore-*N*- [(R)-3- (2, 4-diméthyl-pyrrolidine-1-yl)-2-hydroxypropyl]-2-hydroxy-benzènesulfonamide,
le *N*- [(R)-3- (2, 4-diméthylpyrrolidine-yl)-2-hydroxypropyl]-2,4,6-triisopropyl-benzènesulfonamide,
le *N*-[(R)-3-(2,4-diméthylpyrrolidine-1-yl)-2-hydroxypropyl]-4-nitro-benzènesulfonamide,
le *N*-[(R)-3-(2, 4-diméthylpyrrolidine-1-yl)-2-hydroxy-propyl]-4-méthoxy-benzènesulfonamide,
le [(R)-3-(2,4-diméthylpyrrolidine-1-yl)-2-hydroxy-propyl]-amide d'acide (E)-2-phényl-éthènesulfonique,
l'ester méthylique d'acide[ (R)-3-(2,4-diméthylpyrrolidine 1-yl)-2-hydroxypropylsulfamoyl]-benzoïque,
le *N*- [(R)-3- (2, 4-diméthylpyrrolidine-1-yl)-2-hydroxypropyl]-bis-trifluorométhyl-benzènesulfonamide,
le 3,4-dichlor-*N*-[(R)-3-(2,4-diméthyl-pyrrolidine-1-yl)-2-hydroxypropyl]benzènesulfonamide,
le *N*-[(R)-3-(2, 4-diméthylpyrrolidine-1-yl)-2-hydroxypropyl]-4-propyl-benzènesulfonamide,
le 4 -brom-*N*-[(R)-3-(2, 4-diméthylpyrrolidine-1-yl)-2-hydroxypropyl]-2,5-difluoro-benzènesulfonamide,
le *N*-[(R)-3-(2,4-diméthylpyrrolidine-1-yl)-2-hydroxypropyl]-3-fluoro-benzènesulfonamide,
le 4 -chloro-*N*- [(R)-3- (2,4-diméthyl-pyrrolidine-1-yl) -2-hydroxypropyl]-3-fluoro-benzènesulfonamide,
le 2 -chloro-*N*-[(R)-3-(2,4-diméthyl-pyrrolidine-1-yl)-2-hydroxypropyl]-benzènesulfonamide,
le [(R) -3-(2,4-diméthyl-pyrrolidine-1-yl)-2-hydroxppropy]amide d'acide 2,5-dichlorothiophène-3-sulfonique,
le *N*-[(R)-3-(2,4-diméthylpyrrolidine-1-yl)-2-hydroxypropyl] C-trifluorométhyl-benzènesulfonamide,
le *N*-[(R)-3-(2, 4-diméthylpyrrolidine-1-yl)-2-hydroxypropyl]-3-méthyl-benzènesulfonamide,
le 2,3-dichloro-*N*-[(R)-3-(2,4-diméthyl-pyrrolidine1-yl)-2-hydroxypropyl]-benzènesulfonamide,
le [(R)-3-(2,4-diméthylpyrrolidine-1-yl)-2-hydroxy-propyl]-amide d'acide 3-bromo-5-chlorothiophène-2-sulfonique,
le 2 -cyano-*N*-[(R)-3-(2,4-diméthylpyrrolidine-1-yl)-2-hydroxypropyl]-benzènesulfonamide,
le *N*-[(R)-3-(2,4-diméthylpyrrolidine-1-yl)-2-hydroxypropyl]-2,5-difluoro-benzènesulfonamide,
le 5-bromo-2-chloro-*N*-[(R)-3-(2,4-diméthylpyrrolidine-1-yl)-2-hydroxypropyl]-benzènesulfonamide,
le [(R)-3-(2,4-diméthylpyrrolidine-1-yl)-2-hydroxy-propyl]-amide d'acide 2,2,5,7,8-pentaméthyl-chromane-6-sulfonique,
le [(R)-3-(2,4-diméthylpyrrolidine-1-yl) -2-hydroxy-propyl]-amide d'acide benzo[1,2,5]thiadiazole-4-sulfonique,
le [(R)-3-(2,4-diméthylpyrididine-1-yl)-2-hydroxypropyl]-amide d'acide benzo[1,2,5]oxadiazole-4-sulfonique,
le [(R)-3- (2, 4-diméthylpyrrolidin-1-yl)-2-hydroxy-propyl]-amide d'acide biphényl-4-sulfonique,
le *N*- [(R)-3- (2, 4-diméthylpyrrolidine-1-yl)-2-hydroxypropyl]-4-méthyl-3-nitro-benzènesulfonamide,
le [(R) -3-(2,4-diméthy 1-pyrrolidine-1-yl)-2-hydroxypropyl]-amide d'acide 5 -chloro-3-méthyl-benzo[b]thiophène-2-sulfonique,
le 4 -butyl-*N*-[(R)-3-(2, 4-diméthylpyrrolidine-1-yl)-2-hydroxy propyl]-benzènesulfonamide,
le [(R)-3-(2,4-diméthyl-pyrrolidine-1-yl)-2-hydroxypropyl]-amide d'acide 5-chloro-benzo[b]oxadiazole-4-sulfonique,
le 4-butyl-*N*-[(R)-3-(2,4-diméthyl-pyrrolidinel-yl)-2-hydroxy propyl]-benzènesulfonamide,
le [(R)-3-(2,4-diméthyl-pyrrolidine-1-yl)-2-hydroxypropyl]-amide d'acide 6-chloro-imidazo[2,1-*b*]thiazole-5-sulfonique,
le *N*-[(R)-3-(2,4-diméthylpyrrolidine-1-yl)-2-hydroxypropyl]-3-méthoxy-benzènesulfonamide,
le [(R)-3-(2,4-diméthyl-pyrrolidine-1-yl)-2-hydroxypropyl]amide d'acide 5-iodo-naphtalène-1-sulfonique,
le 2-bromo-*N*-[(R)-3-(2,4-diméthylpyrrolidine-1-yl)-2-hydroxy propyl]-benzènesulfonamide,
le *N*-[(R)-3-(2,4-diméthyl-pyrrolidine-1-yl)-2-hydroxypropyl]-5-fluoro-2-méthyl-benzènesulfonamide,
le [(R)-3-(2,4-diméthylpyrrolidine-1-yl)-2-hydroxypropyl]-amide d'acide naphtalène-1-sulfonique,
le *N*-[(R)-3-(2,4-diméthyl-pyrrolidine-1-yl)-2-hydroxypropyl]-2,4,6-triméthyl-benzènesulfonamide,
le *N*-[(R)-3-(2, 4-diméthylpyrrolidine-1-yl)-2-hydroxypropyl]-trifluorométhylbenzènesulfonamide,
le [(R)-3-(2,6-diéthylpipéridine-1-yl)-2-hydroxy-propyl]-amide d'acide naphtalène-1-sulfonique,
le 2,3-dichloro-N-[(R)-3-((2S,6R)-2,6-diméthyl-pipéridine-1-yl)-2-hydroxypropyl]-benzènesulfonamide,
le 2,3,4-trichloro-*N*-[(R)-3-((2S,6R)-2,6-diméthylpipéridine-1-yl)-2-hydroxypropyl]-benzènesulfonamide,
le [(R)-3-((2S,6R)-2,6-diméthylpipéridine-1-yl)-2-hydroxy propyl]-amide d'acide 2,5-dichlorothiophène-3-sulfonique,
le [(R)-3-((2R,6S)-2,6-diméthyl-pipéridine-1-yl)-2-hydroxy propyl]amide d'acide 4,5-dibromo-thiophène-2-sulfonique,
le [(R)-3-((2S,6R)-2,6-diméthyl -pipéridine-1-yl)-2-hydroxy propyl]-amide d'acide 4-bromo 2,5 - dichloroth iophène-3-sulfonique,
le [(R)-3-((2S,6R)-2,6-diméthyl-pipéridine-1-yl)-2-hydroxy propyl]-amide d'acide 5-chloro-naphtalène-1-sulfonique,
le [(R)-3-((2S,6R)-2,6-diméthyl-pipéridine-1-yl)-2-hydroxy propy]-amide d'acide 5-chloro-naphtalène-2-sulfonique,
le [(R)-3-((2S,6R)-2,6-diméthyl-pipéridine-1-yl)-2-hydroxy propyl]-amide d'acide naphtalène-2-sulfonique,
le N-[(R)-3-((2S,6R)-2,6-diméthyl-pipéridine1-yl)-2-hydroxy propyl]-benzènesulfonamide,
le [(R)-3-((2S,6R)-2,6-diméthyl-pipéridine-1-yl)-2-hydroxy propyl]-amide d'acide 4'-chloro-biphényl-4-sulfonique,
le [(R)-3-((2S,6R)-2,6-diméthyl-pipéridine-1-yl)-2-hydroxy propyl]-amide d'acide biphényl-4-sulfonique,
le *N*-[(R)-3-((2S, 6R)-2,6-diméthyl-pipéridine-1-yl)-2-hydroxy propyl]-4-phénoxybenzènesulfonamide,
le 3, 4 -dichloro-*N*-[(R)-3-((2S,6R)-2,6-diméthylpipéridine-1-yl)-2-hydroxypropyl]-benzènesulfonamide,
le [(R)-3-((2S,6R)-2,6-diméthyl-pipéridine-1-yl)-2-hydroxy propyl]-amide d'acide quinoléine-5-sulfonique,
le [(R)-3-((2S,6R)-2,6-diméthyl-pipéridine-1-yl)-2-hydroxy propyl]-amide d'acide quinoléine-8-sulfonique,
le [(R)-3-((2S,6R)-2,6-diméthyl-pipéridine-1-yl)-2-hydroxypropyl]-amide d'acide 5-iodo-naphtalène-1-sulfonique,
le [(R)-3-((2S,6R)-2,6-diméthyl-pipéridine-1-yl)-2-hydroxy propyl]-amide d'acide 5-acétyl-naphtalène-1-sulfonique,
le [(R) -3-((S)-2-éthyl-pipéridine-1-yl)-2-hydroxy-propyl]-amide d'acide isoquinoléine-5-sulfonique,
le [(R) -3-((S)-2-éthyl-pipéridine-1-yl)-2-hydroxypropyl]-amide d'acide quinoléine-8-sulfonique,
le [(R) -3-((S)-2-éthyl-pipéridine-1-yl)-2-hydroxy-propyl]-amide d'acide 5-chloro-3-méthyl-benzo[b]thiophène-2-sulfonique
le *N*-[ (R)-3- ( (S)-2-éthylpipéridine-1-yl)-2-hydroxypropyl]-1-phényl-méthanesulfonamide,
le 2,3-dichloro-*N*-[(R)-3-((S)-2-éthyl-pipéridine-1-yl)-2-hydroxy-propyl]-benzènesulfonamide,
le [(R)-3-((S)-2-éthyl-pipéridine-1-yl)-2-hydroxy-propyl]-amide d'acide thiophène-2-sulfonique,
le [(R)3-((S)-2-éthyl-pipéridine-1-yl)-2-hydroxypropyl]-amide d'acide 2,5-dichlorothiophène-3-sulfonique,
le [(R)-3-((2R,6S)-2,6-diméthyl-pipéridine-1-yl)-2-hydroxy propyl]-amide d'acide 5-méthoxynaphtalène-1-sulfonique,
le [(R)-3-((2R,6S)-2,6-diméthyl-pipéridine-1-yl)-2-hydroxy propyl]-amide d'acide 5-cyanonaphtalène-1-sulfonique,
le [(R)-3-((2R,6S)-2,6-diméthyl-pipéridine-1-yl)-2-hydroxy propyl]-amide d'acide 4-cyanonaphtalène-1-sulfonique,
le [(R)-3-((2R,6S)-2,6-diméthyl-pipéridine-1-yl)-2-hydroxypropyl]-amide d'acide 4-bromonaphtalène-1-sulfonique,
le [(R)3-((2R,6S)-2,6-diméthyl-pip.éridine-1-yl)-2-hydroxypropyl]-amide d'acide 4-fluoronaphtalène-1-sulfonique,
le N-[(R)-3-((2R,6S)-2,6-diméthyl-pipéridine-1-yl)-2-hydroxy-propyl]-2,3-diméthyl-benzènesulfonamide,
le N-[(R)-3-((2R,6S)-2,6-diméthyl-pipéridine-1-yl)-2-hydroxy-propyl]-3,4-diméthyl-benzènesulfonamide,
le N-[(R)-3-((2R,6S)-2,6-diméthyl-pipéridine-1-yl)-2-hydroxy-propyl]-2,3-diméthoxy-benzènesulfonamide,
le [(R)-3-((2R,6S)-2,6-diméthyl-pipéridine-1-yl)-2-hydroxy propyl]-amide d'acide7-trifluorométhylquinoléine-5-sulfonique
le [(R)-3-((2R,6S)-2,6-diméthyl-pipéridine-1-yl)-2-hydroxy propyl]-amide d'acide7-fluoro-2-méthyl]quinoléine-5-sulfonique
et ses sels, produits de solvatation, esters et amides.

18. Composé suivant la revendication 17, ou un de ses sels, produits de solvatation, esters ou amides, destiné à être utilisé en thérapie, mais ne comprenant pas
le N - [3-(2,4-diméthyl-1-pyrrolidinyl)-2-hydroxypropyl]-1-naphtalènesulfonamide,
le N -[2-hydroxy-3-(2-méthyl-1-pipéridinyl)propyl]-1-naphtalènesulfonamide,
le N-[2-hydroxy-3-(1-pipéridinyl)propyl]-1-naphtalène-sulfonamide, et
le N -[2-hydroxy-3-(1-pyrrolidinyl)propyl]-1-naphtalène-sulfonamide,
et leurs sels.

19. Composition pharmaceutique comprenant comme ingrédient actif un composé tel que défini dans la revendication 17 ou un de ses sels, produits de solvatations, esters ou amides, mais ne comprenant pas
le N - [3- (2, 4-diméthyl-1-pyrrolidinyl) -2-hydroxypropyl]-1-naphtalènesulfonamide,
le N - [2-hydroxy-3- (2-méthyl-1-pipéridinyl)pr opyl] -1-naphtalènesulfonamide,
le N-[2-hydroxy-3-(1-pipéridinyl)propyl]-1-naphtalène-sulfonamide, et
le N- [2-hydroxy-3- (1-pyrrolidinyl)propyl]-1-naphtalène-sulfonamide, et leurs sels,
et au moins un support, diluant ou excipient pharmaceutiquement acceptable.

20. Composé suivant la revendication 17 ou un de ses sels, produits de solvatation, esters ou amides, mais ne comprenant pas
le 4 -amino-N-(2-hydroxy -3-pipéridine-1-yl-propyl)-benzènesulfonamide,
le N-[3-(2,4-diméthyl-1-pyrrolidinyl)-2-hydroxypropyl]-1-naphtalènesulfonamide,
le N- [2-hydroxy-3- (2-méthyl-1-pipéridinyl)propyl] -1-naphtalènesulfonamide,
le N-[2-hydroxy-3-(1-pipéridinyl)propyl]-1-naphtalène-sulfonamide, et
le N-[2-hydroxy-3-(1-pyrrolidinyl)propyl]-1-naphtalène-sulfonamide, et leurs sels.
